(19) Europäisches Patentamt
European Patent Office
Office européen des brevets

(11) EP 3 118 219 A1

(12) EUROPEAN PATENT APPLICATION

(43) Date of publication:
**18.01.2017 Bulletin 2017/03**

(51) Int Cl.:
***C07K 14/705*** *(2006.01)* ***C07K 14/47*** *(2006.01)*
***G01N 33/68*** *(2006.01)*

(21) Application number: **15177390.0**

(22) Date of filing: **17.07.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicants:
- **Centre National de la Recherche Scientifique (C.N.R.S.)**
**75016 Paris (FR)**
- **Université de Montpellier**
**34090 Montpellier (FR)**

(72) Inventors:
- **CHARNET, Pierre**
**34270 Saint-Mathieu-de-Tréviers (FR)**
- **CENS, Thierry**
**34920 Le Crès (FR)**
- **ROUSSET, Matthieu**
**34000 Montpellier (FR)**

(74) Representative: **Icosa**
**83 avenue Denfert-Rochereau**
**75014 Paris (FR)**

# (54) NICOTINIC CHANNEL SUBUNITS OF POLLINATOR INSECTS AND THEIR USES THEREOF

(57) The present invention relates to the cloning of the nicotinic acetylcholine receptors (nAChR) subunits in species from the phylum arthropoda and in particular a pollinator insect such as *Apis mellifera* and their uses thereof.



Printed by Jouve, 75001 PARIS (FR)

## Description

### FIELD OF INVENTION

The present invention relates to the expression of the nicotinic acetylcholine receptors (nAChR) subunits in species from the phylum arthropoda and in particular a pollinator insect such as *Apis mellifera* and their uses thereof.

### BACKGROUND OF INVENTION

During the last decades, agriculture has changed to meet the increasing demand to produce food. There has been expansion of cultivated areas in monoculture and increases use of pesticides. The abusive use of pesticides is subjecting pollinator insects to stress as evidenced by a constant decrease in the density of these pollinator insects around agricultural fields in many parts of the world, thus causing economic losses. Pollinators are crucial for the pollination of agricultural crops and natural areas around the world.

Among these pollinator insects, some insects issued from the order of *hymenoptera* or bees are considered excellent pollinating insects in agro-ecosystems because they visit many flowers on the same day. Among these pollinator insects, Bumble bees for instance have already been red-listed and are in danger of extinction.

Another bee such as *Apis mellifera* or honey bee is interesting from an economic perspective because it provides products of great value, such as honey, propolis, royal jelly, wax, and apitoxin (bee venom).

The decline of the bee colonies and pollinator insects due to pesticides is not only marked by the increase of their bee mortality but also modifications related to their memory or social behavior within the hives (Suchail S et al 2001 Environ Toxicol Chem 20 (11):2482-6; Gels JA 2002 J Econ Entomol; 95(4):722-8). A direct link between the uses of phytosanitary products issued from the neonicotinoid family and the decline of the bee colonies has already been established. For this reason, France has decided the ban of these products from January the 1st 2016 while the United States won't deliver any more authorization of marketing any new derivate products from the neonicotinoid family. These products are known to target the nicotinic acetylcholine receptor (nAChR) channels.

Some toxicity assays have already been developed by oral or topical application of a substance on the insect however these assays are not reproducible and are not adapted to the test of a large diversity of compounds (OECD guidelines for the testing of chemicals OECD214 - September 1998). Consequently there is a need to develop *in vitro* high throughput assays highly reproducible to diagnose bee colonies health, to determine any toxicity within the hives or to screen new compounds not targeting bee colonies.

The present application indeed unravels sequences of nAChR subunits and the sequences of chaperone proteins from pollinator insects able to form a functional channel that can respond to a modulator. In addition, the present application discloses a method using the nucleic acid sequences of nAChR channel subunits and chaperone proteins from pollinator insects to analyze the effect of compounds on nAChR channel activity.

### SUMMARY

One object of the invention is a nucleic acid sequence of at least one subunit $\alpha$ of the nicotinic acetylcholine receptor of an arthropod as set forth in SEQ ID NO: 1 to 9 or a variant thereof.

Another object of the invention is a nucleic acid sequence of at least one subunit $\beta$ of the nicotinic acetylcholine receptor of an arthropod as set forth in SEQ ID NO: 10 to 11 or a variant thereof.

Another object of the invention is a nucleic acid sequence of the chaperone protein of the nicotinic acetylcholine receptor of an arthropod as set forth in SEQ ID NO: 12 to 15 or a variant thereof.

Another object of the invention is a vector comprising at least one nucleic acid sequence as defined here above.

Another object of the invention is an isolated cell transfected with at least one vector as defined here above.

Another object of the invention is a functional nicotinic acetylcholine receptor (nAChR) channel comprising at least one subunit $\alpha$ of nAChR encoded by a nucleic acid sequence selected from the group comprising SEQ ID NO: 1 to 9 or a variant thereof as defined here above.

Another object of the invention is a functional nAChR channel comprising at least one subunit $\alpha$ encoded by a nucleic acid sequence selected from the group comprising SEQ ID NO: 1 to 9 or a variant thereof as defined here above and at least one chaperone protein encoded by the nucleic acid sequence selected from the group comprising SEQ ID NO: 12 to 15 or a variant thereof as defined here above.

Another object of the invention is a functional nicotinic acetylcholine receptor (nAChR) channel comprising at least one subunit $\alpha$ of nAChR encoded by a nucleic acid sequence selected from the group comprising SEQ ID NO: 1 to 9 or a variant thereof as defined here above and at least one subunit $\beta$ encoded by a nucleic acid sequence selected from the group comprising SEQ ID NO: 10 to 11 or a variant thereof as defined here above.

Another object of the invention is a functional nAChR channel of an arthropod comprising at least one subunit α of nAChR encoded by a nucleic acid sequence selected from the group comprising SEQ ID NO: 1 to 9 or a variant thereof as defined here above, at least one subunit β of nAChR encoded by the nucleic acid sequence selected from the group comprising SEQ ID NO: 10 to 11 or a variant thereof as defined here above and at least one chaperone protein encoded by nucleic acid sequences selected from the group comprising SEQ ID NO: 12 to 15 or a variant thereof as defined here above.

Another object of the invention is a vector comprising the channel as defined here above.

Another object of the invention is an isolated cell transfected with at least one vector as defined here above, and expressing the channel as defined here above, preferably said cell is an oocyte of Xenopus.

Another object of the invention is an *in vitro* method to determine the effect of a test compound on the modulation of activity of a nAChR channel of an arthropod comprising:

a. contacting an isolated cell as defined here above with at least one test compound,
b. measuring the effect of said test compound on the channel activity, and comparing said effect to the effect without test compound or to the effect of a reference value, thereby determining a modulation of activity of said channel.

In one embodiment, said method is for determining the toxicity of a test compound on an arthropod.

Another object of the invention is an *in vitro* method for screening compounds that modulate the nAChR channel activity of an arthropod comprising:

a. contacting an isolated cell as defined here above with at least one test compound,
b. measuring the effect of said compound on the nAChR channel activity, and

comparing said effect to the effect without test compound or to the effect of a reference value, thereby determining a modulation of activity of said nAChR channel.

Another object of the invention is a kit comprising at least one vector as defined here above, or an isolated cell as defined here above and reagents.

**DEFINITIONS**

In the present invention, the following terms have the following meanings:

- "**Agonists**" refer to compounds that bind to the receptor site where acetylcholine (Ach) or acetylcholine binding protein (AChBP) binds (is also referred as the "active" or "orthosteric" site) and activate it, resulting in increased channel conductance.

- "**Antagonists**" refer to compounds that bind to the receptor site where ACh binds but do not activate it. Though they have no effect on their own, antagonists compete with ACh for binding and thereby inhibit its action, resulting in decreased channel activity.

- "**Positive allosteric modulators**" refer to compounds that bind to allosteric sites on the receptor complex causing increased efficiency of the main site and therefore an indirect increase in channel activity.

- "**Negative allosteric modulators**" refer to compounds that bind to allosteric site on the receptor complex causing decreased efficiency of the main site and therefore an indirect decrease in channel activity.

- "**Open channel blockers**" refer to compounds that block the ion channel pore once it is open, and thus prolong ligand-receptor occupancy, slow activation kinetics and inhibit ion flux in a subunit configuration-dependent and sensitization-state dependent manner.

- "**Non-competitive channel blockers**" refer to compounds that bind to or near the central pore of the receptor complex and directly block channel conductance through the ion channel.

- "**Am**" or "**Amel**" preceding any gene's name refer to the pollinator insect *Apis mellifera.*

- "**About**" preceding a figure and/or a score means plus or less 10% of the value of said figure and/or score.

- "**Derivative**" or "**analog**" of a compound refers broadly to the modification or substitution of one or more chemical moieties on a parent compound and may include functional derivatives, positional isomers, tautomers, zwitterions, enantiomers, diastereomers, racemates, isosteres or stereochemical mixtures thereof.

- "**Identity**" when used in a relationship between the sequences of two or more nucleic acid sequences or amino acid sequences, refers to the degree of sequence relatedness between the sequences, as determined by the number of matches between strings of two or more base pairs of nucleic acid or amino acid residues. "Identity" measures the percent of identical matches between the smaller of two or more sequences with gap alignments (if any) addressed by a particular mathematical model or computer program (i.e., "algorithms"). Identity of related nucleic acid sequences or amino acid sequences can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988). Preferred methods for determining identity are designed to give the largest match between the sequences tested. Methods of determining identity are described in publicly available computer programs. Preferred computer program methods for determining identity between two sequences include the GCG program package, including GAP (Devereux et al., Nucl. Acid. Res. \2, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

- "**Functional channel**", "**functional expression**" refer to the synthesis and any necessary post-translational processing of at least one subunit of the channel and/or at least one of the chaperone protein in an isolated cell so that the subunit or its chaperone protein is inserted properly in the cell membrane and is capable of conducting ions in response to an exposure to appropriate pharmacological agents or test compounds.

- "**Minimal modulation doses**" refer to the lowest concentration of a test compound required to modulate the nAChR channel activity.

- "**Modulation**", "**modulating**" or "**modulator**" of nAChR channel activity refers to the three distinct states that can be modulated and in which a nAChR channel can be configured: closed, open and desensitized. The binding of at least one ligand to the channel makes the transition between a closed (or a resting state) to an open (activated) state. Depending on the subunits constituting the nAChR channel the exposure time or the dose of the agonist (and optionally the co-agonist) the nAChR channel transits to a desensitized state. It also refers to compounds including "**agonists**", "**antagonists**", "**positive allosteric modulators**", "**negative allosteric modulators**", "**non-competitive channel blockers**", "**open channel blockers**" that may affect the current flow within the nAChR channel by inducing modification(s) of conformation on said nAChR channel, subunits assembly or organization, modification of the targeting, trafficking of the channels to the plasma membrane, modification of the life time of the said nAChR channel to the plasma membrane, or blocking the channel gate or any modification of post-translational state like phosphorylating state of the said nAChR channel.

- "**Nucleic acid sequence**" refers to encompass nucleic acids having the sequences set forth below as well as variants thereof including for example fragments, deletions, insertions and substitutions that maintain the ability to encode the different subunits of the nAChR channel of the invention.

- "**Phytosanitary product**" refers to biological or chemical compounds used as insecticides, herbicides, fungicides, fertilizers, antibiotics, or any products used in agriculture, in wine-making, food storage, ship bottom, for animals or domestic purposes.

- "**Sub-lethal doses**" refer to a concentration of a potentially lethal test compound that is not high enough to cause death meaning a concentration under the median lethal dose ($LD_{50}$) but still able to trigger death by a mechanism which is not acute (immediate). At sub-lethal dose, the test compound may induce changes in biological mechanisms that include but are not limited to: colony level behavioral changes, individual level behavioral changes, memory changes, cellular mechanisms or molecular mechanisms changes. The determination of mortality of some of these biological mechanisms changes is well-known in the state of the art. Therefore, technics determining such changes or mortality can easily be determined by the skilled artisan.

- "**Test compound**" refers to a phytosanitary product, a molecule, an organism or extract thereof eventually able to bind and/or modulate the nAChR channel activity of the invention.

- "**Variation**" refers to a single or several mutation(s) including end point mutation(s) or frameshift mutation(s), deletion(s), insertion(s), substitution(s), inversion(s), translocation(s), copy number loss, copy number gain.

**DETAILED DESCRIPTION**

One object of the present invention is the isolated nucleic acid sequences or a variant thereof comprising the subunits of the nAChR channel of a species from the phylum arthropoda.

The structure of the nAChR channel is well conserved between species. They belong to the cysteine-loop family of ligand-gated ion channels. In insects, nAChR channels are supposed to be constituted, by homology with vertebrates, by the assembly of 5 homologous subunits (pentameric structure) that form a membrane complex with an axial pseudo-symmetry and a central ion channel pore. In insects, 9 genes are encoding for $\alpha$ subunits (Am-nAChR$\alpha$1-9) while the $\beta$ subunits are encoded by only two genes (AmnAChR$\beta$1-2). The functioning of the nAChR channel may sometimes require chaperone proteins (ric-3, unc-74, unc-50, emc-6). As with all ligand-gated ion channels, opening of the nAChR channel pore requires the binding of a chemical messenger. Several different terms are used to refer to the molecules that bind receptors, such as ligand.

The phylum arthropod is well-known from the skilled artisan that will recognize which species belong to this family. Arthropod phylum includes but is not limited to: the class of insects or arachnid.

The class of insects includes but is not limited to: pollinator insects, invasive insects.

Pollinator insects of the invention include species from the order *hymenoptera*, the family *Apidae* and the subfamily *Apinae* particularly the non-invasive species that does not damage crops or parasite other and/or native pollinator insects (insect native from a specific region), or damage hives.

Pollinator insects of the invention include in a non-limiting list: bees, honeybees such as *Apis mellifera*, *Apis cerana*, *Apis dorsata*, *Apis florea,* stingless bees such as *Melipona beecheii* or *Melipona yucatanica*, *Melipona qaradrifasciata anthidioides*, orchid bees, bumble bees such as *Bombus franklini, Bombus terricola, Bombus affinis* and *Bombus occidentalis.* Preferably, the pollinator insect of the invention is a honey bee, and most preferably *Apis mellifera.*

Invasive species of the invention include but are not limited to: introduced bees, imported bees, species from the order *hymenoptera,* the family *Vespidae* and the subfamily *Vespinae* particularly Asian predatory wasp (*Vespa velutina*), species from the class of arachnid.

The class of arachnid includes but is not limited to: the parasites, mites from the genus Varroa. The species of Varroa include but are not limited to: *Varroa destructor*, *Varroa jacobsoni, Varroa rindereri, Varroa sinhai, Varroa wongsirii.*

In one embodiment of the invention, the isolated nucleic acid sequence comprises the nicotinic acetylcholine receptor alpha1 subunit nucleic acid sequence as set forth in SEQ ID NO: 1 or a variant thereof consisting of a nucleic acid sequence having a maximum length of less than 2000; 1950; 1930; 1900; 1850 base pairs (bp) and having a minimum length of more than 1400; 1450; 1500; 1550; 1600; 1650; 1700; 1750 bp and having at least 80, 85, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1.

In one embodiment the variant of SEQ ID NO: 1 consists of a nucleic acid sequence of 1806 bp and having at least 80, 85, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1.

In one embodiment, a variation may occur in SEQ ID NO: 1.

In another embodiment of the invention, the isolated nucleic acid sequence comprises the nicotinic acetylcholine receptor alpha2 subunit nucleic acid sequence as set forth in SEQ ID NO: 2 or a variant thereof consisting of a nucleic acid sequence having a maximum length of less than 2000; 1950; 1930; 1900; 1850; 1800; 1750; 1700; 1650 base pairs (bp) and having a minimum length of more than 1300; 1400; 1450; 1500; 1550; 1600 bp and having at least 80, 85, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 2.

In one embodiment, a variation may occur in SEQ ID NO: 2.

In another embodiment of the invention, the isolated nucleic acid sequence comprises the nicotinic acetylcholine receptor alpha3 subunit nucleic acid sequence as set forth in SEQ ID NO: 3 or a variant thereof consisting of a nucleic acid sequence having a minimum length of more than 1702 base pair (bp), having a maximum length of less than 2500; 2400; 2300; 2200; 2100; 2000; 1900; 1850; 1800; 1750 bp and having at least 80, 85, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 3.

In one embodiment, a variation may occur in SEQ ID NO: 3.

In another embodiment of the invention, the isolated nucleic acid sequence comprises the nicotinic acetylcholine receptor alpha4 subunit nucleic acid sequence as set forth in SEQ ID NO: 4 or a variant thereof consisting of a nucleic acid sequence having a minimum length of more than 1500; 1550; 1600; 1650; 1700 bp and having a maximum length of less than 1800; 1750; 1725 base pairs (bp) and having at least 80, 85, 90, 91, 92, 93, 94, 95, 96, 97, 98, 98.1, 98.2, 98.3, 98.4, 98.5, 98.6, 98.7, 98.8, 98.9, 99, 99.1; 99.2; 99.3; 99.4; 99.5; 99.6; 99.7; 99.8; 99.9% of identity with SEQ ID NO: 4.

In one embodiment, a variation may occur in SEQ ID NO: 4.

In another embodiment of the invention, the isolated nucleic acid sequence comprises the nicotinic acetylcholine receptor alpha5 subunit nucleic acid sequence as set forth in SEQ ID NO: 5 or a variant thereof consisting of a nucleic acid sequence having a minimum length of more than 1000; 1100; 1200; 1300; 1350; 1400 bp and having a maximum length of less than 1700; 1600; 1500; 1450 base pairs (bp) and having at least 80, 85, 90, 91, 92, 93, 94, 95, 96, 97, 98, 98.1, 98.2, 98.3, 98.4, 98.5, 98.6, 98.7, 98.8, 98.9, 99, 99.1; 99.2; 99.3; 99.4; 99.5; 99.6; 99.7; 99.8; 99.9% of identity with SEQ ID NO: 5.

In one embodiment, a variation may occur in SEQ ID NO: 5.

In another embodiment of the invention, the isolated nucleic acid sequence comprises the nicotinic acetylcholine receptor alpha6 subunit nucleic acid sequence as set forth in SEQ ID NO: 6 or a variant thereof consisting of a nucleic acid sequence having a minimum length of more than 1400; 1500; 1550; 1580; 1585 bp and having a maximum length of less than 1750; 1700; 1650; 1600 bp and having at least 80, 85, 90, 91, 92, 93, 94, 95, 96, 97, 98, 98.1, 98.2, 98.3, 98.4, 98.5, 98.6, 98.7, 98.8, 98.9, 99, 99.1; 99.2; 99.3; 99.4; 99.5; 99.6; 99.7; 99.8; 99.9% of identity with SEQ ID NO: 6.

In one embodiment, a variation may occur in SEQ ID NO: 6.

In another embodiment of the invention, the isolated nucleic acid sequence comprises the nicotinic acetylcholine receptor alpha7 subunit nucleic acid sequence as set forth in SEQ ID NO: 7 or a variant thereof consisting of a nucleic acid sequence having a maximum length of less than 1800; 1750; 1740; 1700; 1650 base pairs (bp), having a minimum length of more than 1500; 1550; 1600; 1620; 1650 bp and having at least 80, 85, 90, 91, 92, 93, 94, 95, 96, 97, 98, 98.1, 98.2, 98.3, 98.4, 98.5, 98.6, 98.7, 98.8, 98.9, 99, 99.1; 99.2; 99.3; 99.4; 99.5; 99.6; 99.7; 99.8; 99.9% of identity with SEQ ID NO: 7.

In one embodiment, a variation may occur in SEQ ID NO: 7.

In another embodiment of the invention, the isolated nucleic acid sequence comprises the nicotinic acetylcholine receptor alpha8 subunit nucleic acid sequence as set forth in SEQ ID NO: 8 or a variant thereof consisting of a nucleic acid sequence having a maximum length of less than 1900; 1950; 1800; 1850; 1800; 1750; 1700; 1650 base pairs (bp), having a minimum length of more than 1400; 1500; 1520; 1550; 1600 bp and having at least 80, 85, 90, 91, 92, 93, 94, 95, 96, 97, 97, 97.1, 97.2, 97.3, 97.4, 97.5, 97.6, 97.7, 97.8, 97.9, 98, 98.1, 98.2, 98.3, 98.4, 98.5, 98.6, 98.7, 98.8, 98.9, 99, 99.1; 99.2; 99.3; 99.4; 99.5; 99.6; 99.7; 99.8; 99.9% of identity with SEQ ID NO: 8.

In one embodiment, a variation may occur in SEQ ID NO: 8.

In another embodiment of the invention, the isolated nucleic acid sequence comprises the nicotinic acetylcholine receptor alpha9 subunit nucleic acid sequence as set forth in SEQ ID NO: 9 or a variant thereof consisting of a nucleic acid sequence having a minimum length of more than 1000; 1050; 1100; 1150; 1200; 1250 bp and having a maximum length of less than 1500; 1450; 1400; 1350; 1330; 1300 base pairs (bp) and having at least 80, 85, 90, 91, 92, 93, 94, 95, 96, 97, 98, 98.1, 98.2, 98.3, 98.4, 98.5, 98.6, 98.7, 98.8, 98.9, 99, 99.1; 99.2; 99.3; 99.4; 99.5; 99.6; 99.7; 99.8; 99.9% of identity with SEQ ID NO: 9.

In one embodiment, a variation may occur in SEQ ID NO: 9.

In another embodiment of the invention, the isolated nucleic acid sequence comprises the nicotinic acetylcholine receptor beta1 subunit nucleic acid sequence as set forth in SEQ ID NO: 10 or a variant thereof consisting of a nucleic acid sequence having a minimum length of more than 1300; 1400; 1450; 1500; 1550 and having a maximum length of less than 1800; 1700; 1600; 1650; 1500; 1550 base pairs (bp) and having at least 80, 85, 90, 91, 92, 93, 94, 95, 96, 97, 98, 98.1, 98.2, 98.3, 98.4, 98.5, 98.6, 98.7, 98.8, 98.9, 99, 99.1; 99.2; 99.3; 99.4; 99.5; 99.6; 99.7; 99.8; 99.9% of identity with SEQ ID NO: 10.

In one embodiment, a variation may occur in SEQ ID NO: 10.

In another embodiment of the invention, the isolated nucleic acid sequence comprises the nicotinic acetylcholine receptor beta2 subunit nucleic acid sequence as set forth in SEQ ID NO: 11 or a variant thereof consisting of a nucleic acid sequence having a maximum length of less than 1500; 1450; 1400; 1350; 1300 base pairs (bp), having a minimum length of more than 1000; 1100; 1150; 1200; 1250 bp and having at least 80, 85, 90, 91, 92, 93, 94, 95, 96, 97, 98, 98.1, 98.2, 98.3, 98.4, 98.5, 98.6, 98.7, 98.8, 98.9, 99, 99.1; 99.2; 99.3; 99.4; 99.5; 99.6; 99.7; 99.8; 99.9% of identity with SEQ ID NO: 11.

In one embodiment, the variant of SEQ ID NO: 11 consists in a nucleic acid sequence of 1284 base pairs (bp) and having at least 80, 85, 90, 91, 92, 93, 94, 95, 96, 97, 98, 98.1, 98.2, 98.3, 98.4, 98.5, 98.6, 98.7, 98.8, 98.9, 99, 99.1; 99.2; 99.3; 99.4; 99.5; 99.6; 99.7; 99.8; 99.9% of identity with SEQ ID NO: 11.

In one embodiment, a variation may occur in SEQ ID NO: 11.

Another object of the present invention is the isolated nucleic acid sequences or a variant thereof comprising the chaperone protein of a species from the phylum arthropod.

In one embodiment of the invention, the isolated nucleic acid sequence comprises the chaperone protein resistance to inhibitors of cholinesterase 3 (ric-3) nucleic acid sequence as set forth in SEQ ID NO: 12 or variant thereof consisting of a nucleic acid sequence having a minimum length of more than 1100; 1200; 1250; 1300; 1350 bp and having a maximum length of less than 2000; 1900; 1800; 1700; 1600; 1500; 1400 base pairs (bp) and having at least 50, 60, 70, 80, 85, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 12.

In one embodiment, a variation may occur in SEQ ID NO: 12.

In another embodiment of the invention, the isolated nucleic acid sequence comprises the chaperone protein endothelium reticulum (ER) membrane protein complex-6 (emc-6) nucleic acid sequence as set forth as in SEQ ID NO: 13 or a variant thereof consisting of a nucleic acid sequence having a minimum length of more than 100; 150; 200; 250; 300 bp and having a maximum length of less than 500; 400; 450; 300; 350 bp and having at least 50, 60, 70, 75, 80, 85, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 13.

In one embodiment, a variation may occur in SEQ ID NO: 13.

In another embodiment of the invention, the isolated nucleic acid sequence comprises the chaperone protein unc-50 nucleic acid sequence as set forth as in SEQ ID NO: 14 or a variant thereof consisting of a nucleic acid sequence having a minimum length of more than 500; 600; 650; 700; 750 bp and having a maximum length of less than 1000; 950; 900; 850 bp and having at least 50, 60, 70, 80, 85, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 14.

In one embodiment, a variation may occur in SEQ ID NO: 14.

In another embodiment of the invention, the isolated nucleic acid sequence comprises the chaperone protein unc-74 nucleic acid sequence as set forth as in SEQ ID NO: 15 or a variant thereof consisting of a nucleic acid sequence having a minimum length of more than 1000; 1050; 1100; 1150; 1200; 1250 bp and having a maximum length of less than 1600; 1550; 1500; 1450; 1400; 1350; 1300 bp and having at least 50, 60, 70, 80, 85, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 15.

In one embodiment, a variation may occur in SEQ ID NO: 15.

Another object of the present invention is a functional nicotinic acetylcholine receptor (nAChR) channel of a species from the phylum arthropod.

In one embodiment of the invention, the functional nAChR channel is from a pollinator insect.

In another embodiment of the invention, the functional nAChR channel is a parasite from the genus *Varroa.*

In one embodiment of the invention, the functional nAChR channel comprises at least one subunit of the nicotinic acetylcholine receptor (nAChR) channel.

In another embodiment of the invention, the functional nAChR channel comprises at least one subunit $\alpha$ of the nicotinic acetylcholine receptor (nAChR) channel.

In another embodiment of the invention, the functional nAChR channel comprises at least two identical subunits $\alpha$ of the nicotinic acetylcholine receptor (nAChR) channel.

In another embodiment of the invention, the functional nAChR channel does not comprise both subunits $\beta 1$ and $\beta 2$ of the nicotinic acetylcholine receptor (nAChR).

In one embodiment, the at least one subunit of the nicotinic acetylcholine receptor (nAChR) channel of the functional nAChR channel is phosphorylated.

In one embodiment, the at least one subunit of the nicotinic acetylcholine receptor (nAChR) channel of the functional nAChR channel undergoes post-translational modifications.

The assembly of the nAChR subunits, their expression on the cell surface, their interaction with the cytoskeleton, the open/resting, and desensitized state of the channel, the conductance of the channel can be affected by such post-translational modifications thereby modifying the channel functioning.

In another embodiment, the at least one subunit of the nicotinic acetylcholine receptor (nAChR) channel of the functional nAChR channel is palmitoylated.

In another embodiment, the at least one subunit of the nicotinic acetylcholine receptor (nAChR) channel of the functional nAChR channel is glycosylated.

In another embodiment, the at least one subunit of the nicotinic acetylcholine receptor (nAChR) channel of the functional nAChR channel is nitrosylated.

In one embodiment, a variation may occur on the nAChR subunits at the site of phosphorylation, palmitoylation, glycosylation, and/or nitrosylation.

Technics to induce variations such as deletion, mutation or insertion are well known in the state of the art and to the skilled artisan.

In one embodiment, the functional nAChR channel of the invention comprises the nicotinic acetylcholine receptor alpha1 subunit encoded by the nucleic acid sequence as set forth in SEQ ID NO: 1 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof.

In another embodiment, the functional nAChR channel of the invention comprises the nicotinic acetylcholine receptor alpha5 subunit encoded by the nucleic acid sequence as set forth in SEQ ID NO: 5 or a variant thereof.

In another embodiment, the functional nAChR channel of the invention comprises the nicotinic acetylcholine receptor alpha6 subunit encoded by the nucleic acid sequence as set forth in SEQ ID NO: 6 or a variant thereof.

In another embodiment, the functional nAChR channel of the invention comprises the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor beta1 subunit encoded by the nucleic acid sequence as set forth in SEQ ID NO: 10 or a variant thereof.

In another embodiment, the functional nAChR channel of the invention comprises the nicotinic acetylcholine receptor beta2 subunit encoded by the nucleic acid sequence as set forth in SEQ ID NO: 11 or a variant thereof.

In one embodiment of the invention, the functional nAChR channel comprises at least one subunit of the nAChR channel and at least one of the chaperone proteins of a species from the phylum arthropod.

As with all ligand-gated ion channels, opening of the nAChR channel pore requires the binding of a chemical messenger. Several different terms are used to refer to the molecules that bind receptors, such as ligand.

In one embodiment, the functional nAChR channel of the invention is a functional selective cationic channel.

In one embodiment, said functional cationic channel is permeable for ions sodium ($Na^+$), potassium ($K^+$) or for some subunits calcium ($Ca^{2+}$).

In one embodiment, the functional nAChR channel of the invention comprises at least one subunit of nAChR encoded by the nucleic acid sequence consisting of SEQ ID NO: 1 to 9 or a variant thereof.

In one embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha2 subunit encoded by the nucleic acid sequence as set forth in SEQ ID NO: 2 or a variant thereof.

In another embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha3 subunit encoded by the nucleic acid sequence as set forth in SEQ ID NO: 3 or a variant thereof.

In another embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha4 subunit encoded by the nucleic acid sequence as set forth in SEQ ID NO: 4 or a variant thereof.

In another embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha5 subunit encoded by the nucleic acid sequence as set forth in SEQ ID NO: 5 or a variant thereof.

In another embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha6 subunit encoded by the nucleic acid sequence as set forth in SEQ ID NO: 6 or a variant thereof.

In another embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha9 subunit encoded by the nucleic acid sequence as set forth in SEQ ID NO: 9 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha2 subunit encoded by the nucleic acid sequence as set forth in SEQ ID NO: 2 or a variant thereof and the nicotinic acetylcholine receptor alpha3 subunit encoded by the nucleic acid sequence as set forth in SEQ ID NO: 3 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha2 subunit encoded by the nucleic acid sequence as set forth in SEQ ID NO: 2 or a variant thereof and the nicotinic acetylcholine receptor alpha4 subunit encoded by the nucleic acid sequence as set forth in SEQ ID NO: 4 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha2 subunit encoded by the nucleic acid sequence as set forth in SEQ ID NO: 2 or a variant thereof and the nicotinic acetylcholine receptor alpha5 subunit encoded by the nucleic acid sequence as set forth in SEQ ID NO: 5 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha2 subunit encoded by the nucleic acid sequence as set forth in SEQ ID NO: 2 or a variant thereof and the nicotinic acetylcholine receptor alpha6 subunit encoded by the nucleic acid sequence as set forth in SEQ ID NO: 6 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha2 subunit encoded by the nucleic acid sequence as set forth in SEQ ID NO: 2 or a variant thereof and the nicotinic acetylcholine receptor alpha7 subunit encoded by the nucleic acid sequence as set forth in SEQ ID NO: 7 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha2 subunit encoded by the nucleic acid sequence as set forth in SEQ ID NO: 2 or a variant thereof and the nicotinic acetylcholine receptor alpha8 subunit encoded by the nucleic acid sequence as set forth in SEQ ID NO: 8 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha2 subunit encoded by the nucleic acid sequence as set forth in SEQ ID NO: 2 or a variant thereof and the nicotinic acetylcholine receptor alpha9 subunit encoded by the nucleic acid sequence as set forth in SEQ ID NO: 9 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha3 subunit encoded by the nucleic acid sequence as set forth in SEQ ID NO: 3 or a variant thereof and the nicotinic acetylcholine receptor alpha4 subunit encoded by the nucleic acid sequence as set forth in SEQ ID NO: 4 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha3 subunit encoded by the nucleic acid sequence as set forth in SEQ ID NO: 3 or a variant thereof and the nicotinic acetylcholine receptor alpha5 subunit encoded by the nucleic acid sequence as set forth in SEQ ID NO: 5 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha3 subunit encoded by the nucleic acid sequence as set forth in SEQ ID NO: 3 or a variant thereof and the nicotinic acetylcholine receptor alpha6 subunit encoded by the nucleic acid sequence as set forth in SEQ ID NO: 6 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha3 subunit encoded by the nucleic acid sequence as set forth in SEQ ID NO: 3 or a variant thereof and the nicotinic acetylcholine receptor alpha7 subunit encoded by the nucleic acid sequence as set forth in SEQ ID NO: 7 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha3 subunit encoded by the nucleic acid sequence as set forth in SEQ ID NO: 3 or a variant thereof and the nicotinic acetylcholine receptor alpha8 subunit encoded by the nucleic acid sequence as set forth in SEQ ID NO: 8 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha3 subunit encoded by the nucleic acid sequence as set forth in SEQ ID NO: 3 or a variant thereof and the nicotinic acetylcholine receptor alpha9 subunit encoded by the nucleic acid sequence as set forth in SEQ ID NO: 9 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha4 subunit encoded by the nucleic acid sequence as set forth in SEQ ID NO: 4 or a variant thereof and the nicotinic acetylcholine receptor alpha5 subunit encoded by the nucleic acid sequence as set forth in SEQ ID NO: 5 or a variant thereof.

In one embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha4 subunit encoded by the nucleic acid sequence as set forth in SEQ ID NO: 4 or a variant thereof and the nicotinic acetylcholine receptor alpha6 subunit encoded by the nucleic acid sequence as set forth in SEQ ID NO: 6 or a variant thereof.

In one embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha4 subunit encoded by the nucleic acid sequence as set forth in SEQ ID NO: 4 or a variant thereof and the nicotinic acetylcholine receptor alpha7 subunit encoded by the nucleic acid sequence as set forth in SEQ ID NO: 7 or a variant thereof.

In one embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha4 subunit encoded by the nucleic acid sequence as set forth in SEQ ID NO: 4 or a variant thereof and the nicotinic acetylcholine receptor alpha8 subunit encoded by the nucleic acid sequence as set forth in SEQ ID NO: 8 or a variant thereof.

In one embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha4 subunit encoded by the nucleic acid sequence as set forth in SEQ ID NO: 4 or a variant thereof and the nicotinic acetylcholine receptor alpha9 subunit encoded by the nucleic acid sequence as set forth in SEQ ID NO: 9 or a variant thereof.

In one embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha5 subunit encoded by the nucleic acid sequence as set forth in SEQ ID NO: 5 or a variant thereof and the nicotinic acetylcholine receptor alpha6 subunit encoded by the nucleic acid sequence as set forth in SEQ ID NO: 6 or a variant thereof.

In one embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha5 subunit encoded by the nucleic acid sequence as set forth in SEQ ID NO: 5 or a variant thereof and the nicotinic acetylcholine receptor alpha7 subunit encoded by the nucleic acid sequence as set forth in SEQ ID NO: 7 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha5 subunit encoded by the nucleic acid sequence as set forth in SEQ ID NO: 5 or a variant thereof and the nicotinic acetylcholine receptor alpha8 subunit encoded by the nucleic acid sequence as set forth in SEQ ID NO: 8 or a variant thereof.

In one embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha5 subunit encoded by the nucleic acid sequence as set forth in SEQ ID NO: 5 or a variant thereof and the nicotinic acetylcholine receptor alpha9 subunit encoded by the nucleic acid sequence as set forth in SEQ ID NO: 9 or a variant thereof.

In one embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha6 subunit encoded by the nucleic acid sequence as set forth in SEQ ID NO: 6 or a variant thereof and the nicotinic acetylcholine receptor alpha7 subunit encoded by the nucleic acid sequence as set forth in SEQ ID NO: 7 or a variant thereof.

In one embodiment, the functional nAChR channel of the invention comprises the nicotinic acetylcholine receptor alpha 1 subunit encoded by the nucleic acid sequence as set forth in SEQ ID NO: 1 or a variant thereof and the nicotinic acetylcholine receptor alpha6 subunit encoded by the nucleic acid sequence as set forth in SEQ ID NO: 6 or a variant thereof and the nicotinic acetylcholine receptor alpha8 subunit encoded by the nucleic acid sequence as set forth in SEQ ID NO: 8 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha6 subunit encoded by the nucleic acid sequence as set forth in SEQ ID NO: 6 or a variant thereof and the nicotinic acetylcholine receptor alpha9 subunit encoded by the nucleic acid sequence as set forth in SEQ ID NO: 9 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha7 subunit encoded by the nucleic acid sequence as set forth in SEQ ID NO: 7 or a variant thereof and the nicotinic acetylcholine receptor alpha8 subunit encoded by the nucleic acid sequence as set forth in SEQ ID NO: 8 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof and the nicotinic acetylcholine receptor alpha7 subunit encoded by the nucleic acid sequence as set forth in SEQ ID NO: 7 or a variant thereof and the nicotinic acetylcholine receptor alpha9 subunit encoded by the nucleic acid sequence as set forth in SEQ ID NO: 9 or a variant thereof.

In one embodiment, the functional nAChR channel of the invention comprises the nicotinic acetylcholine receptor alpha1 subunit encoded by the nucleic acid sequence as set forth in SEQ ID NO: 1 or a variant thereof, the nicotinic acetylcholine receptor alpha8 subunit encoded by the nucleic acid sequence as set forth in SEQ ID NO: 8 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha3 subunit encoded by the nucleic acid sequence as set forth in SEQ ID NO: 3 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha4 subunit encoded by the nucleic acid sequence as set forth in SEQ ID NO: 4 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha5 subunit encoded by the nucleic acid sequence as set forth in SEQ ID NO: 5 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha6 subunit encoded by the nucleic acid sequence as set forth in SEQ ID NO: 6 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha3 subunit encoded by the nucleic acid sequence as set forth in SEQ ID NO: 3 or a variant thereof and the nicotinic acetylcholine receptor alpha4 subunit encoded by the nucleic acid sequence as set forth in SEQ ID NO: 4 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha3 subunit encoded by the nucleic acid sequence as set forth in SEQ ID NO: 3 or a variant thereof and the nicotinic acetylcholine receptor alpha5 subunit encoded by the nucleic acid sequence as set forth in SEQ ID NO: 5 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha3 subunit encoded by the nucleic acid sequence as set forth in SEQ ID NO: 3 or a variant thereof and the nicotinic acetylcholine receptor alpha6 subunit encoded by the nucleic acid sequence as set forth in SEQ ID NO: 6 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha3 subunit encoded by the nucleic acid sequence as set forth in SEQ ID NO: 3 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha3 subunit encoded by the nucleic acid sequence as set forth in SEQ ID NO: 3 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha3 subunit encoded by the nucleic acid sequence as set forth in SEQ ID NO: 3 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha4 subunit encoded by the nucleic acid sequence as set forth in SEQ ID NO: 4 or a variant thereof and the nicotinic acetylcholine receptor alpha5 subunit encoded by the nucleic acid sequence as set forth in SEQ ID NO: 5 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha4 subunit encoded by the nucleic acid sequence as set forth in SEQ ID NO: 4 or a variant thereof and the nicotinic acetylcholine receptor alpha6 subunit encoded by the nucleic acid sequence as set forth in SEQ ID NO: 6 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha4 subunit encoded by the nucleic acid sequence as set forth in SEQ ID NO: 4 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha4 subunit encoded by the nucleic acid sequence as set forth in SEQ ID NO: 4 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha4 subunit encoded by the nucleic acid sequence as set forth in SEQ ID NO: 4 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha5 subunit encoded by the nucleic acid sequence as set forth in SEQ ID NO: 5 or a variant thereof and the nicotinic acetylcholine receptor alpha6 subunit encoded by the nucleic acid sequence as set forth in SEQ ID NO: 6 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha5 subunit encoded by the nucleic acid sequence as set forth in SEQ ID NO: 5 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha5 subunit encoded by the nucleic acid sequence as set forth in SEQ ID NO: 5 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha5 subunit encoded by the nucleic acid sequence as set forth in SEQ ID NO: 5 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha6 subunit encoded by the nucleic acid sequence as set forth in SEQ ID NO: 6 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha6 subunit encoded by the nucleic acid sequence as set forth in SEQ ID NO: 6 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha6 subunit encoded by the nucleic acid sequence as set forth in SEQ ID NO: 6 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha7 subunit encoded by the nucleic acid sequence as set forth in SEQ ID NO: 7 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha7 subunit encoded by the nucleic acid sequence as set forth in SEQ ID NO: 7 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha2 subunit encoded by the nucleic acid sequence as set forth in SEQ ID NO: 2 or a variant thereof and the nicotinic acetylcholine receptor alpha8 subunit encoded by the nucleic acid sequence as set forth in SEQ ID NO: 8 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha4 subunit encoded by the nucleic acid sequence as set forth in SEQ ID NO: 4 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha5 subunit encoded by the nucleic acid sequence as set forth in SEQ ID NO: 5 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha6 subunit encoded by the nucleic acid sequence as set forth in SEQ ID NO: 6 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha4 subunit encoded by the nucleic acid sequence as set forth in SEQ ID NO: 4 or a variant thereof and the nicotinic acetylcholine receptor alpha5 subunit encoded by the nucleic acid sequence as set forth in SEQ ID NO: 5 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha4 subunit encoded by the nucleic acid sequence as set forth in SEQ ID NO: 4 or a variant thereof and the nicotinic acetylcholine receptor alpha6 subunit encoded by the nucleic acid sequence as set forth in SEQ ID NO: 6 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha4 subunit encoded by the nucleic acid sequence as set forth in SEQ ID NO: 4 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha4 subunit encoded by the nucleic acid sequence as set forth in SEQ ID NO: 4 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha4 subunit encoded by the nucleic acid sequence as set forth in SEQ ID NO: 4 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha5 subunit encoded by the nucleic acid sequence as set forth in SEQ ID NO: 5 or a variant thereof and the nicotinic acetylcholine receptor alpha6 subunit encoded by the nucleic acid sequence as set forth in SEQ ID NO: 6 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha5 subunit encoded by the nucleic acid sequence as set forth in SEQ ID NO: 5 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha5 subunit encoded by the nucleic acid sequence as set forth in SEQ ID NO: 5 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha5 subunit encoded by the nucleic acid sequence as set forth in SEQ ID NO: 5 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha6 subunit encoded by the nucleic acid sequence as set forth in SEQ ID NO: 6 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha6 subunit encoded by the nucleic acid sequence as set forth in SEQ ID NO: 6 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha6 subunit encoded by the nucleic acid sequence as set forth in SEQ ID NO: 6 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha7 subunit encoded by the nucleic acid sequence as set forth in SEQ ID NO: 7 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha7 subunit encoded by the nucleic acid sequence as set forth in SEQ ID NO: 7 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha3 subunit encoded by the nucleic acid sequence as set forth in SEQ ID NO: 3 or a variant thereof and the nicotinic acetylcholine receptor alpha8 subunit encoded by the nucleic acid sequence as set forth in SEQ ID NO: 8 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha5 subunit encoded by the nucleic acid sequence as set forth in SEQ ID NO: 5 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha6 subunit encoded by the nucleic acid sequence as set forth in SEQ ID NO: 6 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha5 subunit encoded by the nucleic acid sequence as set forth in SEQ ID NO: 5 or a variant thereof and the nicotinic acetylcholine receptor alpha6 subunit encoded by the nucleic acid sequence as set forth in SEQ ID NO: 6 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha5 subunit encoded by the nucleic acid sequence as set forth in SEQ ID NO: 5 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha5 subunit encoded by the nucleic acid sequence as set forth in SEQ ID NO: 5 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha5 subunit encoded by the nucleic acid sequence as set forth in SEQ ID NO: 5 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha6 subunit encoded by the nucleic acid sequence as set forth in SEQ ID NO: 6 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha6 subunit encoded by the nucleic acid sequence as set forth in SEQ ID NO: 6 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha6 subunit encoded by the nucleic acid sequence as set forth in SEQ ID NO: 6 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha7 subunit encoded by the nucleic acid sequence as set forth in SEQ ID NO: 7 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha7 subunit encoded by the nucleic acid sequence as set forth in SEQ ID NO: 7 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha4 subunit encoded by the nucleic acid sequence as set forth in SEQ ID NO: 4 or a variant thereof and the nicotinic acetylcholine receptor alpha8 subunit encoded by the nucleic acid sequence as set forth in SEQ ID NO: 8 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha5 subunit encoded by the nucleic acid sequence as set forth in SEQ ID NO: 5 or a variant thereof and the nicotinic acetylcholine receptor alpha6 subunit encoded by the nucleic acid sequence as set forth in SEQ ID NO: 6 or a variant thereof.

In another embodiment, the functional nAChR channel of the invention comprises the nicotinic acetylcholine receptor alpha5 subunit encoded by the nucleic acid sequence as set forth in SEQ ID NO: 5 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha5 subunit encoded by the nucleic acid sequence as set forth in SEQ ID NO: 5 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha5 subunit encoded by the nucleic acid sequence as set forth in SEQ ID NO: 5 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha5 subunit encoded by the nucleic acid sequence as set forth in SEQ ID NO: 5 or a variant thereof and the nicotinic acetylcholine receptor alpha6 subunit encoded by the nucleic acid sequence as set forth in SEQ ID NO: 6 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha5 subunit encoded by the nucleic acid sequence as set forth in SEQ ID NO: 5 or a variant thereof and the nicotinic acetylcholine receptor alpha6 subunit encoded by the nucleic acid sequence as set forth in SEQ ID NO: 6 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha5 subunit encoded by the nucleic acid sequence as set forth in SEQ ID NO: 5 or a variant thereof and the nicotinic acetylcholine receptor alpha6 subunit encoded by the nucleic acid sequence as set forth in SEQ ID NO: 6 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha5 subunit encoded by the nucleic acid sequence as set forth in SEQ ID NO: 5 or a variant thereof and the nicotinic acetylcholine receptor alpha7 subunit encoded by the nucleic acid sequence as set forth in SEQ ID NO: 7 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha5 subunit encoded by the nucleic acid sequence as set forth in SEQ ID NO: 5 or a variant thereof and the nicotinic acetylcholine receptor alpha7 subunit encoded by the nucleic acid sequence as set forth in SEQ ID NO: 7 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha5 subunit encoded by the nucleic acid sequence as set forth in SEQ ID NO: 5 or a variant thereof and the nicotinic acetylcholine receptor alpha8 subunit encoded by the nucleic acid sequence as set forth in SEQ ID NO: 8 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha6 subunit encoded by the nucleic acid sequence as set forth in SEQ ID NO: 6 or a variant thereof and the nicotinic acetylcholine receptor alpha7 subunit encoded by the nucleic acid sequence as set forth in SEQ ID NO: 7 or a variant thereof.

In another embodiment, the functional nAChR channel of the invention comprises the nicotinic acetylcholine receptor alpha6 subunit encoded by the nucleic acid sequence as set forth in SEQ ID NO: 6 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha6 subunit encoded by the nucleic acid sequence as set forth in SEQ ID NO: 6 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha6 subunit encoded by the nucleic acid sequence as set forth in SEQ ID NO: 6 or a variant thereof and the nicotinic acetylcholine receptor alpha7 subunit encoded by the nucleic acid sequence as set forth in SEQ ID NO: 7 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha6 subunit encoded by the nucleic acid sequence as set forth in SEQ ID NO: 6 or a variant thereof and the nicotinic acetylcholine receptor alpha7 subunit encoded by the nucleic acid sequence as set forth in SEQ ID NO: 7 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha6 subunit encoded by the nucleic acid sequence as set forth in SEQ ID NO: 6 or a variant thereof and the nicotinic acetylcholine receptor alpha8 subunit encoded by the nucleic acid sequence as set forth in SEQ ID NO: 8 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha7 subunit encoded by the nucleic acid sequence as set forth in SEQ ID NO: 7 or a variant thereof and the nicotinic acetylcholine receptor alpha8 subunit encoded by the nucleic acid sequence as set forth in SEQ ID NO: 8 or a variant thereof.

In another embodiment, the functional nAChR channel of the invention comprises the nicotinic acetylcholine receptor alpha7 subunit encoded by the nucleic acid sequence as set forth in SEQ ID NO: 7 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha7 subunit encoded by the nucleic acid sequence as set forth in SEQ ID NO: 7 or a variant thereof and the nicotinic acetylcholine receptor alpha8 subunit encoded by the nucleic acid sequence as set forth in SEQ ID NO: 8 or a variant thereof and the nicotinic acetylcholine receptor alpha9 subunit encoded by the nucleic acid sequence as set forth in SEQ ID NO: 9 or a variant thereof.

In another embodiment, the functional nAChR channel of the invention comprises the nicotinic acetylcholine receptor alpha8 subunit encoded by the nucleic acid sequence as set forth in SEQ ID NO: 8 or a variant thereof and In another embodiment, the functional nAChR channel of the invention comprises the nicotinic acetylcholine receptor alpha9 subunit encoded by the nucleic acid sequence as set forth in SEQ ID NO: 9 or a variant thereof.

In one embodiment, the functional nAChR channel of the invention comprises at least one subunit of nAChR encoded by the nucleic acid sequence consisting of SEQ ID NO: 1 to 9 or a variant thereof and at least one chaperone protein encoded by the nucleic acid sequence consisting of SEQ ID NO: 12 to 15 or a variant thereof.

In another embodiment, the functional nAChR channel of the invention comprises at least one subunit of nAChR encoded by the nucleic acid sequence consisting of SEQ ID NO: 1 to 9 or a variant thereof and at least one subunit of nAChR encoded by the nucleic acid sequence consisting of SEQ ID NO: 10 or 11 or a variant thereof.

In another embodiment, the functional nAChR channel of the invention comprises at least one subunit of nAChR encoded by the nucleic acid sequence consisting of SEQ ID NO: 1 to 9 or a variant thereof, at least one subunit of nAChR encoded by the nucleic acid sequence consisting of SEQ ID NO: 10 or 11 or a variant thereof and at least one chaperone protein encoded by the nucleic acid sequence consisting of SEQ ID NO: 12 to 15 or a variant thereof.

In one embodiment, the functional nAChR channel of the invention comprises the subunit of nAChR encoded by the nucleic acid sequence consisting of SEQ ID NO: 1 or a variant thereof.

In one embodiment, the functional nAChR channel of the invention comprises the subunit of nAChR encoded by the nucleic acid sequence consisting of SEQ ID NO: 1 or a variant thereof and the chaperone protein encoded by the nucleic acid sequence consisting of SEQ ID NO: 12 or a variant thereof.

In one embodiment, the functional nAChR channel of the invention comprises the subunit of nAChR encoded by the nucleic acid sequence consisting of SEQ ID NO: 1 or a variant thereof and the nicotinic acetylcholine receptor beta1 subunit encoded by the nucleic acid sequence as set forth in SEQ ID NO: 10 or a variant thereof.

In one embodiment, the functional nAChR channel of the invention comprises the subunit of nAChR encoded by the nucleic acid sequence consisting of SEQ ID NO: 5 or a variant thereof.

In one embodiment, the functional nAChR channel of the invention comprises the subunit of nAChR encoded by the nucleic acid sequence consisting of SEQ ID NO: 5 or a variant thereof and the chaperone protein encoded by the nucleic acid sequence consisting of SEQ ID NO: 12 or a variant thereof.

In one embodiment, the functional nAChR channel of the invention comprises the subunit of nAChR encoded by the nucleic acid sequence consisting of SEQ ID NO: 5 or a variant thereof and the chaperone protein encoded by the nucleic acid sequence consisting of SEQ ID NO: 13 to 15 or a variant thereof.

In one embodiment, the functional nAChR channel of the invention comprises the subunit of nAChR encoded by the nucleic acid sequence consisting of SEQ ID NO: 5 or a variant thereof and the nicotinic acetylcholine receptor beta1 subunit encoded by the nucleic acid sequence as set forth in SEQ ID NO: 10 or a variant thereof.

In another embodiment, the functional nAChR channel of the invention comprises the subunit of nAChR encoded by the nucleic acid sequence consisting of SEQ ID NO: 7 or a variant thereof and the chaperone protein encoded by the nucleic acid sequence consisting of SEQ ID NO: 12 or a variant thereof.

In another embodiment, the functional nAChR channel of the invention comprises the subunit of nAChR encoded by the nucleic acid sequence consisting of SEQ ID NO: 3 or a variant thereof.

In another embodiment, the functional nAChR channel of the invention comprises the subunit of nAChR encoded by the nucleic acid sequence consisting of SEQ ID NO: 3 or a variant thereof and the chaperone protein encoded by the nucleic acid sequence consisting of SEQ ID NO: 12 or a variant thereof.

In another embodiment, the functional nAChR channel of the invention comprises the subunit of nAChR encoded by the nucleic acid sequence consisting of SEQ ID NO: 3 or a variant thereof and the chaperone protein encoded by the nucleic acid sequence consisting of SEQ ID NO: 13 to 15 or a variant thereof.

In another embodiment, the functional nAChR channel of the invention comprises the subunit of nAChR encoded by the nucleic acid sequence consisting of SEQ ID NO: 3 or a variant thereof and the nicotinic acetylcholine receptor beta1 subunit encoded by the nucleic acid sequence as set forth in SEQ ID NO: 10 or a variant thereof.

In another embodiment, the functional nAChR channel of the invention comprises the subunit of nAChR encoded by the nucleic acid sequence consisting of SEQ ID NO: 3 or a variant thereof and the nicotinic acetylcholine receptor beta1 subunit encoded by the nucleic acid sequence as set forth in SEQ ID NO: 10 or a variant thereof and the chaperone protein encoded by the nucleic acid sequence consisting of SEQ ID NO: 12 or a variant thereof.

In another embodiment, the functional nAChR channel of the invention comprises the subunit of nAChR encoded by the nucleic acid sequence consisting of SEQ ID NO: 3 or a variant thereof and the nicotinic acetylcholine receptor beta1 subunit encoded by the nucleic acid sequence as set forth in SEQ ID NO: 10 or a variant thereof and the chaperone protein encoded by the nucleic acid sequence consisting of SEQ ID NO: 13 to 15 or a variant thereof.

In another embodiment, the functional nAChR channel of the invention comprises the subunit of nAChR encoded by the nucleic acid sequence consisting of SEQ ID NO: 6 or a variant thereof and the chaperone protein encoded by the nucleic acid sequence consisting of SEQ ID NO: 13 to 15 or a variant thereof.

In another embodiment, the functional nAChR channel of the invention comprises the subunit of nAChR encoded by the nucleic acid sequence consisting of SEQ ID NO: 6 or a variant thereof and the nicotinic acetylcholine receptor beta1 subunit encoded by the nucleic acid sequence as set forth in SEQ ID NO: 10 or a variant thereof.

In another embodiment, the functional nAChR channel of the invention comprises the subunit of nAChR encoded by the nucleic acid sequence consisting of SEQ ID NO: 9 or a variant thereof.

In another embodiment, the functional nAChR channel of the invention comprises the subunit of nAChR encoded by the nucleic acid sequence consisting of SEQ ID NO: 9 or a variant thereof and the chaperone protein encoded by the nucleic acid sequence consisting of SEQ ID NO: 12 or a variant thereof.

In another embodiment, the functional nAChR channel of the invention comprises the subunit of nAChR encoded by the nucleic acid sequence consisting of SEQ ID NO: 9 or a variant thereof and the nicotinic acetylcholine receptor beta1 subunit encoded by the nucleic acid sequence as set forth in SEQ ID NO: 10 or a variant thereof.

In another embodiment, the functional nAChR channel of the invention comprises the subunit of nAChR encoded by the nucleic acid sequence consisting of SEQ ID NO: 9 or a variant thereof and the nicotinic acetylcholine receptor beta1 subunit encoded by the nucleic acid sequence as set forth in SEQ ID NO: 10 or a variant thereof and the chaperone protein encoded by the nucleic acid sequence consisting of SEQ ID NO: 12 or a variant thereof.

Another object of the invention is an expression vector comprising at least one subunit of the nAChR channel of the invention or a variant thereof and able to express the channel in a suitable isolated cell transfected with said vector.

In one embodiment, the expression vector comprises the nicotinic acetylcholine receptor alpha1 subunit nucleic acid sequence as set forth in SEQ ID NO: 1 or a variant thereof.

In another embodiment, the expression vector comprises the nicotinic acetylcholine receptor alpha2 subunit nucleic acid sequence as set forth in SEQ ID NO: 2 or a variant thereof consisting of a nucleic acid sequence of less than 1930 base pairs (bp) and having at least 80, 85, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 2.

In another embodiment, the expression vector comprises the nicotinic acetylcholine receptor alpha3 subunit nucleic acid sequence as set forth in SEQ ID NO: 3 or a variant thereof.

In another embodiment, the expression vector comprises the nicotinic acetylcholine receptor alpha4 subunit nucleic acid sequence as set forth in SEQ ID NO: 4 or a variant thereof.

In another embodiment, the expression vector comprises the nicotinic acetylcholine receptor alpha5 subunit nucleic acid sequence as set forth in SEQ ID NO: 5 or a variant thereof.

In another embodiment, the expression vector comprises the nicotinic acetylcholine receptor alpha6 subunit nucleic acid sequence as set forth in SEQ ID NO: 6 or a variant thereof.

In another embodiment, the expression vector comprises the nicotinic acetylcholine receptor alpha7 subunit nucleic acid sequence as set forth in SEQ ID NO: 7 or a variant thereof.

In another embodiment, the expression vector comprises the nicotinic acetylcholine receptor alpha8 subunit nucleic acid sequence as set forth in SEQ ID NO: 8 or a variant thereof.

In another embodiment, the expression vector comprises the nicotinic acetylcholine receptor alpha9 subunit nucleic acid sequence as set forth in SEQ ID NO: 9 or a variant thereof.

In another embodiment, the expression vector comprises the nicotinic acetylcholine receptor beta1 subunit nucleic acid sequence as set forth in SEQ ID NO: 10 or a variant thereof.

In another embodiment, the expression vector comprises the nicotinic acetylcholine receptor beta2 subunit nucleic acid sequence as set forth in SEQ ID NO: 11 or a variant thereof.

Another object of the invention is an expression vector comprising at least one chaperone protein or a variant thereof of the invention and able to express said protein in a suitable isolated cell transfected with said vector.

In another embodiment, the expression vector comprises the chaperone protein resistance to inhibitors of cholinesterase 3 (ric-3) nucleic acid sequence as set forth in SEQ ID NO: 12 or variant thereof.

In another embodiment, the expression vector comprises the chaperone protein ER membrane protein complex-6 (emc-6) nucleic acid sequence as set forth as in SEQ ID NO: 13 or a variant thereof.

In another embodiment, the expression vector comprises the chaperone protein unc-50 nucleic acid sequence as set forth as in SEQ ID NO: 14 or a variant thereof.

In another embodiment, the expression vector comprises the chaperone protein unc-74 nucleic acid sequence as set forth as in SEQ ID NO: 15 or a variant thereof.

Another object of the invention is an expression vector comprising at least one subunit of a nAChR channel or a variant thereof and at least one chaperone protein or a variant thereof of the invention and able to express said channel and protein in a suitable isolated cell transfected with said vector.

Another object of the invention is an isolated cell transfected with at least one vector described here above wherein said cell expresses the at least one vector described here above.

The isolated transfected cells as used herein refer to eukaryote cell. Isolated transformed cells are well known in the state of the art for genetic engineering. These cells include in a non-limiting list: prokaryotic cells, eukaryotic cells, in particular bacteria such as *Escherichia coli, Bacillus sp.,* or yeasts such as *Saccharomyces cerevisiae,* fungus such as *Aspergillus niger,* insect cells such as SF9, SL1, or mammalian cells such as CHO, HEK293, PER-C6, amphibians cells such as for example Xenopus oocytes or oocytes of *Xenopus laevis.*

Technics for transfection or genetic engineering as used in the present application are well known by the person skilled in the art. These technics are described in Guide to Molecular Cloning Technics (Editors Berger SL and Kimmel AR 1987 Methods in Enzymology 152: 359-371).

Depending on the cell to be transfected, the person skilled in the art can determine the technology needed for the introduction of the nucleotide sequences of the present application in the selected isolated cell to be transfected and the vector used.

Technics to transfect isolated cells which comprise introducing the nucleic acid molecules into the isolated cells may involve the use of expression vectors which comprise the nucleic acid molecules. These expression vectors (such as plasmids and viruses; viruses including bacteriophage) can then be used to introduce the nucleic acid molecules into suitable isolated cells. For example, nAChR channel expression can be studied in Xenopus oocytes. DNA encoding the nAChR channel of the invention can be injected or transferred or transfected into the oocyte nucleus using a suitable vector, or mRNA encoding the said nAChR channel can be injected directly into the oocyte, in order to obtain expression of a functional nAChR channel in the oocyte.

Various methods are known in the art for introducing nucleic acid molecules into isolated cells. One method is microinjection, in which DNA is injected directly into the nucleus of cells through fine glass needles (or RNA is injected directly into the cytoplasm of cells). Alternatively, DNA can be incubated with an inert carbohydrate polymer (dextran) to which a positively charged chemical group (DEAE, for diethylaminoethyl) has been coupled. The DNA sticks to the DEAE-dextran via its negatively charged phosphate groups. These large DNA-containing particles stick in turn to the surfaces of cells, which are thought to take them in by a process known as endocytosis. Some of the DNA evades destruction in the cytoplasm of the cell and escapes to the nucleus, where it can be transcribed into RNA like any other gene in the cell. In another method, cells efficiently take in DNA in the form of a precipitate with calcium phosphate. In electroporation, cells are placed in a solution containing DNA and subjected to a brief electrical pulse that causes holes to open transiently in their membranes. DNA enters through the holes directly into the cytoplasm, bypassing the endocytotic vesicles through which they pass in the DEAE-dextran and calcium phosphate procedures (passage through these vesicles may sometimes destroy or damage DNA). DNA can also be incorporated into artificial lipid vesicles, liposomes, which fuse with the cell membrane, delivering their contents directly into the cytoplasm. In an even more direct approach, used primarily with plant cells and tissues, DNA is absorbed to the surface of tungsten micro projectiles and fired into cells with a device resembling a shotgun.

Several methods of microinjection, electroporation, and liposome fusion, have been adapted to introduce nucleic acids into cells and are well known by the skilled artisan. Further methods for introducing nucleic acid molecules into cells involve the use of viral vectors. Since viral growth depends on the ability to get the viral genome into cells, viruses have devised clever and efficient methods for doing it. One such virus widely used for protein production is an insect virus, baculovirus. Baculovirus attracted the attention of researchers because during infection, it produces one of its structural proteins (the coat protein) to spectacular levels. If a foreign gene were to be substituted for this viral gene, it too ought to be produced at high level. Baculovirus, like vaccinia, is very large, and therefore foreign genes must be placed in the viral genome by recombination. To express a foreign gene in baculovirus, the gene of interest is cloned in place of the viral coat protein gene in a plasmid carrying a small portion of the viral genome. The recombinant plasmid is cotransfected into insect cells with wild-type baculovirus DNA. At a low frequency, the plasmid and viral DNAs recombine through homologous sequences, resulting in the insertion of the foreign gene into the viral genome. Virus plaques develop, and the plaques containing recombinant virus look different because they lack the coat protein. The plaques with recombinant virus are picked and expanded. This virus stock is then used to infect a fresh culture of insect cells, resulting in high expression of the foreign protein. Various viral vectors have also been used to transfect cells, such as bacteriophage, vaccinia virus, adenovirus, and retrovirus.

Another object of the invention is an isolated cell transfected with at least one vector comprising the acid nucleic sequences as described here above.

Another object of the present invention is an isolated cell expressing the functional channel of the invention comprising the isolated amino acid sequence of at least one subunit of the nAChR channel or a variant thereof.

Another object of the present invention is an isolated cell expressing the functional channel of the invention comprising the isolated amino acid sequence of at least one subunit of the nAChR channel or a variant thereof and at least one chaperone protein of a species from the phylum arthropod or a variant thereof.

In one embodiment of the invention, the isolated amino acid sequence comprises the nicotinic acetylcholine receptor alpha1 subunit amino acid sequence as set forth in SEQ

ID NO: 16 or a variant thereof consisting of an amino acid sequence of at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 16.

In another embodiment of the invention, the isolated amino acid sequence comprises the nicotinic acetylcholine receptor alpha2 subunit amino acid sequence as set forth in SEQ ID NO: 17 or a variant thereof consisting of an amino acid sequence of at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 17.

In another embodiment of the invention, the isolated amino acid sequence comprises the nicotinic acetylcholine receptor alpha3 subunit amino acid sequence as set forth in SEQ ID NO: 18 or a variant thereof consisting of an amino acid sequence of at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 18.

In another embodiment of the invention, the isolated amino acid sequence comprises the nicotinic acetylcholine receptor alpha4 subunit amino acid sequence as set forth in SEQ ID NO: 19 or a variant thereof consisting of an amino acid sequence of at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 19.

In another embodiment of the invention, the isolated amino acid sequence comprises the nicotinic acetylcholine receptor alpha5 subunit amino acid sequence as set forth in SEQ ID NO: 20 or a variant thereof consisting of an amino acid sequence of at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 20.

In another embodiment of the invention, the isolated amino acid sequence comprises the nicotinic acetylcholine receptor alpha6 subunit amino acid sequence as set forth in SEQ ID NO: 21 or a variant thereof consisting of an amino acid sequence of at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 21.

In another embodiment of the invention, the isolated amino acid sequence comprises the nicotinic acetylcholine receptor alpha7 subunit amino acid sequence as set forth in SEQ ID NO: 22 or a variant thereof consisting of an amino acid sequence of at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 22.

In another embodiment of the invention, the isolated amino acid sequence comprises the nicotinic acetylcholine receptor alpha8 subunit amino acid sequence as set forth in SEQ ID NO: 23 or a variant thereof consisting of an amino acid sequence of at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 23.

In another embodiment of the invention, the isolated amino acid sequence comprises the nicotinic acetylcholine receptor alpha9 subunit amino acid sequence as set forth in SEQ ID NO: 24 or a variant thereof consisting of an amino acid sequence of at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 24.

In another embodiment of the invention, the isolated amino acid sequence comprises the nicotinic acetylcholine receptor beta1 subunit amino acid sequence as set forth in SEQ ID NO: 25 or a variant thereof consisting of an amino acid sequence of at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 25.

In another embodiment of the invention, the isolated amino acid sequence comprises the nicotinic acetylcholine receptor beta2 subunit amino acid sequence as set forth in SEQ ID NO: 26 or a variant thereof consisting of an amino acid sequence of at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 26.

Another object of the present invention is the isolated amino acid sequences or a variant thereof comprising the chaperone protein of a species from the phylum arthropod.

In one embodiment of the invention, the isolated amino acid sequence comprises the chaperone protein resistance to inhibitors of cholinesterase 3 (ric-3) amino acid sequence as set forth in SEQ ID NO: 27, or a variant thereof consisting of an amino acid sequence having less than 480 bp and of at least 80, 81, 82, 83, 84, 85, 86, 87, 88, 89, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 27.

In another embodiment of the invention, the isolated amino acid sequence comprises the chaperone protein emc-6 amino acid sequence as set forth in SEQ ID NO: 28 or variant thereof consisting of an amino acid sequence of at least 80, 81, 82, 83, 84, 85, 86, 87, 88, 89, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 28.

In another embodiment of the invention, the isolated amino acid sequence comprises the chaperone protein unc-50 amino acid sequence as set forth in SEQ ID NO: 29 or variant thereof consisting of an amino acid sequence of at least 80, 81, 82, 83, 84, 85, 86, 87, 88, 89, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 29.

In another embodiment of the invention, the isolated amino acid sequence comprises the chaperone protein unc-74 amino acid sequence as set forth in SEQ ID NO: 30 or variant thereof consisting of an amino acid sequence of at least 80, 81, 82, 83, 84, 85, 86, 87, 88, 89, 90, 91, 92, 93, 94, 95, 96, 97, 98, 99, 99.1; 99.2; 99.3; 99.4; 99.5; 99.6; 99.7; 99.8; 99.9% of identity with SEQ ID NO: 30.

Another object of the invention is an *in vitro* method to determine the effect of a test compound on the activity of a nAChR channel of an arthropod comprising:

a. contacting an isolated cell described herein with at least one test compound,
b. measuring the effect of said test compound on the nAChR channel activity, and

comparing said effect to the effect without test compound, thereby determining a modulating activity of said nAChR channel.

Another object of the invention is an *in vitro* method to determine the effect of a test compound on the activity of a nAChR channel of an arthropod comprising:

a. contacting an isolated cell described herein with at least one known nAChR channel modulator,
b. measuring the effect of said modulator on the nAChR channel activity,
c. contacting said isolated cell described herein with at least one test compound and said at least one modulator,
d. measuring the effect of said modulator and test compound on the nAChR channel activity, and

comparing the effect measured in d) to the effect measured in b), thereby determining a modulating activity of said nAChR channel.

Examples of known modulators of nAChR channel include but are not limited to: agonists, partial agonists, antagonists, allosteric modulators, positive allosteric modulators, negative allosteric modulators, non-competitive channel blockers, open channel blockers of the nAChR channel of the invention.

Examples of agonists include but are not limited to: ACh, nicotine, epibatidine, choline, levamisole, carbachol, methyridine, Psysostigmine, Galantamine, Neostigmine, Pyridostigmine, Varenicline, Dimethylphenylpiperazinium (DMPP), Muscarine, Oxotremorine, Bethanechol, Pilocarpine, clothianidin, Imidacloprid, acetamiprid, dinotefuran, derivate of nithiazine, nitenpyram, thiacloprid, thiamthoxan.

Examples of partial agonists include but are not limited to: acetylcholine binding protein (AChBP), benzoylcholine, GTS-21, choline.

Examples of antagonists include but are not limited to: α-bungarotoxin, dihydroxy-β-erythroidine, methyllycaconitine, α-conotoxin, α-tubocuranine, curare derivatives, Pancuronium, Vecuronium, Atracurium, mecamylamine, suxamethonium, Trimethaphan, Mecamylamine, Bupropion, Dextromethophan, Hexamethonium, Atropine, Tolterodine, Vedaclidine, Talsaclidine, Xanomeline, Ipatropium, Pirenzepine, Telenzepine, Darifenacin, N-2-chloroethyl-4-piperidinyl diphenylacetate (4-DAMP), Darifenacin, Solifenacin.

Examples of allosteric modulators include but are not limited to: neurosteroids.

Examples of positive allosteric modulators include but are not limited to: derivatives of (2-amino-5-keto)thiazole, desformylflustrabromine, galanthamine, codeine, serine, ivermectine.

Examples of negative allosteric modulators include but are not limited to: kyruneic acid, derivatives of methyllycaconitine.

Examples of non-competitive channel blockers include but are not limited to: piperidine derivative, mecamylamine, suxaméthonium, Trimethaphan, Mecamylamine, Bupropion, Dextromethophan, Hexamethonium.

In one embodiment of the invention, said known agonist of nAChR channel is applied for example from 1; 2; 3; 4; 5 ms to 10 seconds.

Another object of the invention is an *in vitro* method to determine the effect of a test compound on the activity of a nAChR channel of an arthropod comprising:

a. contacting an isolated cell described herein with a standard reference,
b. measuring the effect of said standard reference on the nAChR channel activity,
c. contacting said isolated cell described herein with at least one test compound and a standard reference,
d. measuring the effect of said standard reference and said at least one test compound on the nAChR channel activity, and

comparing the effect measured in d) to the effect measured in b), thereby determining a modulating activity of said nAChR channel.

In one embodiment, the standard reference is a compound known to modulate the nAChR channel activity of the invention.

Examples of a standard reference include but are not limited to: known agonists, known partial agonists, known antagonists, known allosteric modulators, known positive allosteric modulators, known negative allosteric modulators, known non-competitive channel blockers, known open channel blockers of nAChR channel of the invention.

In another embodiment of the invention, the standard reference is a compound known to modulate the nAChR channel activity of other species such as invasive species. Examples of invasive species include but are not limited to: introduced species, imported bees, species from the order *hymenoptera,* the family *Vespidae* and the subfamily *Vespinae* particularly Asian predatory wasp (*Vespa velutina*), spiders, parasites, endoparasites, ectoparasites from the class *Arachnida*, the family *Varroidae* such as: *Varroa jacobsoni, Varroa destrarctor*, *Varroa underwoodi*, *Varroa rindereri.*

In another embodiment, the standard reference comprises at least one compound known to modulate the nAChR channel of the invention.

In one embodiment, lethal dose of a test compound is added to the culture medium.

In another embodiment, sub-lethal dose of a test compound is added to the culture medium.

In another embodiment, minimal modulation dose of a test compound is added to the culture medium.

In another embodiment, the test compound is not toxic for pollinator insects and toxic for invasive species.

In another embodiment, the test compound is not toxic for *Apis mellifera* and toxic for *varroa.*

Examples of test compounds comprise phytosanitary product which include but are not limited to: insecticides, pesticides, drugs, veterinary drugs (such as veterinary drugs against parasites) that include in a non-limiting list derivatives or analogs of: neonicotinoid family compounds, acetamiprid, clothianidin (Poncho®), dinotefuran, imidacloprid (Gaucho®), derivate of nithiazine, nitenpyram, thiacloprid, and thiamthoxan (Cruiser®).

Examples of test compounds also comprise but are not limited to compounds already known to be toxic on other species such as invasive species as described here above.

Examples of test compounds comprise molecules which include but are not limited to: siRNAs, shRNAs, antisense oligonucleotide, ribozymes or aptamers, ligands, agonist or antagonist of nAChR, antibodies or fragments thereof, diabodies modulating activity of a nAChR of a pollinator insect. These test compounds can also include derivatives, analogs of: neurotoxins such as snake venoms $\alpha$-neurotoxins, toxins from plants, venom of insects, spiders, cones, mollusks, snails, and vertebrates such as snakes, scorpion toxins, or any other natural products used in integrated pest management.

In one embodiment of the invention, said test compound is already known to modulate the nAChR channel activity.

In another embodiment of the invention, said test compound is not yet known to modulate the nAChR channel activity.

In another embodiment of the invention, said test compound is a new chemical entity.

In one embodiment of the invention, said test compound increases or decreases the functional expression of nAChR channel in the isolated transfected cell.

In one embodiment of the invention, said test compound induces or reduces the nAChR channel expression at the cell surface.

In one embodiment, said test compound modulates nAChR channel activity via $\alpha$1, $\alpha$2, $\alpha$3, $\alpha$4, $\alpha$5, $\alpha$6, $\alpha$7, $\alpha$8, and/or $\alpha$9 subunit.

In one embodiment, said test compound modulates nAChR channel activity via $\alpha$1, $\alpha$2, $\alpha$3, $\alpha$4, $\alpha$5, $\alpha$6, $\alpha$7, $\alpha$8, and/or $\alpha$9 subunit and the chaperone protein.

In another embodiment, said test compound modulates nAChR channel activity via $\beta$1 or $\beta$2 subunit.

In another embodiment, said test compound modulates nAChR channel activity via $\beta$1 or $\beta$2 subunit and the chaperone protein.

In another embodiment, said test compound modulates nAChR channel activity via $\alpha$1, $\alpha$2, $\alpha$3, $\alpha$4, $\alpha$5, $\alpha$6, $\alpha$7, $\alpha$8, or $\alpha$9 subunit and $\beta$1 and/or $\beta$2 subunit.

In another embodiment, said test compound modulates nAChR channel activity via $\alpha$1, $\alpha$2, $\alpha$3, $\alpha$4, $\alpha$5, $\alpha$6, $\alpha$7, $\alpha$8, or $\alpha$9 subunit and $\beta$1 and/or $\beta$2 subunit and the chaperone protein.

nAChRs may exist in different interconvertible conformational states. Binding of an agonist stabilizes the open and desensitized states. Opening of the channel allows positively charged ions to move across it; in particular, sodium enters the cell and potassium exits. The net flow of positively-charged ions is inward.

The nAChR is a selective cation channel, meaning that several different positively charged ions can cross through. It is permeable to $Na^+$ and $K^+$, with some subunit combinations that are also permeable to $Ca^{2+}$.

In one embodiment, said test compound modulates the activity of the nAChR channel of the invention.

In one embodiment, said test compound modifies the gating kinetics of the nAChR channel.

In another embodiment, said test compound modulates (increases or decreases) the hyperpolarization due to the activity of the nAChR channel of the invention.

In another embodiment, said test compound modulates (increases or decreases) the depolarization due to the activity of the nAChR channel of the invention.

In another embodiment, said test compound modulates (increases or decreases) the ion uptake by the nAChR channel of the invention.

In another embodiment, said test compound induces deleterious neuronal hyperexcitability.

In another embodiment, said test compound modulates (increases or decreases) the duration of closed states.

In another embodiment, said test compound modulates (increases or decreases) the duration of open states.

In another embodiment, said test compound modulates (increases or decreases) the activation and/or desensitization kinetics of the nAChR channel of the invention.

In another embodiment, said test compound affinity is modulated upon channel opening.

In another embodiment, said test compound affinity is modulated upon channel closed-state.

In another embodiment, said test compound is dependent of the functional states of the nAChR channel with preferred affinity for the close and/or open and/or desensitized states.

In another embodiment, said test compound modulates (increases or decreases) the sorting, the targeting or the translocation of the nAChR channel of the invention.

In another embodiment, said test compound modulates (increases or decreases) the stability of the nAChR channel of the invention.

In another embodiment, said test compound modulates the subunits assembly of the nAChR channel of the invention.

In one embodiment, the method of the invention is a cell-based assay.

In one embodiment, the method of the invention is a high-throughput assay.

In another embodiment, the method of the invention is an electrophysiological method.

In another embodiment, the method of the invention is a fluorometry or luminometry method.

The methods of the invention can be in conventional laboratory format or adapted for high throughput. The term "high throughput" (HTS) refers to an assay design that allows easy analysis of multiple samples simultaneously, and capacity for robotic manipulation. Another desired feature of high throughput assays is an assay design that is optimized to reduce reagent usage, or minimize the number of manipulations in order to achieve the analysis desired.

Methods for measuring the effect of a test compound on a nAChR channel are well known in the state of the art. For example, the person skilled in the art knows that electrophysiological measurements in isolated transfected cells expressing functional nAChR channel can be used to test a compound.

In one embodiment, measuring the effect of a test compound comprises measuring the activation kinetics of the nAChR channel of the invention.

In another embodiment, measuring the effect of a test compound comprises measuring the deactivation kinetics of the nAChR channel during removal of the agonist.

In another embodiment, measuring the effect of a test compound comprises measuring the desensitization kinetics of the nAChR channel of the invention.

In another embodiment, measuring the effect of a test compound comprises measuring the current amplitude of the nAChR channel of the invention.

In another embodiment of the invention, the *in vitro* method can be used to screen a compound that inhibits, prevents or stops the action of a compound known to intoxicate an arthropod through its nAChR channel subunits.

In one embodiment of the invention, the *in vitro* method determine the effect of a test compound to provide modulation reference patterns and databases of modulation reference patterns for a wide range of molecules. The reference patterns are then used for the identification and classification of test molecules. Evaluation of test compounds may be used to achieve different results.

Methods for the classification of compounds according to the spectral density signature of evoked changes in cellular electric potential are known to the person skilled in the art; see, e.g., US patent No 6,377,057. Thus, compounds are classified according to their effect on ion channels, changes in membrane potential and ionic currents, and the frequency content of action potentials that the compound(s) evoke in excitable cells. The spectral density changes of such evoked membrane potential or action potential are a characteristic for each channel type that is modulated by the test compound. A pattern of spectral changes in membrane potential is determined by contacting a responsive cell with a test compound, and monitoring the membrane potential or ionic currents over time. These changes correlate with the effect of that compound, or class of compounds, on the ion channels of the responding cell. This pattern of spectral changes provides a unique signature for the compound, and provides a useful method for characterization of channel modulating agents. The effect of a compound on ion channels, and on the action potential of a living cell, can provide useful information about the classification and identity of the compound. Methods and means for extracting such information are of particular interest for the analysis of molecules, with specific applications in pharmaceutical screening, drug discovery, environmental monitoring, biowarfare detection and classification, and the like. Examples of whole cell-based biosensors are described in Gross et al., Biosensors and Bioelectronics 10 (1995), 553-567.

Another object of the invention is an *in vitro* method to determine the toxicity of a test compound on an arthropod comprising:

a. contacting an isolated cell described herein with at least one test compound,
b. measuring the effect of said at least one test compound on the nAChR channel activity, and

comparing the effect to the effect without test compound, thereby determining the toxicity on said nAChR channel.

In one embodiment of the invention, a test compound is considered as toxic once a modulating effect is measured on the nAChR channel of the invention.

In one embodiment, a test compound is considered as toxic once it modifies the gating kinetics of the nAChR channel of the invention.

In another embodiment, a test compound is considered as toxic once it modulates (increases or decreases) the amplitude of ion current of the nAChR channel of the invention.

In another embodiment, a test compound is considered as toxic once it modulates (increases or decreases) the desensitized state.

In another embodiment, a test compound is considered as toxic once it alters the activation kinetics or voltage dependence of either channel activation or desensitization.

In another embodiment, a test compound is considered as toxic once it induces deleterious neuronal hyperexcitability.

In another embodiment, a test compound is considered as toxic once its affinity is modulated (increased or decreased) upon channel opening.

In another embodiment, a test compound is considered as toxic once it modulated channel closed-state.

In another embodiment, a test compound is considered as toxic once it is dependent of the functional states of the nAChR channel with preferred affinity for the close and/or open and/or desensitized states.

In another embodiment, a test compound is considered as toxic once the sorting, the targeting or the translocation of the nAChR channel of the invention is modified.

In another embodiment, a test compound is considered as toxic once the stability of the nAChR channel of the invention is modified.

In another embodiment, a test compound is considered as toxic once the subunits assembly of nAChR channel of the invention is modified.

In one embodiment, the test compound is not toxic for the pollinator insects of the invention.

In another embodiment, the test compound is toxic for the pollinator insects of the invention.

In another embodiment, the test compound is toxic for invasive species while said test compound is not toxic for the pollinator insects of the invention.

In another embodiment, the test compound is toxic for insects spreading diseases to a human subject. Examples of such insects are mosquitoes. Examples of such diseases include but are not limited to: malaria, dengue fever, Japanese encephalitis, Ross River virus infection, Barmah Forest virus infection, Murray Valley encephalitis, yellow fever, West Nile Virus.

Another object of the invention is an *in vitro* method for screening compounds that modulate the nAChR channel activity of an arthropod that comprises:

a. contacting an isolated cell described herein with at least one test compound,
b. measuring the effect of said at least one test compound on the nAChR channel activity, and

comparing said effect to the effect without test compound, thereby determining a modulation of activity of said nAChR channel and classifying said compound as an agonist, a partial agonist, antagonist, positive allosteric modulator, negative allosteric modulator, non-competitive channel blocker, open channel blocker.

Another object of the invention is an *in vitro* method for screening compounds that modulate the nAChR channel activity of an arthropod that comprises:

a. contacting an isolated cell described herein with at least one agonist,
b. measuring the effect of said agonist on the nAChR channel activity, and
c. contacting the isolated cell described herein with said agonist compound and at least one test compound,
d. measuring the effect of said agonist and said test compound on the nAChR channel activity, and

comparing the effect measured in d) to the effect measured in b), thereby determining a modulation of activity of said nAChR channel and classifying said compound as an agonist, a partial agonist, antagonist, positive allosteric modulator, negative allosteric modulator, non-competitive channel blocker, open channel blocker.

Another object of the invention is an *in vitro* method to detect the binding of a test compound on the nAChR channel of an arthropod comprising:

a. contacting an isolated cell described herein with at least one test compound, and
b. measuring the binding of said test compound on the nAChR channel of a pollinator insect.

In one embodiment, the method of the invention determines the ability of a test compound to bind to the nAChR channel of the invention.

Tests to monitor binding of a ligand to the nAChR channel of the invention are well known in the state of the art. Such tests include but are not limited to: radioligand binding test ([$^3$H]-epibatidine and [$^{125}$I]-a-bungarotoxine).

Another object of the invention is a kit comprising the vector of the invention or the isolated cell expressing the nAChR channel of the invention and reagents for conducting any one of the above described methods of the invention.

Optionally the kit can comprise culture medium, recombinant nucleic acid sequences, reagents, standard reference compounds, etc. Such kit would typically comprise a compartmentalized carrier suitable to hold in close confinement at least one container. The carrier would further comprise reagents useful for performing said methods. The carrier may also contain a means for detection such as labeled enzyme substrates or the like. Instructions can be provided to detail the use of the components of the kit, such as written instructions, video presentations, or instructions in a format that can be opened on a computer (e.g. a diskette or CD-ROM disk). These instructions indicate, for example, how to use the cells to screen test compounds of interest (such as ionotropic drugs).

In addition, the present invention relates to an apparatus and array, respectively, for use in the methods and assays of the present invention described herein. For example, a cell-potential measurement apparatus having a plurality of microelectrodes and which may be used and/or adapted in accordance with the teaching of the present invention is described in European patent application EP 0 689 051.

Furthermore, international application WO98/54294 describes an apparatus and method for monitoring cells and a method for monitoring changes in cells upon addition of a compound to the cell's environment, comprising a device which includes an array of microelectrodes disposed in a cell culture chamber, upon which array a portion of cells adhere to the surfaces of the microelectrodes. The diameter of the cells is larger than the diameters of the microelectrodes. A voltage signal is applied across each of the microelectrodes and a reference electrode. Detection and monitoring of the signals resulting from the application of the voltage signal provides information regarding the electrical characteristics of the individual cells, including impedance (combined cell membrane capacitance and conductance), action potential parameters, cell membrane capacitance, cell membrane conductance, and cell/substrate seal resistance.

The present invention also relates to an automated Voltage-Clamp Screening System for Xenopus oocytes comprising HiClamp robot, USB video camera, HiClamp software, accessories, and consumables which is a well-known technics in the state of the art.

The HiClamp is a fully-automated all-in-one solution for high-throughput functional secondary screening of test compounds based on the standard Xenopus expression system. The HiClamp functionality is based on the use of a novel system in which the oocyte is exposed to a test solution by moving it physically into the solution of interest, whereas in a standard system the solution is applied on the cell.

## BRIEF DESCRIPTION OF THE DRAWINGS

**Figure 1** represents photographs representing tissue expression of Am-nAChR chaperones in *Apis Mellifera* (A: Antenna, L: leg, T: Thorax, B: Brain). These chaperone proteins are expressed ubiquitously in excitable muscles and neurons.

**Figure 2** represents histograms (A) and current traces recorded (B) on oocytes showing the role of chaperone proteins ric3, unc74, unc50 and emc6 in the functional expression of two types of nAChR composed of different $\alpha$ subunits.

**Figure 3** represents ACh dose-response curves for different combinations of nAChR subunits producing functional receptors. A-B. Current traces recorded on oocytes expressing the nAChR$\alpha$5 (A, with unc74+unc50+emc6) or the nAChR$\alpha$7 (B, with ric3) subunits in response to different concentrations of ACh. C. Dose-response curves for ACh on the combination of subunits containing the nAChR$\alpha$5 or the nAChR$\alpha$7 subunit. Note the higher sensitivity of the receptors containing the nAChR$\alpha$7.

**Figure 4** represents pharmacological sensitivity of honeybee nicotinic receptors Am-nAChR$\alpha$5 and Am-nAChR$\alpha$7. A. Current traces showing the effects of ACh and clothianidin (Poncho®, Bayer) at 500 $\mu$M on the two types of receptors. B. Dose-response curves of clothianidin on these two types of receptors. C. Dose-response curves of ACh and clothianidin on Am-nAChR$\alpha$7 recorded on the same oocyte demonstrating that clothianidin is a less effective agonist than ACh. Please note the differential sensitivity of these two types of receptors, Am-nAChRα5 being completely insensitive to clothianidin.

**EXAMPLES**

The present invention is further illustrated by the following examples.

Example 1: Cloning, sequencing and characterization of the nAChR subunits α1-9 and nAChRβ1-2, and the chaperone proteins ric3, emc-6, unc50 and unc74

The structure of the nAChR is well conserved between species. They belong to the cysteine-loop family of ligand-gated ion channels. In insects they are supposed to be constituted, by homology with vertebrates, by the assembly of 5 homologous subunits (pentameric structure) that form a membrane complex with an axial pseudo-symmetry and a central ion channel pore. Binding of acetylcholine to the receptor produces a rapid opening of a pore able to select small cations for entry. Each subunit has 4 transmembrane segments, M1-M4, with the M2 segment being constitutive of the channel pore, and a large extracellular N-terminal region forming a loop thanks to a disulfide bridge between two conserved cysteines distant by 13 amino-acids. The N-terminal region of two adjacent subunits forms the ACh binding-pocket and participates, with the M3-M4 helices to the assembly and the kinetics of the receptors. The subunits that possess this dicystein loop are called α subunits, while those without are called non- α or β subunits. In honeybee, 9 genes are encoding for α subunits (α1-9) while the β subunits are encoded by only two genes. Thus, honeybee, like other insects (nAChR are cloned in 6 insect species) possesses a relatively small family (11 genes) of nAChR compare to vertebrates (15 to 30 genes) (Jones & Sattelle, 2006 Genome Res. 16, 1422-1430; Whitfield et al., 2002 Genome Res. 12, 555-566). Which subunit(s) form the functional nAChR that can be activated in different bee neurons is actually poorly known (but see (Dupuis et al., 2011 J Neurophysiol. 106, 1604-1613). If the sequence of each of these honeybee subunits has been isolated, their functional characterization in expression system such as Xenopus oocytes (or cell lines) has never been done. In other insects also, expression of nicotinic receptors has been done only using heteromers that include mammalian subunits, and receptors containing only insects subunits are reported to express at very low level only.

Using sequence homology with drosophila, the 11 genes coding for honeybee nAChR subunits have been classified in 7 groups, with one group (including the α5-α6-α7 subunits) beholding a common ancestors with the vertebrate subunits sensitive to bungarotoxine, while the 6 other groups (α1, α2, α3, α4, α8, β1) have a distinct ancestor. The α9 and β2 subunits are clearly distinct from these 7 groups. Subunits can oligomerize in oligo- or hetero-pentamers. These combinations, as well as of alternative splicing and RNA editing produce, despite the small number of genes, a very wide diversity of the receptors with different functional properties.

In bee brain these genes are expressed in the structures responsible for the processing of sensory, visual and olfactory information including the optic lobes, antennal neurons, the antennal lobes, and the mushroom bodies.

In vertebrate, bungarotoxine-sensitive (mostly homo-pentamers) and -insensitive (mostly heteropentamers) receptors have been distinguished. In insects, the fact that these subunits cannot be expressed without vertebrate subunits makes this distinction hard to make. However, heteromeric channels sensitive and insensitive to bungarotoxine have been detected. These two types of sensitivity can also been found in native neurons, but these studies are still very sparse. Imidaclopride (Gaucho®) and 6 of its derivatives have an important potency as nAChR agonist and a good selectivity for insect receptors.

Up to now, no honeybee nAChR have been expressed in a heterologous expression system to perform a precise characterization of the different subunits combinations. However, it has recently been shown, in *C-elegans,* but also in insects and in vertebrates, that other transmembrane proteins (from intracellular membrane) such as ric3, unc50, unc74 or emc-6 may play an important role for the targeting and the recycling of the nAChR at the membrane.

Our analysis of the *Apis mellifera* genome identified 11 genes for α (α 1-9) and β (β1 and β2) subunits of nAChR. Sequence homology among these different subunits are between 25 to 70% as a function of their belonging to a specific group. These sequences are ~99% homologous to those identified from the annotated genome of *Apis mellifera* in the public databases (Jones et al., 2006; Elsik et al., BMC Genomics. 2014 Jan 30;15:86). We have also identified and cloned in *Apis mellifera* 4 chaperone proteins homologous to the ric3, unc50, unc74 and emc-6 proteins of *C-elegans.* These honeybee chaperones show 13, 38, 40 and 30% homology with their homologues in *C-elegans,* respectively, and 23% of homology are calculated between the honeybee and the drosophila ric3, the only chaperone isolated in drosophila. None of these chaperones was identified in honeybee before this work.

We have cloned, sequenced and characterized the nAChR subunits α 1-9 and nAChR subunits β1-2. We have also cloned honeybee ric3, emc-6, unc50 and unc74 and analyze their homology with *C-elegans* and drosophila subunits. We demonstrate expression of these subunits in different bee muscular and nervous tissues (such as *Apis mellifera* tissues, Figure 1).

Example 2: Pharmacology and sensitivity of nAChR channel in combination with chaperone protein to insecticides

We also show that functional expression in Xenopus laevis oocytes of these subunits are under the control of the chaperones proteins in the nAChR subunit-specific manner (Figure 2). For example, ric3 (from *C.elegans*, the nucleic acid sequence being SEQ ID NO: 64 and the amino acid sequence being SEQ ID NO: 65, or honeybee) is clearly necessary to get a reliable expression of the nAChR α7 subunit and a response to ACh, while for the nAChRα5 subunit a cocktail of emc6, unc50 and unc74 chaperone proteins improves expression with response amplitude between 0.1 to several μA for ACh concentration between 1 to 1000μM (Figure 2). In the case of the nAChRα3 subunits, expression can be obtain either alone, or in combination with different cocktails of chaperone or with the vertebrate β2 nAChR subunits. Each of these combinations has a specific affinity for ACh. Finally using the α5 and α7 subunits we show by constructing dose-response curves the specific response of each of these subunits to ACh (Figure 3). These two subunits have also a specific pharmacology and sensitivity to insecticides, with the α5 subunit being insensitive to clothianidin (the active substance of Poncho®), while the α7 subunit was half-activated for doses close to 10 μM (Figure 4). These data demonstrate that toxicological tests need to be realized on more than one combination of nAChR subunits to be relevant.

The use of this technology (perfusion of different chemical agents on different combination of nAChR subunits identified on honeybee neuron or muscle) may therefore bring precise and important information on the toxicity of these products on bees (as for example *Apis mellifera,* but also *Apis cerana, Apis dorsata* or *Apis florea*).

To our knowledge, while the nAChR sequences are known (Jones et al., 2006, Elsik et al., 2014), no heterologous expression of these subunits have been reported. Moreover, while the genes coding for ric3, emc-6, unc50 and unc74 have been functionally characterized in *C-elegans* and shown to play a role in nAChR expression (Boulin et al., Proc Natl Acad Sci USA. 2008 Nov 25; 105(47): 18590-18595), no data are available on the role of their homologues in honeybee. As a consequence, up to now, no cellular model were available to test the toxicity of environmental stressor on bee nAChR without the use of either whole animals (adult or larva), or primary culture of muscle cells or neurons.

The fact that different combinations of *Apis mellifera* nAChR subunits can now be expressed in heterologous system and used to test the toxicity of chemical opens the way to more standardized and automated methods to set-up toxicological tests but also for differential screening new insecticide safe for pollinators.

***Materials and Methods***

Total RNAs purification from bees.

Bees have been anesthetized at 4°C. Whole brains, legs, antennas and abdomens, have been rapidly dissected under a binocular and then stored on ice. Total RNAs have been purified from whole brains with the RNeasy Mini Kit (Qiagen). For the other tissues and for larva (Day7 and Day 18), total RNAs have been purified with the RNAwiz reagent (Life Technologies). The tissues have been homogenized in 600 μl of RLT Buffer (Qiagen) or 4ml of RNAwiz (Life Technologies). The remaining of the procedure has been carried on following the instructions of the manufacturers. The final concentration of total RNAs have been determined by using a spectrophotometer (Biophotometer, Eppendorf), and RNA integrity has been checked by running an aliquot of RNA on an agarose gel. Total RNAs have then been stored at -80°C until use.

Reverse transcription and PCR amplification.

The first strand of the cDNAs has been obtained with the SuperScript II Reverse Transcriptase (Life Technologies) and with Oligo(dT)18 primers 1 μl of the obtained cDNAs has been subjected to PCR amplification with the Herculase II fusion polymerase (Agilent Technologies). The temperature and the duration of the denaturation step (92-98°C, 20-60s), of the hybridization step (55-65°C, 20-60s) and of the extension step (68 or 72°C, 30s-3mn), together with the final concentration of DMSO (0 to 8%) of the PCR reactions have been empirically optimized for each couple of primers. The PCRs displayed on the Figure 1 (Am-ric3, Am-unc50, Am-unc74, Am-emc6 have been obtained with the following couple of primers amric3-008S and amric3-005AS, amunc50-001S and amunc50-002AS, amunc74-001S and amunc74-002AS and amemc6-001S and amemc6-002AS, respectively (Table 1). When appropriate, amplified fragments have been purified from agarose gel with the NucleoSpin Extract II kit (Macherey-Nagel), phosphorylated with the T4 kinase (Life Technologies) and ligated into the expression vector pCMV-PL10 which is a modified version of pcDNA3.1(+) (Life Technologies) with the sequence of the Alfalfa Mosaic Virus (AMV) immediately before the start codon and the 3'-UTR sequence of the Xenopus β-globin gene after the stop codon to boost expression in Xenopus oocytes. Recombinant plasmids have been sequenced on both strands by Eurofins MWG Operon.

Amplification of the sequence of the Am-nAChR$\alpha$1 subunit.

A PCR carried out using the primer amnachra1-001S (SEQ ID NO: 31) and amnachra1-002AS (SEQ ID NO: 32) has allowed the identification of the nucleotides 1 to 1806 (SEQ ID NO: 1).

Amplification of the sequence of the Am-nAChR $\alpha$2 subunit.

A PCR carried out using the primer amnachra2-001S (SEQ ID NO: 33) and amnachra2-002AS (SEQ ID NO: 34) has allowed the identification of the nucleotides 1 to 1626 (SEQ ID NO: 2).

Amplification of the sequence of the Am-nAChR $\alpha$3 subunit.

A PCR carried out using the primer amnachra3-001S (SEQ ID NO: 35) and amnachra3-002AS (SEQ ID NO: 36) has allowed the identification of the nucleotides 1 to 1704 (SEQ ID NO: 3).

Amplification of the sequence of the Am-nAChR $\alpha$4 subunit.

A PCR carried out using the primer amnachra4-001S (SEQ ID NO: 37) and amnachra4-002AS (SEQ ID NO: 38) has allowed the identification of the nucleotides 1 to 1710 (SEQ ID NO: 4).

Amplification of the sequence of the Am-nAChR $\alpha$5 subunit.

A PCR carried out using the primer amnachra5-003S (SEQ ID NO: 39) and amnachra5-002AS (SEQ ID NO: 40) has allowed the identification of the nucleotides 1 to 1446 (SEQ ID NO: 5).

Amplification of the sequence of the Am-nAChR $\alpha$6 subunit.

A PCR carried out using the primer amnachra6-001S (SEQ ID NO: 41) and amnachra6-002AS (SEQ ID NO: 42) has allowed the identification of the nucleotides 1 to 1590 (SEQ ID NO: 6).

Amplification of the sequence of the Am-nAChR $\alpha$7 subunit.

A PCR carried out using the primer amnachra7-001S (SEQ ID NO: 43) and amnachra7-002AS (SEQ ID NO: 44) has allowed the identification of the nucleotides 1 to 1668 (SEQ ID NO: 7).

Amplification of the sequence of the Am-nAChR $\alpha$8 subunit.

A PCR carried out using the primer amnachra8-001S (SEQ ID NO: 45) and amnachra8-002AS (SEQ ID NO: 46) has allowed the identification of the nucleotides 1 to 1614 (SEQ ID NO: 8).

Amplification of the sequence of the Am-nAChR $\alpha$9 subunit.

A PCR carried out using the primer amnachra9-001S (SEQ ID NO: 47) and amnachra9-002AS (SEQ ID NO: 48) has allowed the identification of the nucleotides 1 to 1296 (SEQ ID NO: 9).

Amplification of the sequence of the Am-nAChR$\beta$1 subunit.

A PCR carried out using the primer amnachrb1-001S (SEQ ID NO: 49) and amnachrb1-002AS (SEQ ID NO: 50) has allowed the identification of the nucleotides 1 to 1563 (SEQ ID NO: 10).

Amplification of the sequence of the Am-nAChR$\beta$2 subunit.

A PCR carried out using the primer amnachrb2-001S (SEQ ID NO: 51) and amnachrb2-007AS (SEQ ID NO: 52) has allowed the identification of the nucleotides 1 to 1284 (SEQ ID NO: 11).

Amplification of the sequence of Cele ric3.

A PCR carried out using the primer ceric3-001S (SEQ ID NO: 53) and ceric3-002AS (SEQ ID NO: 54) has allowed the identification of the nucleotides 1 to 1137 (SEQ ID NO: 64)

Amplification of the sequence of am ric3a.

A PCR carried out using the primer amric3-008S (SEQ ID NO: 55) and amric3-007AS (SEQ ID NO: 63) has allowed the identification of the nucleotides 1 to 1365 (SEQ ID NO: 12).

Amplification of the sequence of am emc6.

A PCR carried out using the primer emc6-001S (SEQ ID NO: 57) and emc6-002AS (SEQ ID NO: 58) has allowed the identification of the nucleotides 1 to 342 (SEQ ID NO: 13).

Amplification of the sequence of am unc50.

A PCR carried out using the primer amunc50-001S (SEQ ID NO: 59) and amunc50-002AS (SEQ ID NO: 60) has allowed the identification of the nucleotides 1 to 804 (SEQ ID NO: 14).

Amplification of the sequence of am unc74.

A PCR carried out using the primer amunc74-001S (SEQ ID NO: 61) and amunc74-002AS (SEQ ID NO: 62) has allowed the identification of the nucleotides 1 to 1296 (SEQ ID NO: 15).

Oligonucleotides/primers.

Oligonucleotides have been designed thanks to the public library (NCBI) that contains the sequences of the contigs of genomic DNA and the assembled cDNAs of *Apis mellifera.* Lyophilized oligonucleotides (Eurofins MWG Operon) have been resuspended at 100 μM in distilled water, aliquoted at 10 μM and stored at -20°C until use.

Table 1: List of primers used to identify nAChR-gated ion channel subunits and their chaperones.

| SEQ ID NO: | Name | Sequence (5' to 3') |
|---|---|---|
| 55 | amric3-008S | CATGGCTGAAATAACAGATTTCG |
| 56 | amric3-005AS | CATGTGGAGGTGTACGTCGTTCTTG |
| 59 | amunc50-001S | CATGAAATATTCTACATCACCACCAGTAAG |
| 60 | amunc50-002AS | TATTCAAACAACTCGATAATGATAAAATTCC |
| 61 | amunc74-001S | CATGGTAATAATAACGAAATTGATGTTTATTG |
| 62 | amunc74-002AS | CAATTATTCTAATCTTTCTTCATATGATTTG |
| 57 | amemc6-001S | CATGTTGGGAAAGATTAAAACAAAACAAG |
| 58 | amemc6-002AS | CTTTTAATCAGTATACATGTACCATTCCATAT |
| 31 | amnachra1-001S | CATGGCGACGGCCATTTCCTGTC |
| 32 | amnachra1-002AS | CTAGTCCTCCTCGGGACCCATG |
| 33 | amnachra2-001S | CATGATACTCCAGACGATCATCTTGATCC |
| 34 | amnachra2-002AS | CTACACGAGAGTTTCCAAAAAGTCATCG |
| 35 | amnachra3-001S | CATGATGAAGAGCCTGGTGGGGATC |
| 36 | amnachra3-002AS | TTAGAGGCTCGTAACGATGTGTGG |
| 37 | amnachra4-001S | CATGCCCCCCATAATAGGGGAAAC |
| 38 | amnachra4-002AS | CTATTGTGGCGGACAGTTTACCAC |
| 39 | amnachra5-003S | CATGTCGCCTTTGGTCCTGTTC |
| 40 | amnachra5-002AS | CTCTTAACCCTCTTTGGCAATGTTCG |
| 41 | amnachra6-001S | CATGCGCGCAAGTAGTGTATTACAAGC |

(continued)

| SEQ ID NO: | Name | Sequence (5' to 3') |
|---|---|---|
| 42 | amnachra6-002AS | CTTATTGGACGATTATGTGTGGCGC |
| 43 | amnachra7-001S | CATGAGACGTTGGACTCTCATGGCG |
| 44 | amnachra7-002AS | GAATCACGTGACGATGATGTGTGGC |
| 45 | amnachra8-001S | CATGTTTAAAATGCAAATATTGACGCTTGG |
| 46 | amnachra8-002AS | TTATCCTTCTGGAGAAATGTCTATATTTGG |
| 47 | amnachra9-001S | CATGAAAATGAGAATAATAACAGCTCTTGG |
| 48 | amnachra9-002AS | TCACGTGGATGGTACAAGAGTGATC |
| 49 | amnachrb1-001S | CATGCATAATATTTGCTCGAGGCTCG |
| 50 | amnachrb1-002AS | TTATTTTCCACGGTAGATCTCTATTATATG |
| 51 | amnachrb2-001 S | CATGTTAAACATGAAGAATATATTCCCCG |
| 52 | amnachrb2-007AS | GAAAAAGATTAACACAAGTTACATTCCATAAG TTAAC |
| 53 | ceric3-001S | CATGCCAAAAACTGAACGGCGTC |
| 54 | ceric3-002AS | TCAAGTCTTTTTAGGTCTCCGCCTTC |
| 63 | amric3-007AS | CAAGGCTCCACAGATTCAGATTTGATTC |

Electrophysiology.

*RNA preparation*

Plasmids coding for Am-nAChRα and β subunits.

The nAChRα and β subunits of the nAChR gated ion channel, as obtained above, have been linearized at a restriction site localized in the 3' sequence just following the STOP codon using the appropriate restriction enzyme (Table 2). The reaction medium (volume of 50μl) contained 10 μg of plasmid, 3μl of the restriction enzyme (New England Biolabs France, Evry, France), 5μl of 10X reaction buffer provided by the manufacturer and $H_2O$ to 50μl. The reaction was then incubated for 3 hours at 37°C. The linearized plasmids were then purified using the NucleoSpin Extract II (Macherey-Nagel EURL, Hoerd, France) kit following manufacturer instructions, and resuspended at a concentration of 1μg/μl using deionized water (concentration was verified by measuring the OD at 260nm with a Biophotometer (Eppendorf France SAS, Le Pecq, France)). The efficiency of the linearization was also checked by agarose gel using 3μg of each plasmid.

RNA for each subunit was then obtained by *in vitro* transcription using the kit T3- or T7-mMessage mMachine (Life Technologies, Saint Aubin, France); following manufacturer recommendations (3-4 hours at 37°C). mRNA were then purified by using the RNeasy Mini Kit (Qiagen SAS, Courtaboeuf, France), and resuspended in desioned water at a concentration of 1μg/μl (verified by measuring the OD at 260nm with a Biophotometer (Eppendorf France SAS, Le Pecq, France). The length of each mRNA was also verified on agarose gel using 0.5μg of RNA. Each mRNA was then aliquoted at 2μl and stored at -20°C for further use.

Table 2: cDNA, vector, restriction enzyme and RNA polymerases used for in vitro transcription.

| cDNA SEQ ID | Vector | Enzyme | Polymerase |
|---|---|---|---|
| Am-nAChRα1 SEQ ID NO: 1 | pCMV-PL10 | NotI | T7 |
| Am-nAChRα2 SEQ ID NO: 2 | pCMV-PL10 | NotI | T7 |
| Am-nAChRα3 SEQ ID NO: 3 | pCMV-PL10 | NotI | T7 |
| Am-nAChRα4 SEQ ID NO: 4 | pCMV-PL10 | NotI | T7 |
| Am-nAChRα5 SEQ ID NO: 5 | pCMV-PL10 | NotI | T7 |

(continued)

| cDNA SEQ ID | Vector | Enzyme | Polymerase |
|---|---|---|---|
| Am-nAChRα6 SEQ ID NO: 6 | pCMV-PL10 | NotI | T7 |
| Am-nAChRα7 SEQ ID NO: 7 | pCMV-PL10 | NotI | T7 |
| Am-nAChRα8 SEQ ID NO: 8 | pCMV-PL10 | NotI | T7 |
| Am-nAChRα9 SEQ ID NO: 9 | pCMV-PL10 | NotI | T7 |
| Am-nAChRβ1 SEQ ID NO: 10 | pCMV-PL10 | NotI | T7 |
| Am-nAChRβ2 SEQ ID NO: 11 | pCMV-PL10 | NotI | T7 |
| Am-ric3a SEQ ID NO: 12 | pCMV-PL10 | NotI | T7 |
| Am-unc50 SEQ ID NO: 14 | pCMV-PL10 | NotI | T7 |
| Am-unc74 SEQ ID NO: 15 | pCMV-PL10 | NotI | T7 |
| Am-emc6 SEQ ID NO: 13 | pCMV-PL10 | NotI | T7 |
| Ce-ric3 SEQ ID NO: 64 | pCMV-PL10 | NotI | T7 |

*Oocyte preparation*

The *Xenopus laevis* female (from CRBM, UMR 5237, CNRS, Montpellier) was first anaesthetized by immersion using MS222 (ethyl-aminobenzoate methane sulfonate, ref A5040, Sigma) at 0.2% (pH=7) diluted in water during 20-30 minutes. Then a small 1-2 cm incision on the abdomen in between the midline and the lateral aspect of the abdomen was made through the fascia and muscle to visualize the oocytes. Fascia and muscle were picked up with forceps before cutting to avoid cutting the liver. Oocyte strands were then gently externalized cut and placed in a Petri dish filled with the OR2 solution. The incision was closed by suturing both the fascia and skin layer in two layers using surgical thread. The *Xenopus* was then allowed to recover in dedicated tank water for 1-2 hours.

Oocytes were then washed 3 times with OR2 and transferred in a 50 ml Falcon tube filled 30 ml of OR2 supplemented with collagenase 1A (1mg/ml; ref C9891 Sigma), and placed in an orbital shaker for 2-3 hours. The proper enzymatic digestion of the follicular cell layer was followed by inspection of the oocytes under a 30X binocular. After completion oocytes were washed 2-3 times with OR2 and 2 times with ND96S (Table 3). Nicely isolated stage VI oocytes were selected and collected in batches of 30 oocytes in 30mm Petri dishes for injection.

Table 3: OR2 and ND96S solution.

| **OR2** | | | **ND96S** | | |
|---|---|---|---|---|---|
| | mM | g/l | | mM | g/l |
| NaCl | 82.5 | 4.81 | NaCl | 96 | 5.6 |
| KCl | 2 | 0.15 | KCl | 2 | 0.15 |
| MgCl2 | 1 | 0.2 | $CaCl_2$ | 1.8 | 0.264 |
| Hepes | 5 | 1.19 | $MgCl_2$ | 1 | 0.2 |
| | | | HEPES | 5 | 1.19 |
| pH=7.2 (NaOH) | | | Pyruvate | 2.5 | 0.275 |
| | | | Gentamicyne | 0.05 | 1ml |
| | | | pH=7.5-7.6 (NaOH) | | |

*RNA or cDNA injection*

RNA injection was performed using glass pipets (Clark Electromedical Instrument CG150T1) pulled using a Sutter Inst. P30 microelectrode puller, giving a final sharp tip a 2-5 μm. Under a binocular microscope, the pipette was then mounted on a micromanipulator and connected a homemade pressure-injection system. The pipette is first filled with the RNA mixture by backfilling the tip. The Tip is immersed in a drop (2 μL) of the RNA mixture, and filled by connecting the pipet, via the injection system, to vacuum. The 1 μl of RNA are taken by the pipet with special care to avoid any air bubble. Then a batch of 20-30 oocytes are injected individually, under visual inspection with the microscope, with 25-50 nl of RNA mixture using the injection apparatus. The pipette is changed for each RNA mixture. For RNA, the point of injection on the oocytes is the equatorial ring, for DNA injection, the point of injection is the middle of the black/brown animal pole.

The mixture of RNA that have been used here are:

- Am-nAChRax subunit alone or in combination with another Am-nAChRαx and/or Am-nAChRβy and/or Am-Ric3, and/or [Am-unc50 + Am-unc74 + Am-emc6]

With x ∈ [1..9] and y ∈ [1,2]

The final concentration of RNA was always 1μg/μl.

After injection, each batch of oocytes was placed again on the orbital shaker (1 rotation /2sec) for 2-5 days prior recording, with the incubation medium (ND96S) renewed daily.

*Electrophysiological measurements*

*Two electrodes voltage-clamp set-up*

A home-made recording chamber (50 μl) is placed under a stereomicroscope and connected to an array of 8 reservoirs (50 ml syringes) containing the various solutions. The flow of solution from each syringe can individually be automatically switched ON or OFF by micro-electrovalves connected to the voltage-clamp recording software version 9.0 of the pClamp program (Axon Inst., Molecular devices).

The chamber is electrically connected to the ground by Agar bridges. Clark capillaries (with filament, GC150F10) are bent at ~120°C under flame and subsequently immersed in 60mm Petri dishes filled with almost boiling agar (high gel strength) dissolved at 1% in 3M KCl (5-10 capillaries can be placed per dish). When immersed, these bridges are usually filled naturally (by capillarity) by the hot agar solution. After cooling, two Agar-bridges are "dissected" from the agar and placed in the bath-electrode holder previously filled with 3M KCl. For two-electrodes voltage-clamp, voltage and current electrodes were pulled from Clark Electromedical Instrument borosilicate glass capillaries (GC150T-10) using a P-97 Sutter Instrument Company puller. They have a resistance of 0.5-2 MΩ when filled with 3M KCl.

In both two-electrodes voltage-clamp and single-channel recordings voltage protocols and current recordings are made using version 9.0 of the pClamp program (Axon Inst., Molecular devices) running on a PC computer connected, via Digidata 1200 interface to the Geneclamp 500 amplifier (Axon Instruments, Inc.) for two-electrodes voltage-clamp or to the Axopatch 200B amplifier (Axon Instruments, Inc.) for single channel recordings. Thus, voltage-command, sampling, acquisition and analysis are done using pClamp program. Additional analysis was performed using the Microsoft Excel software. All experiments are performed at room temperature (20-25 °C).

Typically, an oocyte, injected 2 to 5 days before with a given mixture of RNA is placed in the recording chamber filled with the desired solution (usually Na100). Junction potentials (typically less than 3-5 mV) at the two electrodes are first cancelled using the zero button of the amplifier in Na100 solution, and the electrodes resistance is checked before introduction into the oocyte (between 0.3 to 1.5 MΩ). Both electrodes are then impaled into the oocyte and the resting membrane potential is measured (typically -30/- 50 mV). The oocyte is then voltage-clamped usually at - 60mV. The effect of Ach on expressed channels is then check by switching the perfusion system from on reservoir of control solution (i.e. ND96 or Na100) from one containing ACh at a given concentration (usually between 100 to 1000 μM), or a neon-icotinoid (imidacloprid, clothanidin or thiamethoxam). Sometime, the effect of a test compound can be determined directly, by applying 50 μl of the compound directly to the bath (with the main perfusion stopped) at the final working concentration (usually 100-1000 μM), after proper dilution from a stock solution (usually 10 mM in water or DMSO or ethanol) into the desired recording solution. We assume that there is no dilution in the recording chamber which has a volume of around 50 μl.

Membrane currents are measured either at steady voltage as the difference between the current amplitudes before and during the application of ACh. These measures can also be done at different membrane potential (steady potential from -60 to -100 mV or voltage - ramps from -80mV to +20 mV, with the change in membrane conductance followed by the modification in the slope of the current-voltage curve: current recorded during the voltage ramp, with the corresponding voltage axis). In these conditions, washing-out of the test compound is done by switching-on the gravity-driven perfusion of the chamber with the same solution without test compound. Similar experiments are performed with oocytes injected with different nAChR subunits and chaperone proteins RNA.

The tested compound can either decrease or increase ACh-induced current or modified other parameters, such as the channel kinetics, or channel selectivity for example. Such modifications can be considered as toxic for bees, thus revealing a potential toxicity of these products for bees.

Dose-response curves are obtained by measuring the ACh-induced current amplitude ($I_{ACh}$), as measured before, for different concentrations of ACh or the tested compounds. From these measured, the $I_{ACh}$ = f(log([ACh]) is constructed and a non-linear regression using the following equation is performed.

$$I_{ACh} = I_{ACh}Max / (1+exp([ACh]-EC_{50})/h)$$

Where $I_{ACh}$ is the ACh-induced current, $I_{ACh}$MAX is the current amplitude recorded for a saturating ACh concentration, [ACh] are the various ACh concentrations perfused, "h" is a slope factor. For each nAChR subunit combinations showing functional expression the EC50 and the "h" factor is then calculated.

Ionic selectivity is calculated by measuring the current reversal potentials of ACh-induced currents in recording solutions. Current Reversal potential (Erev(X)) is then measured as the potential at which the ACh-induced current is 0 during voltage ramps. This measure is done after digital subtraction of traces recorded before and after ACh application. All these measurements are usually performed on 3-15 different oocytes of each batch for each concentration or compounds, and the resulting values represents the average result between these oocytes. The statistical significance is tested using the student t-test at 5%.

## Claims

1. A nucleic acid sequence of at least one subunit a of the nicotinic acetylcholine receptor of an arthropod as set forth in SEQ ID NO: 1 to 9 or a variant thereof.

2. A nucleic acid sequence of at least one subunit β of the nicotinic acetylcholine receptor of an arthropod as set forth in SEQ ID NO: 10 to 11 or a variant thereof.

3. A nucleic acid sequence of the chaperone protein of the nicotinic acetylcholine receptor of an arthropod as set forth in SEQ ID NO: 12 to 15 or a variant thereof.

4. A vector comprising at least one nucleic acid sequence according to any one of claims **1** to **3**.

5. An isolated cell transfected with at least one vector of claim **4**.

6. A functional nicotinic acetylcholine receptor (nAChR) channel comprising at least one subunit α of nAChR encoded by a nucleic acid sequence selected from the group comprising SEQ ID NO: 1 to 9 or a variant thereof according to claim **1**.

7. A functional nAChR channel comprising at least one subunit α encoded by a nucleic acid sequence selected from the group comprising SEQ ID NO: 1 to 9 or a variant thereof according to claim **1** and at least one chaperone protein encoded by the nucleic acid sequence selected from the group comprising SEQ ID NO: 12 to 15 or a variant thereof according to claim **3**.

8. A functional nicotinic acetylcholine receptor (nAChR) channel comprising at least one subunit α of nAChR encoded by a nucleic acid sequence selected from the group comprising SEQ ID NO: 1 to 9 or a variant thereof according to claim **1** and at least one subunit β encoded by a nucleic acid sequence selected from the group comprising SEQ ID NO: 10 to 11 or a variant thereof according to claim **2**.

9. A functional nAChR channel of an arthropod comprising at least one subunit α of nAChR encoded by a nucleic acid sequence selected from the group comprising SEQ ID NO: 1 to 9 or a variant thereof according claim **1**, at least one subunit β of nAChR encoded by the nucleic acid sequence selected from the group comprising SEQ ID NO: 10 to 11 or a variant thereof according to claim **2** and at least one chaperone protein encoded by nucleic acid sequences selected from the group comprising SEQ ID NO: 12 to 15 or a variant thereof according to claim **3**.

10. A vector comprising the channel according to claim **6** or **7** or **8** or **9**.

11. An isolated cell transfected with at least one vector of claim **10**, and expressing the channel according to claim **6** or **7** or **8** or **9**, preferably said cell is an oocyte of Xenopus.

12. An *in vitro* method to determine the effect of a test compound on the modulation of activity of a nAChR channel of an arthropod comprising:

    a. contacting an isolated cell according to of claim **5** or **11** with at least one test compound,
    b. measuring the effect of said test compound on the channel activity, and comparing said effect to the effect without test compound or to the effect of a reference value, thereby determining a modulation of activity of said channel.

13. The method according to claim **12**, for determining the toxicity of a test compound on an arthropod.

14. An *in vitro* method for screening compounds that modulate the nAChR channel activity of an arthropod comprising:

    a. contacting an isolated cell according to claim **5** or **11** with at least one test compound,
    b. measuring the effect of said compound on the nAChR channel activity, and

comparing said effect to the effect without test compound or to the effect of a reference value, thereby determining a modulation of activity of said nAChR channel.

**15.** A kit comprising at least one vector according to claim **4** or **10**, or an isolated cell according to claim **5** or **11** and reagents.

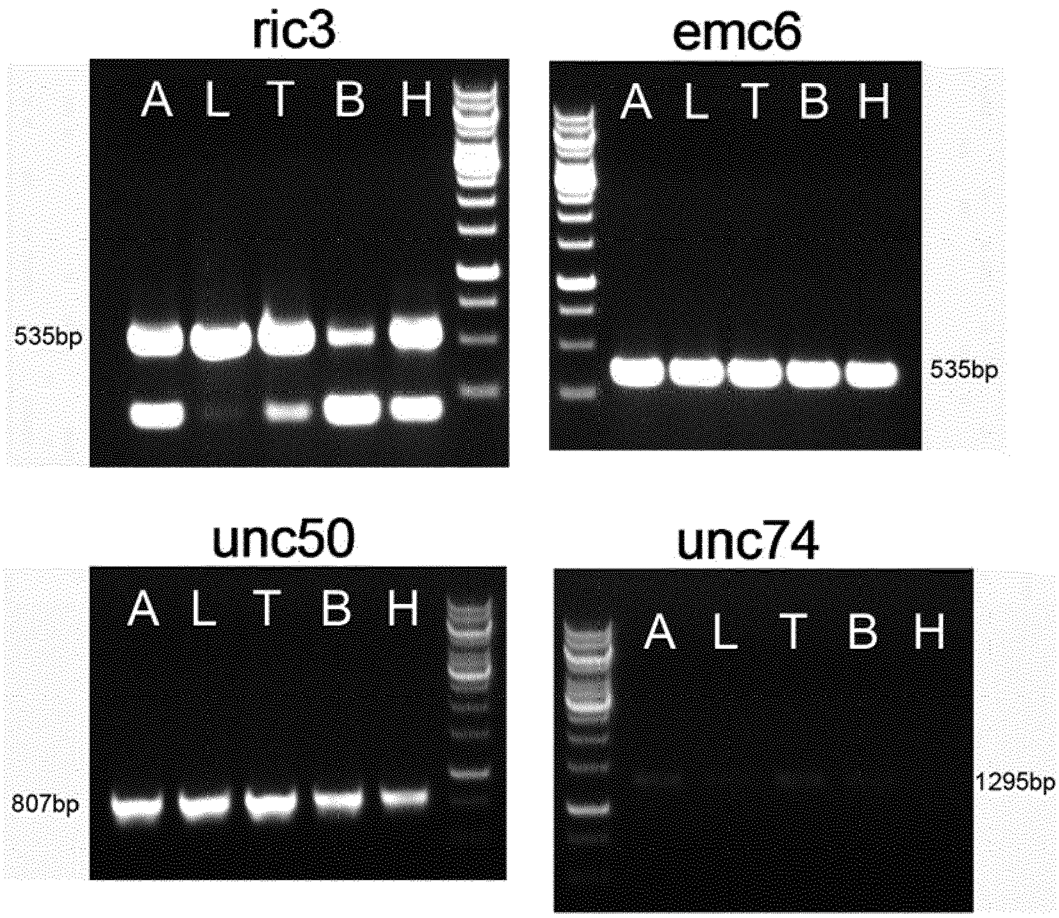
ric3
A L T B H
535bp
emc6
A L T B H
535bp
unc50
A L T B H
807bp
unc74
A L T B H
1295bp

FIG. 1

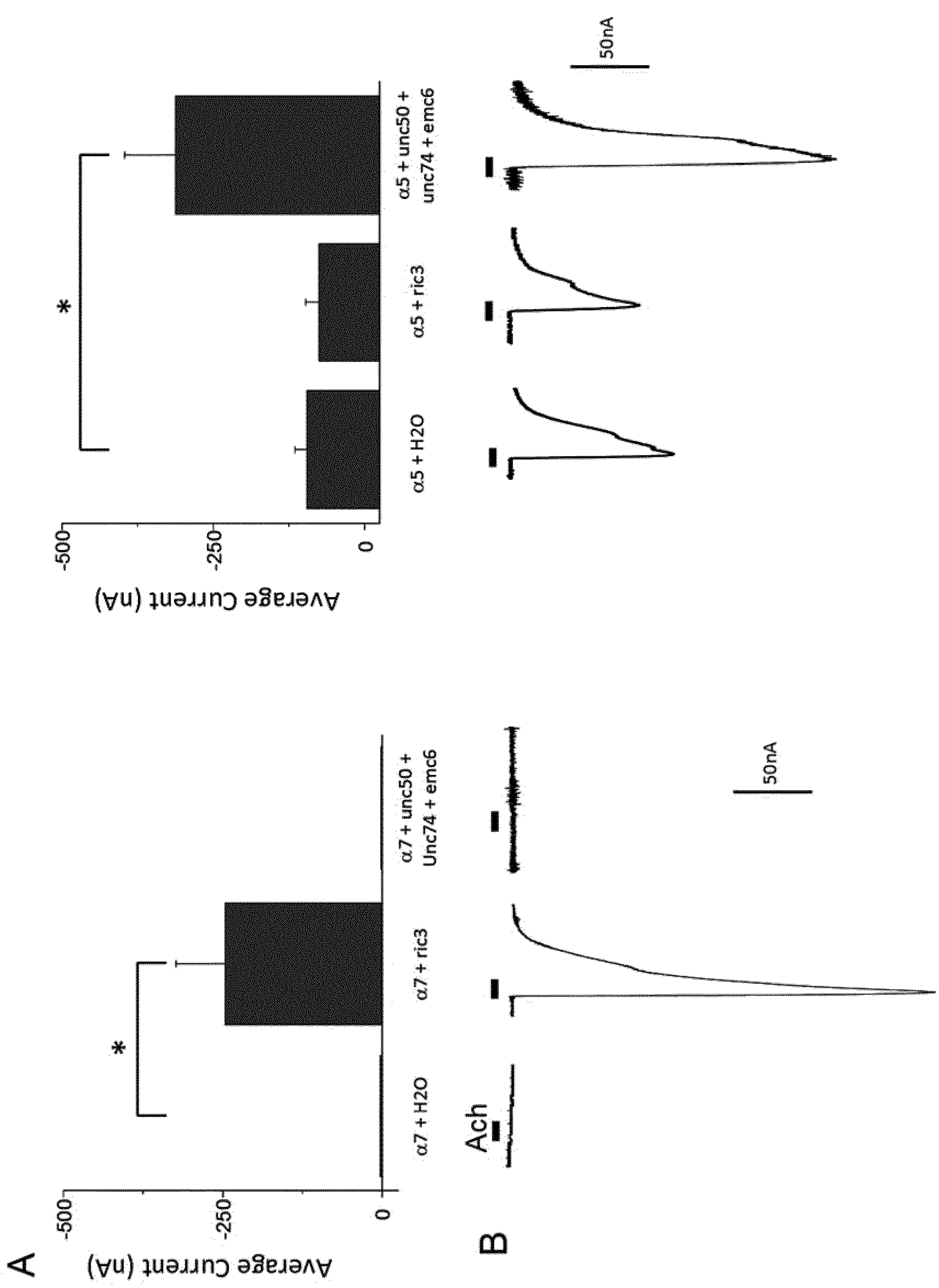
A
B
Average Current (nA)
-500
-250
0
α7 + H2O
α7 + ric3
α7 + unc50 + Unc74 + emc6
α5 + H2O
α5 + ric3
α5 + unc50 + unc74 + emc6
*
Ach
50nA

FIG. 2

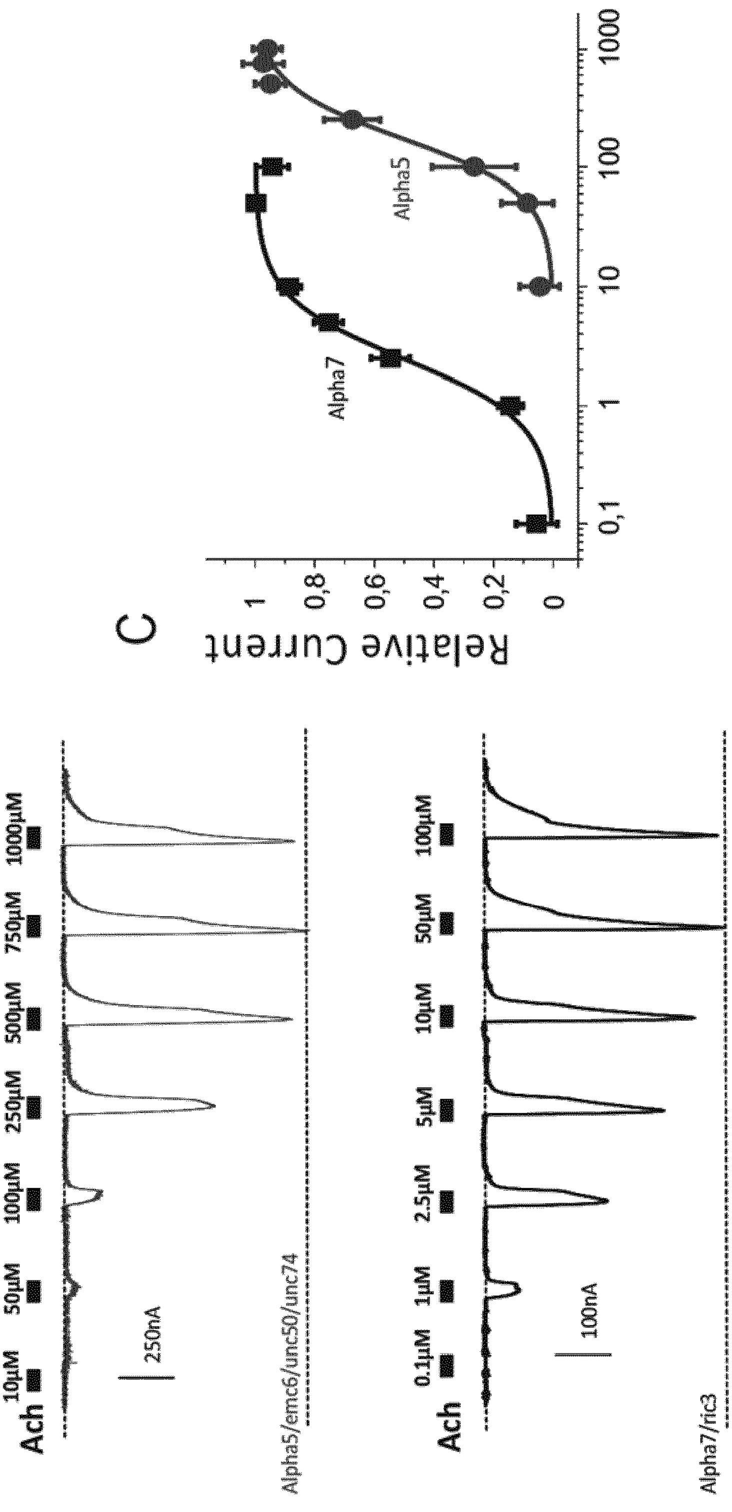
A
Ach
10µM
50µM
100µM
250µM
500µM
750µM
1000µM
250nA
Alpha5/emc6/unc50/unc74
B
Ach
0.1µM
1µM
2.5µM
5µM
10µM
50µM
100µM
100nA
Alpha7/ric3
C
Relative Current
1
0,8
0,6
0,4
0,2
0
0,1
1
10
100
1000
Alpha7
Alpha5

FIG. 3

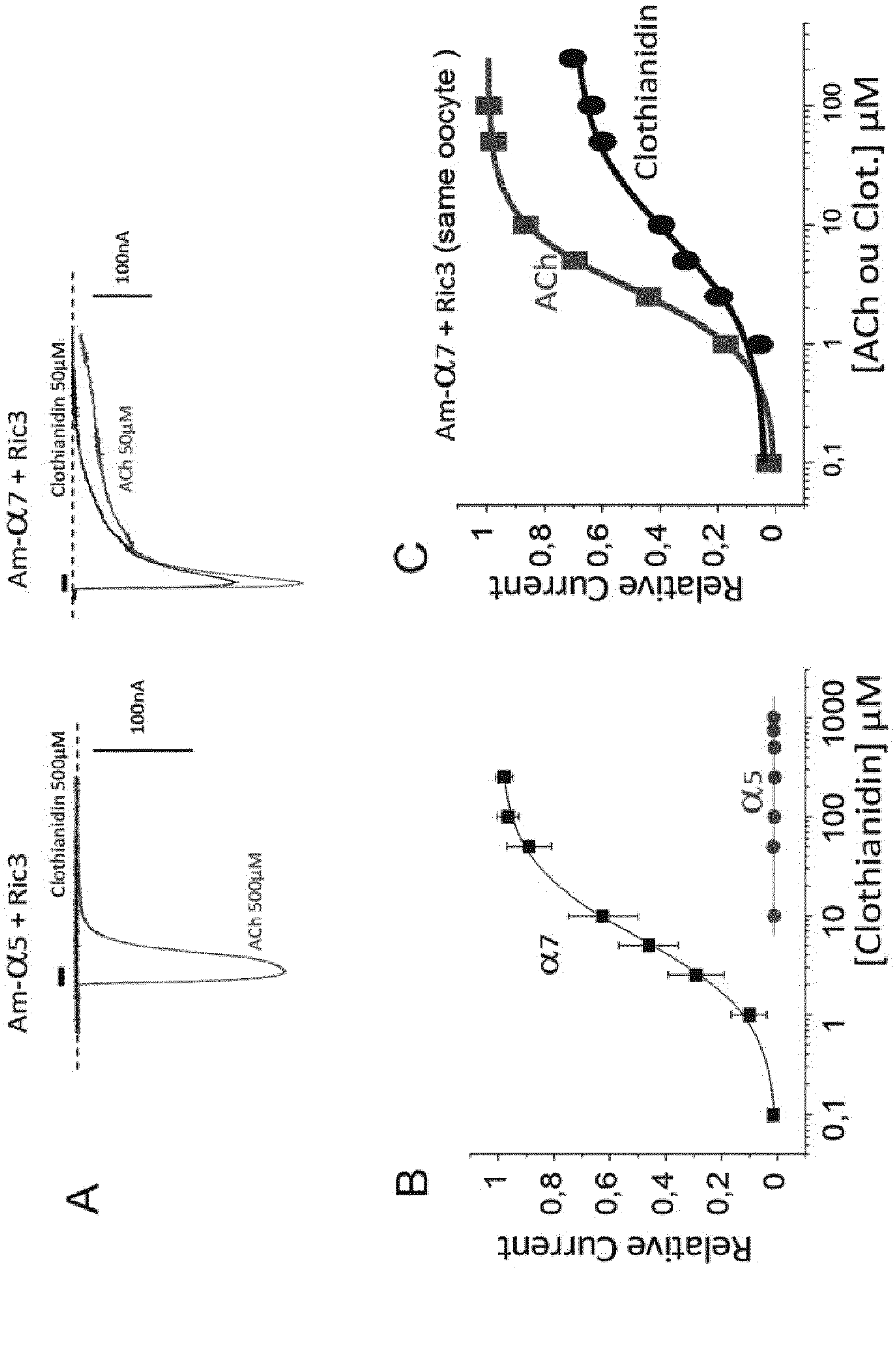
A
Am-α5 + Ric3
Clothianidin 500µM
ACh 500µM
100nA
Am-α7 + Ric3
Clothianidin 50µM
ACh 50µM
100nA
B
Relative Current
α7
α5
[Clothianidin] µM
C
Am-α7 + Ric3 (same oocyte )
ACh
Clothianidin
Relative Current
[ACh ou Clot.] µM

FIG. 4

Europäisches Patentamt
European Patent Office
Office européen des brevets

# EUROPEAN SEARCH REPORT

Application Number
EP 15 17 7390

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X<br><br><br><br><br><br><br><br><br><br>Y | CHRISTINE G ELSIK ET AL: "Finding the missing honey bee genes: lessons learned from a genome upgrade", BMC GENOMICS, vol. 15, no. 1, 30 January 2014 (2014-01-30), page 86, XP021175420, BIOMED CENTRAL LTD, LONDON, UK ISSN: 1471-2164, DOI: 10.1186/1471-2164-15-86<br>* abstract *<br>----- | 1-5,10, 15<br><br><br><br><br><br><br><br><br><br>6-9, 11-14 | INV. C07K14/705 C07K14/47 G01N33/68 |
| X | A. K. JONES ET AL: "The nicotinic acetylcholine receptor gene family of the honey bee, Apis mellifera", GENOME RESEARCH, vol. 16, no. 11, 25 October 2006 (2006-10-25), pages 1422-1430, XP055223583, ISSN: 1088-9051, DOI: 10.1101/gr.4549206<br>* table 1 *<br>----- | 1-15 | |
| | | | TECHNICAL FIELDS SEARCHED (IPC) |
| X<br><br><br><br><br><br><br><br><br><br>Y | DATABASE EMBL [Online]<br><br>2 July 2015 (2015-07-02), "Apis mellifera clone 1113L6.5 nicotinic acetylcholine receptor alpha1 subunit (nAChRalpha1) mRNA, complete cds.", XP002748811, retrieved from EBI accession no. EM_STD:KJ939588 Database accession no. KJ939588<br>* the whole document *<br>-----<br>-/-- | 1,4,5, 10,15<br><br><br><br><br><br><br><br><br><br>2,3,6-9, 11-14 | C07K<br>G01N |

The present search report has been drawn up for all claims

| Place of search | Date of completion of the search | Examiner |
|---|---|---|
| Munich | 28 October 2015 | Griesinger, Irina |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 17 7390

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE EMBL [Online] 2 July 2015 (2015-07-02), "Apis mellifera clone 1013L21.1 nicotinic acetylcholine receptor alpha3 subunit (nAChRalpha3) mRNA, complete cds.", XP002748812, retrieved from EBI accession no. EM_STD:KJ939590 Database accession no. KJ939590 | 1,4,5, 10,15 | |
| Y | * the whole document * | 2,3,6-9, 11-14 | |
| X | DATABASE EMBL [Online] 2 July 2015 (2015-07-02), "Apis mellifera clone 1113L11.2 nicotinic acetylcholine receptor beta1 subunit (nAChRbeta1) mRNA, complete cds.", XP002748813, retrieved from EBI accession no. EM_STD:KJ939597 Database accession no. KJ939597 | 2,4,5, 10,15 | |
| Y | * the whole document * | 1,3,6-9, 11-14 | TECHNICAL FIELDS SEARCHED (IPC) |
| X | DATABASE EMBL [Online] 2 July 2015 (2015-07-02), "Apis mellifera clone 1013L19.2 receptor associated protein RIC-3, alternatively spliced (Ric-3) mRNA, complete cds.", XP002748814, retrieved from EBI accession no. EM_STD:KJ939599 Database accession no. KJ939599 | 3-5,10, 15 | |
| Y | * the whole document * | 1,2,6-9, 11-14 | |

-/--

The present search report has been drawn up for all claims

3

| Place of search | Date of completion of the search | Examiner |
|---|---|---|
| Munich | 28 October 2015 | Griesinger, Irina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 17 7390

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE EMBL [Online] 2 July 2015 (2015-07-02), "Apis mellifera clone 1013L4.1 ER membrane protein complex subunit6-like protein (LOC551477) mRNA, complete cds.", XP002748815, retrieved from EBI accession no. EM_STD:KJ939604 Database accession no. KJ939604 | 3-5,10, 15 | |
| Y | * the whole document * | 1,2,6-9, 11-14 | |
| X | DATABASE EMBL [Online] 2 July 2015 (2015-07-02), "Apis mellifera clone 1013L5.1 unc-50-like protein (LOC550684) mRNA, complete cds.", XP002748816, retrieved from EBI accession no. EM_STD:KJ939605 Database accession no. KJ939605 | 3-5,10, 15 | TECHNICAL FIELDS SEARCHED (IPC) |
| Y | * sequence * | 1,2,6-9, 11-14 | |
| X | BOULIN T ET AL: "Functional reconstitution of Haemonchus contortus acetylcholine receptors in Xenopus oocytes provides mechanistic insights into levamisole resistance", BRITISH JOURNAL OF PHARMACOLOGY, vol. 164, no. 5, November 2011 (2011-11), pages 1421-1432, XP002748817, * abstract * | 1-15 | |

-/--

The present search report has been drawn up for all claims

3

| Place of search | Date of completion of the search | Examiner |
|---|---|---|
| Munich | 28 October 2015 | Griesinger, Irina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 17 7390

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BOULIN THOMAS ET AL: "Eight genes are required for functional reconstitution of the Caenorhabditis elegans levamisole-sensitive acetylcholine receptor (+ Supporting Information)", PROCEEDINGS OF THE NATIONAL ACADEMY OF SCIENCES OF THE UNITED STATES OF AMERICA, vol. 105, no. 47, November 2008 (2008-11), pages 18590-18595+2, XP002748818, ISSN: 0027-8424, DOI: 10.1073/pnas.0806933105 * abstract * ----- | 1-15 | |
| X | WO 2006/119497 A2 (UNIV UTAH RES FOUND [US]; WILLIAMS DANIEL C [US]; JORGENSEN ERIK M [US) 9 November 2006 (2006-11-09) * claims 6,8,16,28 * ----- | 1-15 | |
| X | MILLAR N S: "RIC-3: a nicotinic acetylcholine receptor chaperone", BRITISH JOURNAL OF PHARMACOLOGY, vol. 153, no. Suppl. 1, March 2008 (2008-03), pages S177-S183, XP002748819, ISSN: 0007-1188 * abstract * ----- | 1-15 | TECHNICAL FIELDS SEARCHED (IPC) |

The present search report has been drawn up for all claims

3

| Place of search | Date of completion of the search | Examiner |
|---|---|---|
| Munich | 28 October 2015 | Griesinger, Irina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 15 17 7390

This annex lists the patent family members relating to the patent documents cited in the above-mentioned European search report. The members are as contained in the European Patent Office EDP file on
The European Patent Office is in no way liable for these particulars which are merely given for the purpose of information.

28-10-2015

| Patent document cited in search report | Publication date | Patent family member(s) | Publication date |
|---|---|---|---|
| WO 2006119497 A2 | 09-11-2006 | US 2010160217 A1 | 24-06-2010 |
| | | WO 2006119497 A2 | 09-11-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6377057 B
- EP 0689051 A
- WO 9854294 A


### Non-patent literature cited in the description


- **SUCHAIL S et al.** *Environ Toxicol Chem,* 2001, vol. 20 (11), 2482-6
- **GELS JA.** *J Econ Entomol,* 2002, vol. 95 (4), 722-8
- Computational Molecular Biology. Oxford University Press, 1988
- Biocomputing: Informatics and Genome Projects. Academic Press, 1993
- Computer Analysis of Sequence Data. Humana Press, 1994
- **VON HEINJE, G.** Sequence Analysis in Molecular Biology. Academic Press, 1987
- Sequence Analysis Primer. M. Stockton Press, 1991
- **CARILLO et al.** *SIAM J. Applied Math.,* 1988, vol. 48, 1073
- **DEVEREUX et al.** *Nucl. Acid. Res. \,* 1984, vol. 2, 387
- **ALTSCHUL et al.** *J. Mol. Biol.,* 1990, vol. 215, 403-410
- Guide to Molecular Cloning Technics. Methods in Enzymology. 1987, vol. 152, 359-371
- **GROSS et al.** *Biosensors and Bioelectronics,* 1995, vol. 10, 553-567
- **JONES ; SATTELLE.** *Genome Res.,* 2006, vol. 16, 1422-1430
- **WHITFIELD et al.** *Genome Res.,* 2002, vol. 12, 555-566
- **DUPUIS et al.** *J Neurophysiol,* 2011, vol. 106, 1604-1613
- **ELSIK et al.** *BMC Genomics,* 30 January 2014, vol. 15, 86
- **BOULIN et al.** *Proc Natl Acad Sci USA.,* 25 November 2008, vol. 105 (47), 18590-18595

SEQUENCE LISTING

<110> Centre National de la Recherche Scientifique (CNRS)
CHARNET , Pierre

<120> NICOTINIC CHANNEL SUBUNITS OF POLLINATOR INSECTS AND THEIR USES THEREOF

<130> CV - 509/EP

<160> 65

<170> BiSSAP 1.2

<210> 1
<211> 1806
<212> DNA
<213> Apis mellifera

<220>
<221> source
<222> 1..1806
<223> /mol_type="unassigned DNA"
/note="nicotinic acetylcholine receptor alpha1 subunit nucleic acid sequence"
/organism="Apis mellifera"

<400> 1
atggcgacgg ccatttcctg tcttgttgcc ccgtttccgg gtgcctcggc caattcggaa 60

gcgaagcgtc tctacgacga cctgctgtcc aactacaacc gcctcatccg ccccgttggc 120

aacaacagcg atcgcctcac tgtcaaaatg ggactgcgcc tctcccagct catcgacgtt 180

aacctgaaga accagatcat gacgacgaac gtttgggtgg aacaagagtg gaacgattac 240

aagctgaagt ggaacccgga tgattatggc ggtgtcgaca ccctccacgt gccgtcggag 300

catatatggt tgccggacat tgttctttac aacaacgccg atggcaacta cgaggtgacc 360

attatgacca aggcaatttt gcaccatacg gggaaggtcg tgtggaaacc gccggccatc 420

tataaatcat tttgcgagat cgacgtggag tacttcccct ttgacgagca gacttgcttc 480

atgaagtttg gttcgtggac ttacgacggg tacacggtcg acttgcgaca ccttgcccag 540

accgaagact cgaaccagat cgaggtcggg atcgacctga ccgactacta catttccgtc 600

gagtgggaca tcataaaagt gcctgccgtg aggaacgagg cgttctacat atgctgcgag 660

gagccctacc ccgatatcgt gttcaatatc accctacgcc gcaagaccct cttctacacg 720

gtgaacctga tcataccgtg cgtgggcata tcttttctct cggtgctggt gttctacctg 780

ccgagcgaca gcggggagaa ggtctcgctc tcgatctcga tcctgctgtc gttgacggtg 840

ttcttcctac tgctcgccga gataatcccg cccacgtcgc taacggtgcc gttgctcggc 900

aagtacctac tgttcaccat ggtgctggtc accctttccg tggtggtgac gatagccgtg 960

ctgaacgtga actttcgatc gcccgtcacc caccggatgg cgaggtgggt gagggtggtg 1020

ttcatacaag tgttgccgcg tttccttctc atcgagaggc cgaagaagga cgaggacgag 1080

gaggaggagg aggaggtggt ggtggtgggg aggaacgggg ccggggttgg cgcgatgaac 1140

gccaacgggg aggcggtcgg cgaggtggac gaggacgacg acgacgacgg ggacgacgac 1200

gacgacgacg acgtggaggc ggcgaacggg aaaccggccg agggcatgct caccgatgtg 1260

ttccatgtac aagagactga caagtacgac gcgtactacg gcaacaggtt cagcggggag 1320

tacgagatac ctgcccacgg tctgccaccg tcggcgacca gatacgacct tggcgcggtg 1380

gccaccgtgg gcacgaccgt agcgccctgc ttcgaggagc ccctaccctc gctcccactg 1440

ccgggggctg acgacgacct tttcggcccg gccagccccg catacgttca cgaggacgtc 1500

agccccacat tcgagaagcc gttggttcgc gagatcgaga agaccatcga cgatgccagg 1560

tttatcgctc agcacgccaa gaacaaggac aagttcgaga gcgtggagga ggactggaaa 1620

tacgtggcga tggtgctcga ccggatcttc ctgtggatat tcacggtggc ctgcgtgctc 1680

ggcacggtgt tgatcattct ccaggcgccg agcctgtacg ataccacgaa gccgatcgac 1740

atcaagtaca gcaagatcgc gaagaagaag atgatgctga tgaacatggg tcccgaggag 1800

gactag 1806

<210> 2
<211> 1626
<212> DNA
<213> Apis mellifera

<220>
<221> source
<222> 1..1626
<223> /mol_type="unassigned DNA"
/note="nicotinic acetylcholine receptor alpha2 subunit nucleic acid sequence"
/organism="Apis mellifera"

<400> 2
atgatactcc agacgatcat cttgatcctc tccgcgagga tatgccacgg gaatccggac 60

gcgaagaggc tgtacgacga tttgctgtcg aattacaatc gactgatacg ccccgtttcg 120

aataacaacg acacggtggt cgttaaacta ggacttcgcc tgtcccaact gatcgatctc 180

aatttgaagg atcaaattct cacgaccaac gtttggctcg agcacgaatg gcaggatcac 240

aagtttcagt gggacccggc agagtacgga ggtgtcaccg aactctacgt accgagcgag 300

cacatatggc ttcccgacat cgtactctac aataacgcgg acggggagta cggagttacc 360

acgatgacca aagccatcct gcattacacg ggcaaggtat tgtggacgcc tccggctatc 420

ttcaaatcct cgtgcgagat agacgttcga tattttccct tcgatcaaca aacttgcttc 480

atgaagttcg gttcctggac gtacgacggt attcagatcg atttgaaaca catcaatcaa 540

aacatgggag acaaagtgga gattgggatc gatcttcgcg agtactaccc cagcgtcgag 600

tgggacatat tgggtgttcc agctgaacgg cacaagaaat actatccctg ttgcgacgag 660

ccctatcccg acatcttctt caacatcact ctacgtcgga aaacgttgtt ctacacggtg 720

aatttgatcg tcccgtgcgt gagcatctcg tatttatcgg tgctcgcttt ctaccttccc 780

gcggactccg gcgagaaaat cgccctctgc atcaacatcc tgctctctca aacaatgttc 840

ttcctcttaa tatcggagat aataccatcc acctcgttgg cgttgccatt gctaggcaaa 900

tacttgctgt tcaccatgat cctcgtcggc ctctcggtag taataacgat cgttatattg 960

aacgtgcatt accggaagcc gagcactcac aaaatggcgc cttgggtgag gaagatcttc 1020

ataaggagat tgccgaagct tcttctgatg agggtgcccg acgatctgtt gaacgatctc 1080

gccgcgcata agatgcacgg aagaggaaga tccgggaaga agagcaaatt caacgccgcc 1140

gtcgctgccg cagtgcaatc gtcctccata gtctcgtccc ccgattccgc gcgtcatcaa 1200

cgaatcggcg ggtgcaacgg attgcacacg acaaccgcgc ataacagatt cttggggggg 1260

atcggtggat acaatggact gcccacggtg atgtccgggc tggacgagtc cctgagcgac 1320

gtaacgccca ggaagaaata tcctttcgag ctcgagaaag ctttgcacaa cgtgatgttc 1380

attcagcacc atatacaacg gcaggacgag ttcaacgcgg aagatcagga ttggggtttc 1440

gtagcgatgg ttctcgacag acttttcctg tggatcttca ctatagcatc gatcgttggc 1500

acgttcatca ttctctgcga ggcaccggcc ctccgcgaca acacgaaacc gatcgacatg 1560

gaatattcct ccgtggccca gcaacaattc cttcctcacg atgacttttt ggaaactctc 1620

gtgtag 1626

<210> 3
<211> 1704
<212> DNA
<213> Apis mellifera

<220>
<221> source
<222> 1..1704
<223> /mol_type="unassigned DNA"
/note="nicotinic acetylcholine receptor alpha3 subunit nucleic acid sequence"
/organism="Apis mellifera"

<400> 3
atgatgaaga gcctggtggg gatcatgtgg atagtgttgg tgctcatatc aggatgctcg 60

ggaaatccgg acgcgaagcg gctgtacgac gacctcctgt cgaattacaa caagctggtt 120

cgtccagtgg tcaacgtcac agacgccctc accgttaaaa tcaagctcaa actctctcag 180

ttgatcgacg taaatctgaa aaatcaaatc atgacaacga acctctgggt agaacagtca 240

tggtacgatt acaagttaaa atgggatcca aaggaatatg gtggggtgga aatgctacac 300

gtgccatccg atcatatatg gaggcccgat atagttttgt acaacaacgc cgacggtaat 360

ttcgaggtga cgctagcaac gaaggcgacg ctgaattaca cagggagggt cgaatggaag 420

ccaccggcca tatacaaatc ctcttgcgag atcgacgtag aatactttcc gttcgacgag 480

caaacgtgcg tcatgaagtt cggctcgtgg acttatgacg gcttccaggt tgatcttcga 540

cacatcgacg aaattcgtgg caaaaatgtc gtcgacatcg gcgttgatct gtccgagttt 600

tatacttccg tcgagtggga tatcttagaa gtcccagccg tgcgaaatga aaaattttac 660

acgtgctgcg acgagcccta cctcgacatc acgttcaaca tcaccatgag acggaagact 720

ctattttaca ccgtcaatct tataataccg tgtatgggta tctcgtttct cacggtgttg 780

gtcttctatc tgccaagcga cagtggcgag aaggtcagcc tatcaatttc aattctactg 840

tcgctgactg tgttcttcct tctgctggcc gaaattatcc cgcccacatc gctcgtagta 900

ccgcttcttg gtaaattcgt cctttcacc atgatcctag acaccttcag tatatgcgta 960

accgtggtag tcctcaatgt tcacttccgg tcgccacaga cgcacgtgat ggcaccatgg 1020

gtccgtcgtg tttttattca tgttctgcca agactactgg tgatgcgcag gtataatacg 1080

ccttcgaaga gatccgatta tgattccagg ccgcaatacc agatagataa acgtagcatg 1140

ggtagtcatc atggtcaacg agtaatggtc agaacgtgca atggtctcga attgcgggat 1200

ccgtcacttt ttgtagagac ttcagcctcg gagctggttg aatcttccgt tttgtttccg 1260

agtctcgatt cgcgggacga attacatcct cgggaattag aagctgtgaa tttaggaagt 1320

gcgtgtcgca ttcacggttc accagcgacc acggctgctc ctccaccgca actgccgacc 1380

gaggagagcg tggacgctct ctgtaacact cttcatcatt ggcatcactg cccagaaata 1440

tataaagcta tcgaaggcat tcgtttcatt gctgatcata cgaaaagaga agaggattct 1500

accagagtca aggaagattg gaaatatgtc gcgatggtgc ttgacaggct atttctctgg 1560

atttttacat tagccgtagt cgtcggtacg gcaggaatca tcctacaggc accaacgctt 1620

tatgacgatc gcattcccat tgacgtacga ctgtccgaga tcgcttccac cactgccaag 1680

ccacacatcg ttacgagcct ctaa 1704

<210> 4
<211> 1710
<212> DNA
<213> Apis mellifera

<220>
<221> source
<222> 1..1710
<223> /mol_type="unassigned DNA"
/note="nicotinic acetylcholine receptor alpha4 subunit nucleic acid sequence"
/organism="Apis mellifera"

<400> 4
atgcccccca taatagggga aacgttgcgt gtatggttcc tctcggcgtt ggtggttcac 60

ggtgctgtcg ctggcaatcc ggacgcgaaa cgcctttacg acgacttgct ttctaactat 120

aacaaactag tgcgccctgt tgtgaatacc tcggacgtgc tacgcgtgtg catcaagttg 180
aaactctccc agctcatcga cgtgaatttg aagaatcaaa tcatgacgac gaatctgtgg 240
gtggaacagt catggtacga ttacaagcta cgatgggagc cgaaggagta cggaggagtt 300
aaaatgttac acgtgccatc cgatcacata tggcggcccg atatagtcct ctacaacaac 360
gccgacggga actacgaggt gacgctgatg accaaggcca cggtctacta ttcgggattg 420
gtcgtctggc aaccgccggc cgtatataaa tcctcctgct ccatcgacgt tgagttcttt 480
ccgtacgacg tacagacctg tgttttgaag ttaggctcct ggacctatga cggttttaag 540
gtcgatctaa ggcatatgga cgagaaatcg ggaagcaatg tggtggatgt cggagtcgac 600
ctctccgaat tctacatgtc ggtagaatgg gatattttag aagtacctgc agtacgaaac 660
gaaaaatttt acacctgctg cgacgagccg tacctggata ttacatttaa tataacaatg 720
cgaagaaaaa cacttttcta cactgtgaac ataattattc cgtgtatggg aatttccttt 780
cttacggtac tgacgtttta tctacctagc gacagcgggg aaaaggtcac gctctccatt 840
tccattctca tcagtcttca cgtgtttttc ctgctggtcg tcgagatcat tccacctacg 900
tcgctggtcg tgccgctgct tggaaagtac ctgatattcg ccatgatact cgtttcgata 960
agtatatgcg tgacagtggt tgtacttaat gtccacttcc gatcgccaca aactcacaaa 1020
atggcacctt gggtgaagag ggttttcatc catatccttc ctcgcttgtt ggtcatgcga 1080
aggcctcaat ataaattcga aacaaataga tatagttctg gaagagtatt aatgagaaca 1140
gtcagaggaa aggaaaaaac ctgttattat ccttatcatc catctactca agaggatagc 1200
gaagaacatc taacgccaaa acgatttcac agtcgtgccg catcaaaaga agatctttca 1260
ccttccagtc tcgctgatgg cgcaaggttc ggcggaagtt gcttaatcca cgggccgcca 1320
ttaccaccgc ttcctcttca cacggaatgc gaagacctat ctgtttccgg cgaggcaggg 1380
atcgaggagg ttaaaagtcc tgtcctccga agccctcctg ccttctcgca ttcgcgctgc 1440
cctcccgaaa ttcacaaatc ctgcatctgt gtgcgcttca tcgcggaaca cacgaaaatg 1500
ctcgaagact cgacaaaggt gaaagaagac tggaaatacg tggcgatggt cttagacagg 1560
ctgttcctct ggatcttcac gttggcggtg ctggtaggca cagctgggat catcctacag 1620
gcaccgaccc tctacgacga ccgggtacca attgataaaa aattcaacga attcggaacc 1680
acggcggtgg taaactgtcc gccacaatag 1710

<210> 5
<211> 1445
<212> DNA
<213> Apis mellifera

<220>
<221> source
<222> 1..1445

<223> /mol_type="unassigned DNA"
/note="nicotinic acetylcholine receptor alpha5 subunit nucleic acid sequence"
/organism="Apis mellifera"

<400> 5
atgtcgcctt tggtcctgtt ctttcattac ggagttttgg ccatcatttt cgggaacggt 60

tttggggatg aacacgaata tagattgacg aaatatctgc tcgatggata cgacgccggt 120

gtacgtccag ccgagaattc ctcccaaccc ctggcggtcg tctttggcct ttctcttcat 180

cacataatcg acgtggacga gaagaatcag atactgacga cgaattgctg ggtgacacag 240

atttggacgg accatcacct gaaatggaac gcctcggagt tcgcggggat ccgagtgatt 300

cgtgttcctt acaatcgcgt ctggaggccg gacacgatcc tctacaacaa cgccgatcca 360

cagtacagct cggcagtcat aaatacgaac gtgatagtga gtcacacggg ggaggtggtg 420

tggttgagcc atgggatatt tcgcagcagc tgcgacatcg acgtcgagtt cttccccttc 480

gacgagcaac gctgtgtgtt gaaatgggcc tcctggacgt acgatggata ccaattggag 540

ttggagaagc aaagcgagca aggagacgtg agcaattatc aagcgaacgg tgaattcgac 600

ctggtcaatt tctccgcgag aaggaacgtg gagtattact cctgctgccc ggaaccgtat 660

ccggacatca cttacgagat acgattgcga cgtcgaccga tgttttacgt cttcaatctg 720

atacttccat gcatactcat caacagtgtc gccctgttgg tattctacgt gccgtctgaa 780

tcgggggaaa aagtcaccct cgggatttcg gccctcctct ctatgacggt cttcctaatg 840

actattcgcg agtcactgcc gcccacggag aaaacaccat taataagtct ttattacggt 900

gtcagtatat gcctggtgac attcgcttca gcgttagcgg tggtcacgtt gaatcttcat 960

caccgcggtg ttcgtggtac cagagtaccg ggtatcgtta gatcgttggt gttggacaag 1020

ctcgcaagga tagtgctcct caactttcaa gaggagagaa gatcggaacc ggtcgaaccg 1080

ccgagaagaa aaaacaattg tccgctgcac tgcaaaccgg agctgggcca atcgcaatcg 1140

tcgcccaagt tcgtagccag aagggaggag agtaatagca gcagtcctag tttagatctt 1200

ggaaaagaag gtggccttga ggcgcaatgg tcacgcgtgc tagggagggt gcacgcgact 1260

atagagagaa acgagaaacg gctcgcggag caggatcgtc gcgagcgaat ggaatttgat 1320

tggaaacagg tcgcgttggt cagcgatcga gcgcttcttt gcgttttttt tttgaccacg 1380

atcgtcagca ccacggtcat cctgtgtggt tctccgccaa gcgcgaacat tgccaaagag 1440

ggtta 1445

<210> 6
<211> 1590
<212> DNA
<213> Apis mellifera

<220>

<221> source
<222> 1..1590
<223> /mol_type="unassigned DNA"
/note="nicotinic acetylcholine receptor alpha6 subunit nucleic acid sequence"
/organism="Apis mellifera"

<400> 6
atgcgcgcaa gtagtgtatt acaagctgag agtgatgtgt catcgtgcgt tatttttggt 60

gtattatttg tgttattttc ctttctgagg acatgtacga agctccaaac tacttatttt 120

catcatatat acatcataca tcaaagcctg tgcggacgtc acgagaaacg tttgttgaac 180

gagctgttgt cgtcatacaa cactctggag cggcccgtcg ccaatgagag cgagcctctc 240

gaagtgaaat ttggcatcac gctacagcag atcatagatg tggatgagaa gaatcaaata 300

ttaactacga acgcgtggct caagttggaa tgggtggact acaacctcca atggaacgag 360

tccgagtacg gaggtgtaaa ggaccttcga attacaccaa acaagctctg gaagccagac 420

attctcatgt ataacagtgc ggatgagggt ttcgacggga cataccaaac aaacgtggta 480

gtcacgcatg acggcagttg cctgtacgtt cctccgggca tcttcaagag cacttgcaag 540

atagacatca cttggttccc ctttgacgac caacactgtg acatgaagtt cggatcctgg 600

acctacgacg gcaaccaggt cgacctcgtg ctcagctcgg agacgggcgg tgacctgtcc 660

gactttatca cgaatggaga atggtacctg atcggaatgc cgggcaagaa gaacacgata 720

acgtaccaat gctgtccgga gccttacgtc gacgtcacat tcaccataca gatccgtaga 780

aggaccctct actatttttt caacctgatc gtgccgtgcg tgctgatatc gagcatggcc 840

ctcctggggt tcacccttcc gccagattcc ggggagaagc tcaccttagg agtgaccatt 900

ctactgtcgc tgacagtatt cctgaacctc gttgcagaaa gtatgccgac cacctcggac 960

gctgtccctc taatagggtc gtacttcaat tgcatcatgt tcatggtggc gagcagcgtc 1020

gttctgacag tgttggtgct gaatttccat cacagaacgc ccgacagata cgtgatgcca 1080

tcgtggatta aaatgctgtt tttacaatgg ttgccgtgct tgctgcgcat gagtcgtcca 1140

ggaaagaaga tcacgaagaa aaccattctc ggaggcaata gaagggcaaa gggcatggaa 1200

ttgcaggaga agagtagcaa gagtttgcta gcaaatgtat tagatatcga cgacgacatt 1260

cgtcacaaga atagtgcggc aaatcctcct tccggttaca taaggtcggc gttcggtacg 1320

ccgatatcta ccgggagacc agcaacagtc gaagatacgt ccgcatcgtt acctcttagc 1380

gggatgcagg aggaattgca cacgattctt aaggaattac gattcattac ggatcgtatg 1440

aaaaaggcgg atgaaagcga cgagatcatt agcgactgga aattcgccgc tatggtggtg 1500

gacaggcttt gcttgatcgt tttcacgttg ttcacggttt tggccacgat agtgatattg 1560

tgtcgtgcgc cacacataat cgtccaataa 1590

<210> 7
<211> 1668
<212> DNA
<213> Apis mellifera

<220>
<221> source
<222> 1..1668
<223> /mol_type="unassigned DNA"
/note="nicotinic acetylcholine receptor alpha7 subunit nucleic acid sequence"
/organism="Apis mellifera"

<400> 7
atgagacgtt ggactctcat ggcggctata gccctggctg catcggggct ggtaaacggc 60

ggttctcacg agaaacggct gctaaacgac ctgctggata catacaacgt gctggagcgt 120

ccggttggca atgagtccga gcccctcgtg ttgagctttg gccttacact aatgcaaata 180

atcgacgttg acgaaaagaa ccaattgctc atcacgaatc tctggttaaa attggaatgg 240

aacgatgtga acatgagatg gaatgtgtca gattatgggg gagtgagaga cctcaggatc 300

ccaccacaca gactttggaa gcctgacgtt ctcatgtata acagcgcgga cgaagggttc 360

gacggcactt atccgacgaa cgtcgttgtg aagaacaatg ggacctgctt atacgtgccg 420

cccggcatat tcaagagcac ttgcaagata gacattacct ggttcccctt tgacgatcaa 480

cgctgcgaga tgaaattcgg ctcctggacg tacgacggct ttcagttgga cctgcaactg 540

caagacgaag cgggaggtga catcagcagt ttcatcacta acggcgagtg ggatctgttg 600

ggggtgcctg gtaaaagaaa cgaaatttat tacaattgct gcccagaacc gtatatagat 660

ataacgttcg tggtgatcat cagaaggcga actctttact atttcttcaa cctgatcgtg 720

ccgtgtgtcc tgattgccag catggccgtt ctgggattca ccttgccacc cgattccggc 780

gagaagctat ctctaggggt aaccatcctc ttgtccctca ctgtgttcct gaatatggtg 840

gccgagacaa tgccagcgac ttcggacgcc gtgcctctgc tggggacgta cttcaactgt 900

attatgttta tggtggccag cagtgttgtc agcaccattc ttattttaaa ttatcatcat 960

cggaatgctg acactcacga aatgtctgaa tgggtgaaag tggtattcct ttattggctg 1020

ccttgtatac ttcgtatgtc acgaccaagt gataaggagg agagggaggc gcaaaaatct 1080

caaaaaccgt ctccagtcac cggcgctagc aagagtcacg gtgacttgga gcttcgtcaa 1140

aggagcagca aatccctcct agcgaatgtc ctggacctcg aggacaacgc tctggcgtcc 1200

cacaataatc tgttgaacaa cgtgtacagc accccctggcc cccaccatca cacgatgggc 1260

cacgggcaca gtcacataca cgcgaccccc catcatcatc acagccacgc tgcaacgccg 1320

catcatcaac actcgacgcc actggcacac tcctcttatc cggcagcgat tcagattggt 1380

cacacgccgc atcatcatcc gcatccgcca gagacgcctg gccctcaggt cgaaacgata 1440

cttcaaaacg cgtgtttctg cgcccgaaac gagctcatga tgatcctcaa agagattaag 1500

ataatcacgg atcagctgaa aagcgaggag ttgaacacga aggtgacgaa cgactggaag 1560

ttcgcggcga tggtgatcga cagaatgtgc ctaatcattt ttactctgtt caccatcatc 1620

gcaaccatcg ccgtcctact gtcggcgcca cacatcatcg tcacgtga 1668

<210> 8
<211> 1614
<212> DNA
<213> Apis mellifera

<220>
<221> source
<222> 1..1614
<223> /mol_type="unassigned DNA"
/note="nicotinic acetylcholine receptor alpha8 subunit nucleic acid sequence"
/organism="Apis mellifera"

<400> 8
atgtttaaaa tgcaaatatt gacgcttggt gttcttttta atactcttca tattatatac 60

agtgttgctg gattgaaaat tttcgaagca aatcctgaca caaagagact ttatgatgac 120

ttattatcga attataatag acttatacga cctgttatga acaatactga aaccttgaca 180

gttcaactcg gtttaaaatt atcacaatta attgaaatga atttaaaaaa tcaagtgatg 240

accaccaatg tctgggtaga acagagatgg aatgattata aactaaaatg gaatccagaa 300

gaatacggtg gcgtggaaat gctatatgta ccttccgaaa atatttggtt accagatatt 360

gttctatata ataatgctga cggtaattat gaagtgacgc ttatgacaaa agctacatta 420

aaatatactg gtgatgtatc ttggaaacca cctgcaattt ataaatcatc ttgtgaaatt 480

aatgtagaat atttcccatt cgacgaacaa tcgtgtatta tgaaattcgg ttcatggact 540

tataatggcg ctcaggtaga tttaaaacat atgaaacaag aagctggtag caatttagtt 600

gcaaaaggaa tagatttaag cgatttctat ttatcagtag aatgggacat tttagaagta 660

ccagcatcga gaaatgaaga atattatcca tgttgcacag aaccctattc tgatattacc 720

tttaatatta caatgcgaag aaaaacatta ttctatacgg ttaacttaat aattccttgc 780

gtgggtatta cattccttac agtacttgta ttttatctac caagtgattc tggtgaaaaa 840

gtatcattat gttcttccat tctcctttca ttgacggtat tctttttatt attagccgaa 900

attattccac caacatctct ggctatacca cttttaggaa aatatttact atttaccatg 960

attttagtca ctttgtcgat atggattact gtatgtgttt taaatgtcca ttttcgatct 1020

ccatctacac ataatatgtc accatgggtt agacaagtat ttttaaattg gatgccgcga 1080

cttctaatga tgcgtagaac tccatattca actccagaat atgatgatac atatatggat 1140

agcggatata ctaatgaaat agattttagt tttcctgata gcgttagtga ttatccactg 1200

gaattaaaag gaagtcctga tggatttgaa agtgttacct ctcaatataa aaatatacga 1260

gaagatgatg ctcgacatat tccacatgca tcagttactg atagtgaaaa tacagtccca 1320

agatatttat ctccggatgt tatctcagct cttaaaggtg tgcgttttat tgctcaacat 1380

ataaaaaatg cagacaaaga taatgaagtt atagaagatt ggaaattcgt agctatggtt 1440

ttagatagac tttttctttg ggtttttaca ttggcatgta ctgcaggaac acttggtata 1500

atatttcaag caccaagttt atatgataca agagagcctg tggaccaaca actttctgga 1560

atatcacctc gcaattatat gtatccaaat atagacattt ctccagaagg ataa 1614

<210> 9
<211> 1296
<212> DNA
<213> Apis mellifera

<220>
<221> source
<222> 1..1296
<223> /mol_type="unassigned DNA"
/note="nicotinic acetylcholine receptor alpha9 subunit nucleic acid sequence"
/organism="Apis mellifera"

<400> 9
atgaaaatga gaataataac agctcttgga ttcctccttt tttggcagaa ttcggcaagc 60

ttagaaaacg acaatgaatt cggattctcg tatctgttaa attgcaagaa ttacgaccat 120

ccaaccacct tgttgaaatt gaagagatat cttttctgcg agtatgatcc taatgtccga 180

ccgatctcgt cccatcaaat cgccaacaac gtgaccatgc aactacttcc aaaactgatg 240

gaattcgatg attggaccag cgtgatggaa ttgcacagtt ggatgactct catgtggacc 300

gactctcatc tgtcatggaa gcctagcgac ttcgacggga ttaattatat ttacgtgaag 360

agcgacgata tttgggttcc cgacatttcc gtttacaatt caggcgacat gacgttcgat 420

caaactggta taccaccgac cacgtgcctg gtgttcagtt cgggttcggt aagctgcgta 480

ccatcggtga aacacgttgc caaatgcgcg acagatttct cctcgtggcc ctacgacact 540

catcgatgcc ggattaattt cggctcgtgg gtccattcag gggaggaagt gaacattttt 600

ctcgacaaaa aggggttcca catggacggg tatacgaata actcgaaatg ggatttcaaa 660

gtgataaaag cgaccaaagt gttgaagatg tacgcttgtt gcccgaacga cacttatccg 720

atgatcgttt acgaattctc cataagccga cattacggca tattgcacgc cacctacgtc 780

atacctgccg taacgatgat gttgctcacg ttgacggtat tatggttaga ttcgagatca 840

acggagagaa tgatcgcagc gagcgtgaat ttgatctgtc atatactttg catgtccgat 900

ttgcattggc aattgcctca taatagcacc aatcctccca atatactgct ctactataga 960

gattcgcttg ccttgtccgt attcgcttta attttaaccg ctctgcttcg gaaaatgcaa 1020

gaaatgagca tcgaggtgcc atattggatt tctacgacaa catcgttcgt cttgaacaac 1080

agagccgggc gtttccttat tcttaccgag agcgatacca aattgtccag ccaggggata 1140

cttggcgaag acgttgagaa caattcggaa gtttcgaaat ctcggactaa ggaatcggcg 1200

tggagacatt tcgcggccat aatcgagtgg ctctcgttct tcatcgtgat tttcacttac 1260

atcatcattc tgatcactct tgtaccatcc acgtga 1296

<210> 10
<211> 1563
<212> DNA
<213> Apis mellifera

<220>
<221> source
<222> 1..1563
<223> /mol_type="unassigned DNA"
/note="nicotinic acetylcholine receptor beta1 subunit nucleic acid sequence"
/organism="Apis mellifera"

<400> 10
atgcataata tttgctcgag gctcgggcga attctgctca tctccgccgt tttctgcgtc 60

ggtctctgct ccgaggatga ggaaagattg gtgcgagact tgttcagagg ttacaacaaa 120

ctcattagac ccgtgcagaa catgacagag aaagtgcacg tgaattttgg cctcgctttc 180

gtgcaactga tcaacgtgaa cgagaaaaat caaattatga agtcgaacgt ttggctgaga 240

ttcatctgga cggattatca gctgcaatgg gacgaggcag actatggcgg tatcggggta 300

cttagattac cacccgacaa agtatggaaa cctgacatcg tgttgtttaa caacgcggac 360

gggaactacg aggtgcgata caagagcaac gtgctcatct atccgaacgg cgacgtcctc 420

tgggtgccac cggccatcta ccagagttcc tgcaccatcg acgtcacgta cttcccgttc 480

gaccagcaaa cctgcatcat gaaattcggc tcctggacgt tcaacggcga ccaagtgtcc 540

ctggctttgt acaacaacaa gaacttcgtc gatctgtccg actattggaa gagcggcacc 600

tgggacataa tcaacgttcc tgcctacctc aacacctaca aaggcgactt tccaaccgag 660

actgacatca ccttctacat aatcatcaga cgaaaaacgt tgttctacac ggtgaacctg 720

atcctgccta ccgtgctcat ctctttccta tgcgtgctcg tgttctacct tccagccgag 780

gctggcgaga aagtgacgct tgggatcagc atcctcctct cgttggtcgt gttcctgttg 840

ttggtcagca agatcctgcc gccgacctcg ttggttctcc cgttgatagc caagtatctt 900

ctgttcacgt tcatcatgaa cacggtcagc atcctggtca cggtgatcat aatcaattgg 960

aacttcaggg ggccgaggac ccacagaatg ccccaattga tcagaaagat attcctcaag 1020

tatctgccca ctatactgat gatgaggagg cccaagaaga cgcgtctcag gtggatgatg 1080

gagattccga acgtgacgtt gcccacttcc acttactcgg gcagcccgac agagttgccc 1140

aaacatctgc ccacctcgtt gacgaaatcc aagatggagg tgatggagtt gtcggatctt 1200

```
catcatccga attgcaagat caacaggaag gttcatcaca cgacgagcag ctcgagcgcg    1260

gccggcgggg aaggcctagc agatagaagg ggatcggaga gctccgattc cgtgttgctc    1320

agccccgagg ctagcaaagc tacggaggct gtcgagttca ttgccgagca tctgagaaac    1380

gaagatttgt acatacagac gagggaagat tggaagtacg tggcgatggt gatcgatcga    1440

ctgcagcttt acattttctt cctggtgacg acagcgggta ccatcgggat cctgatggac    1500

gcacctcaca tcttcgagta cgtggaccag gatcatataa tagagatcta ccgtggaaaa    1560

taa                                                                  1563
```

```
<210> 11
<211> 1284
<212> DNA
<213> Apis mellifera

<220>
<221> source
<222> 1..1284
<223> /mol_type="unassigned DNA"
      /note="nicotinic acetylcholine receptor beta2 subunit nucleic
      acid sequence"
      /organism="Apis mellifera"

<400> 11
atgttaaaca tgaagaatat attccccgtt ttattcgtga tcattaatgt tttattacat      60

ggacaagtga tctgttttgt ttgcaaagac atcacaagca cttctgcact ttacagattg     120

aaactgtatc tgttctgcga ttacgacagg gacattattc ccgaacaaaa aaatgccact     180

aaaatcgatt ttggattaag cattcaacat tataacgttg atgaatattc gcacaccgtc     240

gatttccacg ttatgctaaa attgatgtgg gaacagagtc acctcacgtg gaaatcatcc     300

gaattcgatt ctatcaattc tattcgcgta aagagttacg aaatctgggt ccccgatatc     360

gtgatgcaca gcgtaacaag cgtcggtatc gatcttgaga tgccctctgt cgaatgcata     420

gtgttcaatt ctggcactat actttgcgta ccgttcacta cgtatacacc tgtttgcgaa     480

tatgatcaca cttggtggcc ttacgatata ttgaactgca ctatacacat tgcctcgtgg     540

tcccacggaa gcaacgagat taaattaaac tctttggata ctgaacaaat tttagatgat     600

atgtacaata ataacacgga atgggagata gtgcatatgt ctcacagcga gagcacgata     660

gattccaaat ttggtttagg ttttaccact gatttgttat cttataatat tctgctgaga     720

agacattact ccatgaacag tacgacgtat gtgactttga ccatagtatt aatgaccatg     780

acattgatga cattatggtt agagccaagt tctacggagc gtatgatcat agcaaatctg     840

aattttattc tgcatctatt ttgcttgtta gatgtgcaat ggaggattcc tttcaatgga     900

attcaaatgc caaacttgat ggtgttctat gagaaatctc ttgccctagc cgcattctcg     960

cttatgttga caagcatctt gcgatatttg caagaattac acgttgacgc tccaacatgg    1020
```

atatcctccg tcactgaatc ggttttaaaa agtaaaatag gacaagtgtt tttgatcact 1080

attctggatt caaaagtatc ggcaaggatc gaaatgaacg aggatgataa cacgagtttg 1140

gtatctctgg ataagaaaca gtatacatgg agacacacgt ccgttttaat cggatggagt 1200

gctttttat gcatttcatt tgtttatata attatgctaa ttatttttat accaagaaac 1260

atatatgaaa attttataag ttaa 1284

<210> 12
<211> 1365
<212> DNA
<213> Apis mellifera

<220>
<221> source
<222> 1..1365
<223> /mol_type="unassigned DNA"
/note="chaperone protein resistance to inhibitors of
cholinesterase 3 (ric-3) nucleic acid sequence"
/organism="Apis mellifera"

<400> 12
atggctgaaa taacagattt cggtccgaga aagacgattt ttatcttagc cattgtagcc 60

gggtgttttg cggtattatg gccgaaaatt ttctatccta tgctcacagc atctgtcaat 120

ccacatcata taaccgacaa ttctgcatgc tgcggtgtaa tctttgaaag tgatgttact 180

gcagctgata ttatgtatga aatatgtcag aatatattga aacaccatca aattgatcca 240

agaataagag atgctttgag aactataaaa ttaacaccac aaagtgcaag tttatgtaga 300

gaagaaattt tagctagatg tggtatagat ttatctacat ttcttgctaa aagagaacat 360

ctggaaaaat cttataaaca agttttagaa gaaataagat catttaatag ttctctttgt 420

cttaaaataa attttggtat acctctttct caactaggaa ctccacattt aatcagatat 480

catattttaa tgccacacaa tacaataaaa caagaacgac gtacacctcc acatgcaggt 540

ggtttacatc ctgcattgag agaacgtggt agagctatac cttcttctca tattgtacca 600

aaggtttcag atagacctga tcatgttgtg ccaaaaatga gaccaccttt aggtggtgca 660

ggacatgttg ttcctgcacc aaaaggaagt ggtaccatgg gaattattat gccattgtat 720

actttaggaa ttgtactttt tttcttatat acaattgtta aggtgctgag aaaaaattcg 780

gatagtgaaa ttatttcaga atatccaggt gctgcagcag aaaaagaatt tcgaaaaatg 840

gtattcagtc ctgaagcttt tgctactgca atgactggtg gtacaatgaa ttatcagaag 900

gaaagatctc catcaccaca aagacctact cctaccttag aggaattaaa agacctagat 960

gggcctccaa agtccaaagg cctggcaggg attgatcgct catctaagga caataccatt 1020

gctcaggttc aatatagtac agaagataaa gtagaaaaca tagaacattc accaactatt 1080

aaagtcatgg gtatggaaat gacagctagt tgtgagaata agggtagtag acctactact 1140

cctataattc caatttcacc aagtcatatt gagagggaaa aaacaccacc aaaaccaata 1200

tatttagaag gtgcacttcc tccacaatgt gaattgcttg taacagattc agaaactcaa 1260

gctcagaaag cagaagaaga tgtagaggca cctgttgtac tttcaggcaa aatgactctc 1320

tctctcatca gtctcgacca aaatgcagct gtaagaaaat attag 1365

<210> 13
<211> 342
<212> DNA
<213> Apis mellifera

<220>
<221> source
<222> 1..342
<223> /mol_type="unassigned DNA"
/note="chaperone protein ER membrane protein complex-6 (emc-6) nucleic acid sequence"
/organism="Apis mellifera"

<400> 13
atgttgggaa agattaaaac aaaacaagag aaaggagaga tcattgcata tagtgaatct 60

gctgttgtaa ataatgctgc agtagtagaa tattgtagga tatcaatggc agcattatca 120

ggtggcacag ctggtttatt aggattaact ggattatatg gttttggttt ttacattttt 180

gctgtatttg gattatgggc aatgttattg ttaaaagctg gaggtcaatg gaaaaaatat 240

tttataagta gacgaaatct tctaactagt ggatttttttg gaggcctttt tacatatgtg 300

ctgttttgga catttttata tggaatggta catgtatact ga 342

<210> 14
<211> 804
<212> DNA
<213> Apis mellifera

<220>
<221> source
<222> 1..804
<223> /mol_type="unassigned DNA"
/note="chaperone protein unc-50 nucleic acid sequence"
/organism="Apis mellifera"

<400> 14
atgaaatatt ctacatcacc accagtaagt agatgtaatt ctcctggtcc tagtgaaaca 60

agttcaaatt tacccatgcc agtaacttac aggcaagatt gtatgggtgc agcaacaaaa 120

tgttacaagt acttaagaaa attattaaaa tttgaacaaa tggattttga attcgcttta 180

tggcaaatga tttttttatt tatatcacca caaaaagtat atagaaattt tcaaaataga 240

aaacaaacaa aatctcaatt tgcaagagat gatcctgcat ttttggtatt attaatgtgt 300

tgtctatgta tatcttctgt tggttttacc atagttttgg gattaggatt tcttcaattt 360

ataaaattat tattttatat gatatttatt gattatcttg cagctggttt aatagtagct 420

acaatatttt ggtttataac aaatcattat ttaagaattg ataaaactca ggatgtagaa 480

tggggttatg catttgatat acatttaaat gcattttttc cacctttaat tatactccat 540

atcgtacaat tgtttttata taaaagtttt ataaactatg atacattttc atcacgattt 600

gttggaaaca caatctggtt aatagctgtt agttactata tttacataac atttttaggt 660

tacacaagta tggaaatagt tcataagaca catatgatat tatcaacatt accaataata 720

ttattgatat atattatgac attatgtgca ggaattaata ttagtcattt agttatggaa 780

ttttatcatt atcgagttgt ttga 804

<210> 15
<211> 1296
<212> DNA
<213> Apis mellifera

<220>
<221> source
<222> 1..1296
<223> /mol_type="unassigned DNA"
/note="chaperone protein unc-74 nucleic acid sequence"
/organism="Apis mellifera"

<400> 15
atggtaataa taacgaaatt gatgtttatt gcagttattt atgtttttc aggtactttt 60

acttctgtaa tagcatcacg tgtacttgaa ttaagtgata gatttttgga tattcataaa 120

gatggacaat ggcttgtaat gatgtacgca ccatggtgtg cacattgcaa aagattagaa 180

ccaatttggg ctcatgtagc acaatattta catgcaacat caatacgagt tggacgagtt 240

gattgtacta gatttacaaa tgttgctcat gcttttaaag ttaaaggatt tccaactatt 300

atatttttaa aaggtgagca agaatttata tataatgggg atagaacacg agatgaaata 360

gtaaaatttg cattaagagt aagtggccct ccagttcagg gaataacaaa aactcaaagt 420

tttgacacta ttaaaaagga acatgatata tattttttat atgttggaga acgatctggt 480

ccattatggg aattttatca taagactgca aatgtgttcc aaccacatgc atttttctac 540

caatctcatc caaatattgt tagtaaacat gctcctgtag aaaatactcc agcattattt 600

gtttataagg aaaatataca ttacaacttc agtgatcata atatagatga tatagaaaaa 660

ttaaatgaaa caatgtataa atggataaat gcagaacgtt ttcccacatt tccaaaagta 720

acaagaggaa acataaatca actttttttg acaaataaaa atttggtttt ggcagtagta 780

gaagaaaatc aattagaaaa aataccatta cacatggcac gatttaaaga tatggtagaa 840

tctatcatta aaaataaacg agaaaaatat catgattatt ttcagtttgg ttggatagct 900

agtccagatc tagtcaatag tatagcaatg atggttttac cattaccaag tttaattgtt 960

attaatacta caacaaatca tcatcacatt ccggaggatg aaaccgaaaa attaactcct 1020

```
catgtgatag aattattcct agagcaaatt cgaaatgaaa gtgctccgcg gtatggtgga    1080

aatagttggt tgatacgtat atatagatca tggtttgaat taaaaacaac tctttctgca    1140

atgtggatgg gtaatcccat tctaacaatg gtcttatttg gtttaccagc aggattctta    1200

tcattaatat gttatggtat ttgttgtcca gatattttag atgcagatga tgacgaagaa    1260

gaccatccag gagcaaatca tatgaagaaa gattag                              1296


<210> 16
<211> 601
<212> PRT
<213> Apis mellifera

<220>
<223> nicotinic acetylcholine receptor alpha1 subunit amino acid
      sequence

<400> 16
Met Ala Thr Ala Ile Ser Cys Leu Val Ala Pro Phe Pro Gly Ala Ser
1               5                   10                  15
Ala Asn Ser Glu Ala Lys Arg Leu Tyr Asp Asp Leu Leu Ser Asn Tyr
            20                  25                  30
Asn Arg Leu Ile Arg Pro Val Gly Asn Asn Ser Asp Arg Leu Thr Val
        35                  40                  45
Lys Met Gly Leu Arg Leu Ser Gln Leu Ile Asp Val Asn Leu Lys Asn
    50                  55                  60
Gln Ile Met Thr Thr Asn Val Trp Val Glu Gln Glu Trp Asn Asp Tyr
65                  70                  75                  80
Lys Leu Lys Trp Asn Pro Asp Asp Tyr Gly Gly Val Asp Thr Leu His
                85                  90                  95
Val Pro Ser Glu His Ile Trp Leu Pro Asp Ile Val Leu Tyr Asn Asn
            100                 105                 110
Ala Asp Gly Asn Tyr Glu Val Thr Ile Met Thr Lys Ala Ile Leu His
        115                 120                 125
His Thr Gly Lys Val Val Trp Lys Pro Pro Ala Ile Tyr Lys Ser Phe
    130                 135                 140
Cys Glu Ile Asp Val Glu Tyr Phe Pro Phe Asp Glu Gln Thr Cys Phe
145                 150                 155                 160
Met Lys Phe Gly Ser Trp Thr Tyr Asp Gly Tyr Thr Val Asp Leu Arg
                165                 170                 175
His Leu Ala Gln Thr Glu Asp Ser Asn Gln Ile Glu Val Gly Ile Asp
            180                 185                 190
Leu Thr Asp Tyr Tyr Ile Ser Val Glu Trp Asp Ile Ile Lys Val Pro
        195                 200                 205
Ala Val Arg Asn Glu Ala Phe Tyr Ile Cys Cys Glu Glu Pro Tyr Pro
    210                 215                 220
Asp Ile Val Phe Asn Ile Thr Leu Arg Arg Lys Thr Leu Phe Tyr Thr
225                 230                 235                 240
Val Asn Leu Ile Ile Pro Cys Val Gly Ile Ser Phe Leu Ser Val Leu
                245                 250                 255
Val Phe Tyr Leu Pro Ser Asp Ser Gly Glu Lys Val Ser Leu Ser Ile
            260                 265                 270
Ser Ile Leu Leu Ser Leu Thr Val Phe Phe Leu Leu Leu Ala Glu Ile
        275                 280                 285
Ile Pro Pro Thr Ser Leu Thr Val Pro Leu Leu Gly Lys Tyr Leu Leu
    290                 295                 300
Phe Thr Met Val Leu Val Thr Leu Ser Val Val Val Thr Ile Ala Val
305                 310                 315                 320
Leu Asn Val Asn Phe Arg Ser Pro Val Thr His Arg Met Ala Arg Trp
                325                 330                 335
```

```
Val Arg Val Val Phe Ile Gln Val Leu Pro Arg Phe Leu Leu Ile Glu
            340                 345                 350
Arg Pro Lys Lys Asp Glu Asp Glu Glu Glu Glu Glu Glu Val Val Val
        355                 360                 365
Val Gly Arg Asn Gly Ala Gly Val Gly Ala Met Asn Ala Asn Gly Glu
    370                 375                 380
Ala Val Gly Glu Val Asp Glu Asp Asp Asp Asp Asp Gly Asp Asp Asp
385                 390                 395                 400
Asp Asp Asp Asp Val Glu Ala Ala Asn Gly Lys Pro Ala Glu Gly Met
                405                 410                 415
Leu Thr Asp Val Phe His Val Gln Glu Thr Asp Lys Tyr Asp Ala Tyr
            420                 425                 430
Tyr Gly Asn Arg Phe Ser Gly Glu Tyr Glu Ile Pro Ala His Gly Leu
        435                 440                 445
Pro Pro Ser Ala Thr Arg Tyr Asp Leu Gly Ala Val Ala Thr Val Gly
    450                 455                 460
Thr Thr Val Ala Pro Cys Phe Glu Glu Pro Leu Pro Ser Leu Pro Leu
465                 470                 475                 480
Pro Gly Ala Asp Asp Asp Leu Phe Gly Pro Ala Ser Pro Ala Tyr Val
                485                 490                 495
His Glu Asp Val Ser Pro Thr Phe Glu Lys Pro Leu Val Arg Glu Ile
            500                 505                 510
Glu Lys Thr Ile Asp Asp Ala Arg Phe Ile Ala Gln His Ala Lys Asn
        515                 520                 525
Lys Asp Lys Phe Glu Ser Val Glu Glu Asp Trp Lys Tyr Val Ala Met
    530                 535                 540
Val Leu Asp Arg Ile Phe Leu Trp Ile Phe Thr Val Ala Cys Val Leu
545                 550                 555                 560
Gly Thr Val Leu Ile Ile Leu Gln Ala Pro Ser Leu Tyr Asp Thr Thr
                565                 570                 575
Lys Pro Ile Asp Ile Lys Tyr Ser Lys Ile Ala Lys Lys Lys Met Met
            580                 585                 590
Leu Met Asn Met Gly Pro Glu Glu Asp
        595                 600
```

<210> 17
<211> 541
<212> PRT
<213> Apis mellifera

<220>
<223> nicotinic acetylcholine receptor alpha2 subunit amino acid sequence

<400> 17

```
Met Ile Leu Gln Thr Ile Ile Leu Ile Leu Ser Ala Arg Ile Cys His
1               5                   10                  15
Gly Asn Pro Asp Ala Lys Arg Leu Tyr Asp Asp Leu Leu Ser Asn Tyr
            20                  25                  30
Asn Arg Leu Ile Arg Pro Val Ser Asn Asn Asn Asp Thr Val Val Val
        35                  40                  45
Lys Leu Gly Leu Arg Leu Ser Gln Leu Ile Asp Leu Asn Leu Lys Asp
    50                  55                  60
Gln Ile Leu Thr Thr Asn Val Trp Leu Glu His Glu Trp Gln Asp His
65                  70                  75                  80
Lys Phe Gln Trp Asp Pro Ala Glu Tyr Gly Gly Val Thr Glu Leu Tyr
                85                  90                  95
Val Pro Ser Glu His Ile Trp Leu Pro Asp Ile Val Leu Tyr Asn Asn
            100                 105                 110
Ala Asp Gly Glu Tyr Gly Val Thr Thr Met Thr Lys Ala Ile Leu His
        115                 120                 125
Tyr Thr Gly Lys Val Leu Trp Thr Pro Pro Ala Ile Phe Lys Ser Ser
    130                 135                 140
```

Cys Glu Ile Asp Val Arg Tyr Phe Pro Phe Asp Gln Gln Thr Cys Phe
145 150 155 160
Met Lys Phe Gly Ser Trp Thr Tyr Asp Gly Ile Gln Ile Asp Leu Lys
165 170 175
His Ile Asn Gln Asn Met Gly Asp Lys Val Glu Ile Gly Ile Asp Leu
180 185 190
Arg Glu Tyr Tyr Pro Ser Val Glu Trp Asp Ile Leu Gly Val Pro Ala
195 200 205
Glu Arg His Lys Lys Tyr Tyr Pro Cys Cys Asp Glu Pro Tyr Pro Asp
210 215 220
Ile Phe Phe Asn Ile Thr Leu Arg Arg Lys Thr Leu Phe Tyr Thr Val
225 230 235 240
Asn Leu Ile Val Pro Cys Val Ser Ile Ser Tyr Leu Ser Val Leu Ala
245 250 255
Phe Tyr Leu Pro Ala Asp Ser Gly Glu Lys Ile Ala Leu Cys Ile Asn
260 265 270
Ile Leu Leu Ser Gln Thr Met Phe Phe Leu Leu Ile Ser Glu Ile Ile
275 280 285
Pro Ser Thr Ser Leu Ala Leu Pro Leu Leu Gly Lys Tyr Leu Leu Phe
290 295 300
Thr Met Ile Leu Val Gly Leu Ser Val Val Ile Thr Ile Val Ile Leu
305 310 315 320
Asn Val His Tyr Arg Lys Pro Ser Thr His Lys Met Ala Pro Trp Val
325 330 335
Arg Lys Ile Phe Ile Arg Arg Leu Pro Lys Leu Leu Leu Met Arg Val
340 345 350
Pro Asp Asp Leu Leu Asn Asp Leu Ala Ala His Lys Met His Gly Arg
355 360 365
Gly Arg Ser Gly Lys Lys Ser Lys Phe Asn Ala Ala Val Ala Ala Ala
370 375 380
Val Gln Ser Ser Ser Ile Val Ser Ser Pro Asp Ser Ala Arg His Gln
385 390 395 400
Arg Ile Gly Gly Cys Asn Gly Leu His Thr Thr Thr Ala His Asn Arg
405 410 415
Phe Leu Gly Gly Ile Gly Gly Tyr Asn Gly Leu Pro Thr Val Met Ser
420 425 430
Gly Leu Asp Glu Ser Leu Ser Asp Val Thr Pro Arg Lys Lys Tyr Pro
435 440 445
Phe Glu Leu Glu Lys Ala Leu His Asn Val Met Phe Ile Gln His His
450 455 460
Ile Gln Arg Gln Asp Glu Phe Asn Ala Glu Asp Gln Asp Trp Gly Phe
465 470 475 480
Val Ala Met Val Leu Asp Arg Leu Phe Leu Trp Ile Phe Thr Ile Ala
485 490 495
Ser Ile Val Gly Thr Phe Ile Ile Leu Cys Glu Ala Pro Ala Leu Arg
500 505 510
Asp Asn Thr Lys Pro Ile Asp Met Glu Tyr Ser Ser Val Ala Gln Gln
515 520 525
Gln Phe Leu Pro His Asp Asp Phe Leu Glu Thr Leu Val
530 535 540

<210> 18
<211> 567
<212> PRT
<213> Apis mellifera

<220>
<223> nicotinic acetylcholine receptor alpha3 subunit amino acid sequence

<400> 18
Met Met Lys Ser Leu Val Gly Ile Met Trp Ile Val Leu Val Leu Ile
1 5 10 15

Ser Gly Cys Ser Gly Asn Pro Asp Ala Lys Arg Leu Tyr Asp Asp Leu
20 25 30
Leu Ser Asn Tyr Asn Lys Leu Val Arg Pro Val Val Asn Val Thr Asp
35 40 45
Ala Leu Thr Val Lys Ile Lys Leu Lys Leu Ser Gln Leu Ile Asp Val
50 55 60
Asn Leu Lys Asn Gln Ile Met Thr Thr Asn Leu Trp Val Glu Gln Ser
65 70 75 80
Trp Tyr Asp Tyr Lys Leu Lys Trp Asp Pro Lys Glu Tyr Gly Gly Val
85 90 95
Glu Met Leu His Val Pro Ser Asp His Ile Trp Arg Pro Asp Ile Val
100 105 110
Leu Tyr Asn Asn Ala Asp Gly Asn Phe Glu Val Thr Leu Ala Thr Lys
115 120 125
Ala Thr Leu Asn Tyr Thr Gly Arg Val Glu Trp Lys Pro Pro Ala Ile
130 135 140
Tyr Lys Ser Ser Cys Glu Ile Asp Val Glu Tyr Phe Pro Phe Asp Glu
145 150 155 160
Gln Thr Cys Val Met Lys Phe Gly Ser Trp Thr Tyr Asp Gly Phe Gln
165 170 175
Val Asp Leu Arg His Ile Asp Glu Ile Arg Gly Lys Asn Val Val Asp
180 185 190
Ile Gly Val Asp Leu Ser Glu Phe Tyr Thr Ser Val Glu Trp Asp Ile
195 200 205
Leu Glu Val Pro Ala Val Arg Asn Glu Lys Phe Tyr Thr Cys Cys Asp
210 215 220
Glu Pro Tyr Leu Asp Ile Thr Phe Asn Ile Thr Met Arg Arg Lys Thr
225 230 235 240
Leu Phe Tyr Thr Val Asn Leu Ile Ile Pro Cys Met Gly Ile Ser Phe
245 250 255
Leu Thr Val Leu Val Phe Tyr Leu Pro Ser Asp Ser Gly Glu Lys Val
260 265 270
Ser Leu Ser Ile Ser Ile Leu Leu Ser Leu Thr Val Phe Phe Leu Leu
275 280 285
Leu Ala Glu Ile Ile Pro Pro Thr Ser Leu Val Val Pro Leu Leu Gly
290 295 300
Lys Phe Val Leu Phe Thr Met Ile Leu Asp Thr Phe Ser Ile Cys Val
305 310 315 320
Thr Val Val Val Leu Asn Val His Phe Arg Ser Pro Gln Thr His Val
325 330 335
Met Ala Pro Trp Val Arg Arg Val Phe Ile His Val Leu Pro Arg Leu
340 345 350
Leu Val Met Arg Arg Tyr Asn Thr Pro Ser Lys Arg Ser Asp Tyr Asp
355 360 365
Ser Arg Pro Gln Tyr Gln Ile Asp Lys Arg Ser Met Gly Ser His His
370 375 380
Gly Gln Arg Val Met Val Arg Thr Cys Asn Gly Leu Glu Leu Arg Asp
385 390 395 400
Pro Ser Leu Phe Val Glu Thr Ser Ala Ser Glu Leu Val Glu Ser Ser
405 410 415
Val Leu Phe Pro Ser Leu Asp Ser Arg Asp Glu Leu His Pro Arg Glu
420 425 430
Leu Glu Ala Val Asn Leu Gly Ser Ala Cys Arg Ile His Gly Ser Pro
435 440 445
Ala Thr Thr Ala Ala Pro Pro Pro Gln Leu Pro Thr Glu Glu Ser Val
450 455 460
Asp Ala Leu Cys Asn Thr Leu His His Trp His His Cys Pro Glu Ile
465 470 475 480
Tyr Lys Ala Ile Glu Gly Ile Arg Phe Ile Ala Asp His Thr Lys Arg
485 490 495
Glu Glu Asp Ser Thr Arg Val Lys Glu Asp Trp Lys Tyr Val Ala Met
500 505 510
Val Leu Asp Arg Leu Phe Leu Trp Ile Phe Thr Leu Ala Val Val Val

```
        515                 520                 525
Gly Thr Ala Gly Ile Ile Leu Gln Ala Pro Thr Leu Tyr Asp Asp Arg
    530                 535                 540
Ile Pro Ile Asp Val Arg Leu Ser Glu Ile Ala Ser Thr Thr Ala Lys
545                 550                 555                 560
Pro His Ile Val Thr Ser Leu
                565

<210> 19
<211> 569
<212> PRT
<213> Apis mellifera

<220>
<223> nicotinic acetylcholine receptor alpha4 subunit amino acid
      sequence

<400> 19
Met Pro Pro Ile Ile Gly Glu Thr Leu Arg Val Trp Phe Leu Ser Ala
1               5                   10                  15
Leu Val Val His Gly Ala Val Ala Gly Asn Pro Asp Ala Lys Arg Leu
            20                  25                  30
Tyr Asp Asp Leu Leu Ser Asn Tyr Asn Lys Leu Val Arg Pro Val Val
        35                  40                  45
Asn Thr Ser Asp Val Leu Arg Val Cys Ile Lys Leu Lys Leu Ser Gln
    50                  55                  60
Leu Ile Asp Val Asn Leu Lys Asn Gln Ile Met Thr Thr Asn Leu Trp
65                  70                  75                  80
Val Glu Gln Ser Trp Tyr Asp Tyr Lys Leu Arg Trp Glu Pro Lys Glu
                85                  90                  95
Tyr Gly Gly Val Lys Met Leu His Val Pro Ser Asp His Ile Trp Arg
            100                 105                 110
Pro Asp Ile Val Leu Tyr Asn Asn Ala Asp Gly Asn Tyr Glu Val Thr
        115                 120                 125
Leu Met Thr Lys Ala Thr Val Tyr Tyr Ser Gly Leu Val Val Trp Gln
    130                 135                 140
Pro Pro Ala Val Tyr Lys Ser Ser Cys Ser Ile Asp Val Glu Phe Phe
145                 150                 155                 160
Pro Tyr Asp Val Gln Thr Cys Val Leu Lys Leu Gly Ser Trp Thr Tyr
                165                 170                 175
Asp Gly Phe Lys Val Asp Leu Arg His Met Asp Glu Lys Ser Gly Ser
            180                 185                 190
Asn Val Val Asp Val Gly Val Asp Leu Ser Glu Phe Tyr Met Ser Val
        195                 200                 205
Glu Trp Asp Ile Leu Glu Val Pro Ala Val Arg Asn Glu Lys Phe Tyr
    210                 215                 220
Thr Cys Cys Asp Glu Pro Tyr Leu Asp Ile Thr Phe Asn Ile Thr Met
225                 230                 235                 240
Arg Arg Lys Thr Leu Phe Tyr Thr Val Asn Ile Ile Ile Pro Cys Met
                245                 250                 255
Gly Ile Ser Phe Leu Thr Val Leu Thr Phe Tyr Leu Pro Ser Asp Ser
            260                 265                 270
Gly Glu Lys Val Thr Leu Ser Ile Ser Ile Leu Ile Ser Leu His Val
        275                 280                 285
Phe Phe Leu Leu Val Val Glu Ile Ile Pro Pro Thr Ser Leu Val Val
    290                 295                 300
Pro Leu Leu Gly Lys Tyr Leu Ile Phe Ala Met Ile Leu Val Ser Ile
305                 310                 315                 320
Ser Ile Cys Val Thr Val Val Val Leu Asn Val His Phe Arg Ser Pro
                325                 330                 335
Gln Thr His Lys Met Ala Pro Trp Val Lys Arg Val Phe Ile His Ile
            340                 345                 350
Leu Pro Arg Leu Leu Val Met Arg Arg Pro Gln Tyr Lys Phe Glu Thr
```

```
        355                 360                 365
Asn Arg Tyr Ser Ser Gly Arg Val Leu Met Arg Thr Val Arg Gly Lys
    370                 375                 380
Glu Lys Thr Cys Tyr Tyr Pro Tyr His Pro Ser Thr Gln Glu Asp Ser
385                 390                 395                 400
Glu Glu His Leu Thr Pro Lys Arg Phe His Ser Arg Ala Ala Ser Lys
                405                 410                 415
Glu Asp Leu Ser Pro Ser Ser Leu Ala Asp Gly Ala Arg Phe Gly Gly
            420                 425                 430
Ser Cys Leu Ile His Gly Pro Pro Leu Pro Pro Leu Pro Leu His Thr
        435                 440                 445
Glu Cys Glu Asp Leu Ser Val Ser Gly Glu Ala Gly Ile Glu Glu Val
    450                 455                 460
Lys Ser Pro Val Leu Arg Ser Pro Pro Ala Phe Ser His Ser Arg Cys
465                 470                 475                 480
Pro Pro Glu Ile His Lys Ser Cys Ile Cys Val Arg Phe Ile Ala Glu
                485                 490                 495
His Thr Lys Met Leu Glu Asp Ser Thr Lys Val Lys Glu Asp Trp Lys
            500                 505                 510
Tyr Val Ala Met Val Leu Asp Arg Leu Phe Leu Trp Ile Phe Thr Leu
        515                 520                 525
Ala Val Leu Val Gly Thr Ala Gly Ile Ile Leu Gln Ala Pro Thr Leu
    530                 535                 540
Tyr Asp Asp Arg Val Pro Ile Asp Lys Lys Phe Asn Glu Phe Gly Thr
545                 550                 555                 560
Thr Ala Val Val Asn Cys Pro Pro Gln
                565
```

<210> 20
<211> 481
<212> PRT
<213> Apis mellifera

<220>
<223> nicotinic acetylcholine receptor alpha5 subunit amino acid sequence

<400> 20

```
Met Ser Pro Leu Val Leu Phe Phe His Tyr Gly Val Leu Ala Ile Ile
1               5                   10                  15
Phe Gly Asn Gly Phe Gly Asp Glu His Glu Tyr Arg Leu Thr Lys Tyr
            20                  25                  30
Leu Leu Asp Gly Tyr Asp Ala Gly Val Arg Pro Ala Glu Asn Ser Ser
        35                  40                  45
Gln Pro Leu Ala Val Val Phe Gly Leu Ser Leu His His Ile Ile Asp
    50                  55                  60
Val Asp Glu Lys Asn Gln Ile Leu Thr Thr Asn Cys Trp Val Thr Gln
65                  70                  75                  80
Ile Trp Thr Asp His His Leu Lys Trp Asn Ala Ser Glu Phe Ala Gly
                85                  90                  95
Ile Arg Val Ile Arg Val Pro Tyr Asn Arg Val Trp Arg Pro Asp Thr
            100                 105                 110
Ile Leu Tyr Asn Asn Ala Asp Pro Gln Tyr Ser Ser Ala Val Ile Asn
        115                 120                 125
Thr Asn Val Ile Val Ser His Thr Gly Glu Val Val Trp Leu Ser His
    130                 135                 140
Gly Ile Phe Arg Ser Ser Cys Asp Ile Asp Val Glu Phe Phe Pro Phe
145                 150                 155                 160
Asp Glu Gln Arg Cys Val Leu Lys Trp Ala Ser Trp Thr Tyr Asp Gly
                165                 170                 175
Tyr Gln Leu Glu Leu Glu Lys Gln Ser Glu Gln Gly Asp Val Ser Asn
            180                 185                 190
Tyr Gln Ala Asn Gly Glu Phe Asp Leu Val Asn Phe Ser Ala Arg Arg
```

```
        195                 200                 205
Asn Val Glu Tyr Tyr Ser Cys Cys Pro Glu Pro Tyr Pro Asp Ile Thr
    210                 215                 220
Tyr Glu Ile Arg Leu Arg Arg Arg Pro Met Phe Tyr Val Phe Asn Leu
225                 230                 235                 240
Ile Leu Pro Cys Ile Leu Ile Asn Ser Val Ala Leu Leu Val Phe Tyr
                245                 250                 255
Val Pro Ser Glu Ser Gly Glu Lys Val Thr Leu Gly Ile Ser Ala Leu
            260                 265                 270
Leu Ser Met Thr Val Phe Leu Met Thr Ile Arg Glu Ser Leu Pro Pro
        275                 280                 285
Thr Glu Lys Thr Pro Leu Ile Ser Leu Tyr Tyr Gly Val Ser Ile Cys
    290                 295                 300
Leu Val Thr Phe Ala Ser Ala Leu Ala Val Val Thr Leu Asn Leu His
305                 310                 315                 320
His Arg Gly Val Arg Gly Thr Arg Val Pro Gly Ile Val Arg Ser Leu
                325                 330                 335
Val Leu Asp Lys Leu Ala Arg Ile Val Leu Leu Asn Phe Gln Glu Glu
            340                 345                 350
Arg Arg Ser Glu Pro Val Glu Pro Pro Arg Arg Lys Asn Asn Cys Pro
        355                 360                 365
Leu His Cys Lys Pro Glu Leu Gly Gln Ser Gln Ser Ser Pro Lys Phe
    370                 375                 380
Val Ala Arg Arg Glu Glu Ser Asn Ser Ser Ser Pro Ser Leu Asp Leu
385                 390                 395                 400
Gly Lys Glu Gly Gly Leu Glu Ala Gln Trp Ser Arg Val Leu Gly Arg
                405                 410                 415
Val His Ala Thr Ile Glu Arg Asn Glu Lys Arg Leu Ala Glu Gln Asp
            420                 425                 430
Arg Arg Glu Arg Met Glu Phe Asp Trp Lys Gln Val Ala Leu Val Ser
        435                 440                 445
Asp Arg Ala Leu Leu Cys Val Phe Phe Leu Thr Thr Ile Val Ser Thr
    450                 455                 460
Thr Val Ile Leu Cys Gly Ser Pro Pro Ser Ala Asn Ile Ala Lys Glu
465                 470                 475                 480
Gly
```

<210> 21
<211> 529
<212> PRT
<213> Apis mellifera

<220>
<223> nicotinic acetylcholine receptor alpha6 subunit amino acid sequence

<400> 21

```
Met Arg Ala Ser Ser Val Leu Gln Ala Glu Ser Asp Val Ser Ser Cys
1               5                   10                  15
Val Ile Phe Gly Val Leu Phe Val Leu Phe Ser Phe Leu Arg Thr Cys
            20                  25                  30
Thr Lys Leu Gln Thr Thr Tyr Phe His His Ile Tyr Ile Ile His Gln
        35                  40                  45
Ser Leu Cys Gly Arg His Glu Lys Arg Leu Leu Asn Glu Leu Leu Ser
    50                  55                  60
Ser Tyr Asn Thr Leu Glu Arg Pro Val Ala Asn Glu Ser Glu Pro Leu
65                  70                  75                  80
Glu Val Lys Phe Gly Ile Thr Leu Gln Gln Ile Ile Asp Val Asp Glu
                85                  90                  95
Lys Asn Gln Ile Leu Thr Thr Asn Ala Trp Leu Lys Leu Glu Trp Val
            100                 105                 110
Asp Tyr Asn Leu Gln Trp Asn Glu Ser Glu Tyr Gly Gly Val Lys Asp
```

```
        115                 120                 125
Leu Arg Ile Thr Pro Asn Lys Leu Trp Lys Pro Asp Ile Leu Met Tyr
    130                 135                 140
Asn Ser Ala Asp Glu Gly Phe Asp Gly Thr Tyr Gln Thr Asn Val Val
145                 150                 155                 160
Val Thr His Asp Gly Ser Cys Leu Tyr Val Pro Pro Gly Ile Phe Lys
                165                 170                 175
Ser Thr Cys Lys Ile Asp Ile Thr Trp Phe Pro Phe Asp Asp Gln His
            180                 185                 190
Cys Asp Met Lys Phe Gly Ser Trp Thr Tyr Asp Gly Asn Gln Val Asp
        195                 200                 205
Leu Val Leu Ser Ser Glu Thr Gly Gly Asp Leu Ser Asp Phe Ile Thr
    210                 215                 220
Asn Gly Glu Trp Tyr Leu Ile Gly Met Pro Gly Lys Lys Asn Thr Ile
225                 230                 235                 240
Thr Tyr Gln Cys Cys Pro Glu Pro Tyr Val Asp Val Thr Phe Thr Ile
                245                 250                 255
Gln Ile Arg Arg Arg Thr Leu Tyr Tyr Phe Phe Asn Leu Ile Val Pro
            260                 265                 270
Cys Val Leu Ile Ser Ser Met Ala Leu Leu Gly Phe Thr Leu Pro Pro
        275                 280                 285
Asp Ser Gly Glu Lys Leu Thr Leu Gly Val Thr Ile Leu Leu Ser Leu
    290                 295                 300
Thr Val Phe Leu Asn Leu Val Ala Glu Ser Met Pro Thr Thr Ser Asp
305                 310                 315                 320
Ala Val Pro Leu Ile Gly Ser Tyr Phe Asn Cys Ile Met Phe Met Val
                325                 330                 335
Ala Ser Ser Val Val Leu Thr Val Leu Val Leu Asn Phe His His Arg
            340                 345                 350
Thr Pro Asp Arg Tyr Val Met Pro Ser Trp Ile Lys Met Leu Phe Leu
        355                 360                 365
Gln Trp Leu Pro Cys Leu Leu Arg Met Ser Arg Pro Gly Lys Lys Ile
    370                 375                 380
Thr Lys Lys Thr Ile Leu Gly Gly Asn Arg Arg Ala Lys Gly Met Glu
385                 390                 395                 400
Leu Gln Glu Lys Ser Ser Lys Ser Leu Leu Ala Asn Val Leu Asp Ile
                405                 410                 415
Asp Asp Asp Ile Arg His Lys Asn Ser Ala Ala Asn Pro Pro Ser Gly
            420                 425                 430
Tyr Ile Arg Ser Ala Phe Gly Thr Pro Ile Ser Thr Gly Arg Pro Ala
        435                 440                 445
Thr Val Glu Asp Thr Ser Ala Ser Leu Pro Leu Ser Gly Met Gln Glu
    450                 455                 460
Glu Leu His Thr Ile Leu Lys Glu Leu Arg Phe Ile Thr Asp Arg Met
465                 470                 475                 480
Lys Lys Ala Asp Glu Ser Asp Glu Ile Ile Ser Asp Trp Lys Phe Ala
                485                 490                 495
Ala Met Val Val Asp Arg Leu Cys Leu Ile Val Phe Thr Leu Phe Thr
            500                 505                 510
Val Leu Ala Thr Ile Val Ile Leu Cys Arg Ala Pro His Ile Ile Val
        515                 520                 525
Gln
```

<210> 22
<211> 555
<212> PRT
<213> Apis mellifera

<220>
<223> nicotinic acetylcholine receptor alpha7 subunit amino acid sequence

<400> 22

```
Met Arg Arg Trp Thr Leu Met Ala Ala Ile Ala Leu Ala Ala Ser Gly
1               5                   10                  15
Leu Val Asn Gly Gly Ser His Glu Lys Arg Leu Leu Asn Asp Leu Leu
            20                  25                  30
Asp Thr Tyr Asn Val Leu Glu Arg Pro Val Gly Asn Glu Ser Glu Pro
        35                  40                  45
Leu Val Leu Ser Phe Gly Leu Thr Leu Met Gln Ile Ile Asp Val Asp
    50                  55                  60
Glu Lys Asn Gln Leu Leu Ile Thr Asn Leu Trp Leu Lys Leu Glu Trp
65                  70                  75                  80
Asn Asp Val Asn Met Arg Trp Asn Val Ser Asp Tyr Gly Gly Val Arg
                85                  90                  95
Asp Leu Arg Ile Pro Pro His Arg Leu Trp Lys Pro Asp Val Leu Met
            100                 105                 110
Tyr Asn Ser Ala Asp Glu Gly Phe Asp Gly Thr Tyr Pro Thr Asn Val
        115                 120                 125
Val Val Lys Asn Asn Gly Thr Cys Leu Tyr Val Pro Pro Gly Ile Phe
    130                 135                 140
Lys Ser Thr Cys Lys Ile Asp Ile Thr Trp Phe Pro Phe Asp Asp Gln
145                 150                 155                 160
Arg Cys Glu Met Lys Phe Gly Ser Trp Thr Tyr Asp Gly Phe Gln Leu
                165                 170                 175
Asp Leu Gln Leu Gln Asp Glu Ala Gly Gly Asp Ile Ser Ser Phe Ile
            180                 185                 190
Thr Asn Gly Glu Trp Asp Leu Leu Gly Val Pro Gly Lys Arg Asn Glu
        195                 200                 205
Ile Tyr Tyr Asn Cys Cys Pro Glu Pro Tyr Ile Asp Ile Thr Phe Val
    210                 215                 220
Val Ile Ile Arg Arg Arg Thr Leu Tyr Tyr Phe Phe Asn Leu Ile Val
225                 230                 235                 240
Pro Cys Val Leu Ile Ala Ser Met Ala Val Leu Gly Phe Thr Leu Pro
                245                 250                 255
Pro Asp Ser Gly Glu Lys Leu Ser Leu Gly Val Thr Ile Leu Leu Ser
            260                 265                 270
Leu Thr Val Phe Leu Asn Met Val Ala Glu Thr Met Pro Ala Thr Ser
        275                 280                 285
Asp Ala Val Pro Leu Leu Gly Thr Tyr Phe Asn Cys Ile Met Phe Met
    290                 295                 300
Val Ala Ser Ser Val Val Ser Thr Ile Leu Ile Leu Asn Tyr His His
305                 310                 315                 320
Arg Asn Ala Asp Thr His Glu Met Ser Glu Trp Val Lys Val Val Phe
                325                 330                 335
Leu Tyr Trp Leu Pro Cys Ile Leu Arg Met Ser Arg Pro Ser Asp Lys
            340                 345                 350
Glu Glu Arg Glu Ala Gln Lys Ser Gln Lys Pro Ser Pro Val Thr Gly
        355                 360                 365
Ala Ser Lys Ser His Gly Asp Leu Glu Leu Arg Gln Arg Ser Ser Lys
    370                 375                 380
Ser Leu Leu Ala Asn Val Leu Asp Leu Glu Asp Asn Ala Leu Ala Ser
385                 390                 395                 400
His Asn Asn Leu Leu Asn Asn Val Tyr Ser Thr Pro Gly Pro His His
                405                 410                 415
His Thr Met Gly His Gly His Ser His Ile His Ala Thr Pro His His
            420                 425                 430
His His Ser His Ala Ala Thr Pro His His Gln His Ser Thr Pro Leu
        435                 440                 445
Ala His Ser Ser Tyr Pro Ala Ala Ile Gln Ile Gly His Thr Pro His
    450                 455                 460
His His Pro His Pro Pro Glu Thr Pro Gly Pro Gln Val Glu Thr Ile
465                 470                 475                 480
Leu Gln Asn Ala Cys Phe Cys Ala Arg Asn Glu Leu Met Met Ile Leu
                485                 490                 495
```

```
Lys Glu Ile Lys Ile Ile Thr Asp Gln Leu Lys Ser Glu Glu Leu Asn
            500                 505                 510
Thr Lys Val Thr Asn Asp Trp Lys Phe Ala Ala Met Val Ile Asp Arg
        515                 520                 525
Met Cys Leu Ile Ile Phe Thr Leu Phe Thr Ile Ile Ala Thr Ile Ala
    530                 535                 540
Val Leu Leu Ser Ala Pro His Ile Ile Val Thr
545                 550                 555

<210> 23
<211> 537
<212> PRT
<213> Apis mellifera

<220>
<223> nicotinic acetylcholine receptor alpha8 subunit amino acid
      sequence

<400> 23
Met Phe Lys Met Gln Ile Leu Thr Leu Gly Val Leu Phe Asn Thr Leu
1               5                   10                  15
His Ile Ile Tyr Ser Val Ala Gly Leu Lys Ile Phe Glu Ala Asn Pro
            20                  25                  30
Asp Thr Lys Arg Leu Tyr Asp Asp Leu Leu Ser Asn Tyr Asn Arg Leu
        35                  40                  45
Ile Arg Pro Val Met Asn Asn Thr Glu Thr Leu Thr Val Gln Leu Gly
    50                  55                  60
Leu Lys Leu Ser Gln Leu Ile Glu Met Asn Leu Lys Asn Gln Val Met
65                  70                  75                  80
Thr Thr Asn Val Trp Val Glu Gln Arg Trp Asn Asp Tyr Lys Leu Lys
                85                  90                  95
Trp Asn Pro Glu Glu Tyr Gly Gly Val Glu Met Leu Tyr Val Pro Ser
            100                 105                 110
Glu Asn Ile Trp Leu Pro Asp Ile Val Leu Tyr Asn Asn Ala Asp Gly
        115                 120                 125
Asn Tyr Glu Val Thr Leu Met Thr Lys Ala Thr Leu Lys Tyr Thr Gly
    130                 135                 140
Asp Val Ser Trp Lys Pro Pro Ala Ile Tyr Lys Ser Ser Cys Glu Ile
145                 150                 155                 160
Asn Val Glu Tyr Phe Pro Phe Asp Glu Gln Ser Cys Ile Met Lys Phe
                165                 170                 175
Gly Ser Trp Thr Tyr Asn Gly Ala Gln Val Asp Leu Lys His Met Lys
            180                 185                 190
Gln Glu Ala Gly Ser Asn Leu Val Ala Lys Gly Ile Asp Leu Ser Asp
        195                 200                 205
Phe Tyr Leu Ser Val Glu Trp Asp Ile Leu Glu Val Pro Ala Ser Arg
    210                 215                 220
Asn Glu Glu Tyr Tyr Pro Cys Cys Thr Glu Pro Tyr Ser Asp Ile Thr
225                 230                 235                 240
Phe Asn Ile Thr Met Arg Arg Lys Thr Leu Phe Tyr Thr Val Asn Leu
                245                 250                 255
Ile Ile Pro Cys Val Gly Ile Thr Phe Leu Thr Val Leu Val Phe Tyr
            260                 265                 270
Leu Pro Ser Asp Ser Gly Glu Lys Val Ser Leu Cys Ser Ser Ile Leu
        275                 280                 285
Leu Ser Leu Thr Val Phe Phe Leu Leu Leu Ala Glu Ile Ile Pro Pro
    290                 295                 300
Thr Ser Leu Ala Ile Pro Leu Leu Gly Lys Tyr Leu Leu Phe Thr Met
305                 310                 315                 320
Ile Leu Val Thr Leu Ser Ile Trp Ile Thr Val Cys Val Leu Asn Val
                325                 330                 335
His Phe Arg Ser Pro Ser Thr His Asn Met Ser Pro Trp Val Arg Gln
            340                 345                 350
```

Val Phe Leu Asn Trp Met Pro Arg Leu Leu Met Met Arg Arg Thr Pro
355 360 365
Tyr Ser Thr Pro Glu Tyr Asp Asp Thr Tyr Met Asp Ser Gly Tyr Thr
370 375 380
Asn Glu Ile Asp Phe Ser Phe Pro Asp Ser Val Ser Asp Tyr Pro Leu
385 390 395 400
Glu Leu Lys Gly Ser Pro Asp Gly Phe Glu Ser Val Thr Ser Gln Tyr
405 410 415
Lys Asn Ile Arg Glu Asp Asp Ala Arg His Ile Pro His Ala Ser Val
420 425 430
Thr Asp Ser Glu Asn Thr Val Pro Arg Tyr Leu Ser Pro Asp Val Ile
435 440 445
Ser Ala Leu Lys Gly Val Arg Phe Ile Ala Gln His Ile Lys Asn Ala
450 455 460
Asp Lys Asp Asn Glu Val Ile Glu Asp Trp Lys Phe Val Ala Met Val
465 470 475 480
Leu Asp Arg Leu Phe Leu Trp Val Phe Thr Leu Ala Cys Thr Ala Gly
485 490 495
Thr Leu Gly Ile Ile Phe Gln Ala Pro Ser Leu Tyr Asp Thr Arg Glu
500 505 510
Pro Val Asp Gln Gln Leu Ser Gly Ile Ser Pro Arg Asn Tyr Met Tyr
515 520 525
Pro Asn Ile Asp Ile Ser Pro Glu Gly
530 535

<210> 24
<211> 431
<212> PRT
<213> Apis mellifera

<220>
<223> nicotinic acetylcholine receptor alpha9 subunit amino acid sequence

<400> 24
Met Lys Met Arg Ile Ile Thr Ala Leu Gly Phe Leu Leu Phe Trp Gln
1 5 10 15
Asn Ser Ala Ser Leu Glu Asn Asp Asn Glu Phe Gly Phe Ser Tyr Leu
20 25 30
Leu Asn Cys Lys Asn Tyr Asp His Pro Thr Thr Leu Leu Lys Leu Lys
35 40 45
Arg Tyr Leu Phe Cys Glu Tyr Asp Pro Asn Val Arg Pro Ile Ser Ser
50 55 60
His Gln Ile Ala Asn Asn Val Thr Met Gln Leu Leu Pro Lys Leu Met
65 70 75 80
Glu Phe Asp Asp Trp Thr Ser Val Met Glu Leu His Ser Trp Met Thr
85 90 95
Leu Met Trp Thr Asp Ser His Leu Ser Trp Lys Pro Ser Asp Phe Asp
100 105 110
Gly Ile Asn Tyr Ile Tyr Val Lys Ser Asp Asp Ile Trp Val Pro Asp
115 120 125
Ile Ser Val Tyr Asn Ser Gly Asp Met Thr Phe Asp Gln Thr Gly Ile
130 135 140
Pro Pro Thr Thr Cys Leu Val Phe Ser Ser Gly Ser Val Ser Cys Val
145 150 155 160
Pro Ser Val Lys His Val Ala Lys Cys Ala Thr Asp Phe Ser Ser Trp
165 170 175
Pro Tyr Asp Thr His Arg Cys Arg Ile Asn Phe Gly Ser Trp Val His
180 185 190
Ser Gly Glu Glu Val Asn Ile Phe Leu Asp Lys Lys Gly Phe His Met
195 200 205
Asp Gly Tyr Thr Asn Asn Ser Lys Trp Asp Phe Lys Val Ile Lys Ala
210 215 220

```
Thr Lys Val Leu Lys Met Tyr Ala Cys Cys Pro Asn Asp Thr Tyr Pro
225                 230                 235                 240
Met Ile Val Tyr Glu Phe Ser Ile Ser Arg His Tyr Gly Ile Leu His
                245                 250                 255
Ala Thr Tyr Val Ile Pro Ala Val Thr Met Met Leu Leu Thr Leu Thr
            260                 265                 270
Val Leu Trp Leu Asp Ser Arg Ser Thr Glu Arg Met Ile Ala Ala Ser
        275                 280                 285
Val Asn Leu Ile Cys His Ile Leu Cys Met Ser Asp Leu His Trp Gln
    290                 295                 300
Leu Pro His Asn Ser Thr Asn Pro Pro Asn Ile Leu Leu Tyr Tyr Arg
305                 310                 315                 320
Asp Ser Leu Ala Leu Ser Val Phe Ala Leu Ile Leu Thr Ala Leu Leu
                325                 330                 335
Arg Lys Met Gln Glu Met Ser Ile Glu Val Pro Tyr Trp Ile Ser Thr
            340                 345                 350
Thr Thr Ser Phe Val Leu Asn Asn Arg Ala Gly Arg Phe Leu Ile Leu
        355                 360                 365
Thr Glu Ser Asp Thr Lys Leu Ser Ser Gln Gly Ile Leu Gly Glu Asp
    370                 375                 380
Val Glu Asn Asn Ser Glu Val Ser Lys Ser Arg Thr Lys Glu Ser Ala
385                 390                 395                 400
Trp Arg His Phe Ala Ala Ile Ile Glu Trp Leu Ser Phe Phe Ile Val
                405                 410                 415
Ile Phe Thr Tyr Ile Ile Ile Leu Ile Thr Leu Val Pro Ser Thr
            420                 425                 430
```

```
<210> 25
<211> 520
<212> PRT
<213> Apis mellifera

<220>
<223> nicotinic acetylcholine receptor beta1 subunit amino acid
      sequence

<400> 25
Met His Asn Ile Cys Ser Arg Leu Gly Arg Ile Leu Leu Ile Ser Ala
1               5                   10                  15
Val Phe Cys Val Gly Leu Cys Ser Glu Asp Glu Glu Arg Leu Val Arg
            20                  25                  30
Asp Leu Phe Arg Gly Tyr Asn Lys Leu Ile Arg Pro Val Gln Asn Met
        35                  40                  45
Thr Glu Lys Val His Val Asn Phe Gly Leu Ala Phe Val Gln Leu Ile
    50                  55                  60
Asn Val Asn Glu Lys Asn Gln Ile Met Lys Ser Asn Val Trp Leu Arg
65                  70                  75                  80
Phe Ile Trp Thr Asp Tyr Gln Leu Gln Trp Asp Glu Ala Asp Tyr Gly
                85                  90                  95
Gly Ile Gly Val Leu Arg Leu Pro Pro Asp Lys Val Trp Lys Pro Asp
            100                 105                 110
Ile Val Leu Phe Asn Asn Ala Asp Gly Asn Tyr Glu Val Arg Tyr Lys
        115                 120                 125
Ser Asn Val Leu Ile Tyr Pro Asn Gly Asp Val Leu Trp Val Pro Pro
    130                 135                 140
Ala Ile Tyr Gln Ser Ser Cys Thr Ile Asp Val Thr Tyr Phe Pro Phe
145                 150                 155                 160
Asp Gln Gln Thr Cys Ile Met Lys Phe Gly Ser Trp Thr Phe Asn Gly
                165                 170                 175
Asp Gln Val Ser Leu Ala Leu Tyr Asn Asn Lys Asn Phe Val Asp Leu
            180                 185                 190
Ser Asp Tyr Trp Lys Ser Gly Thr Trp Asp Ile Ile Asn Val Pro Ala
        195                 200                 205
```

```
Tyr Leu Asn Thr Tyr Lys Gly Asp Phe Pro Thr Glu Thr Asp Ile Thr
    210                 215                 220
Phe Tyr Ile Ile Ile Arg Arg Lys Thr Leu Phe Tyr Thr Val Asn Leu
225                 230                 235                 240
Ile Leu Pro Thr Val Leu Ile Ser Phe Leu Cys Val Leu Val Phe Tyr
                245                 250                 255
Leu Pro Ala Glu Ala Gly Glu Lys Val Thr Leu Gly Ile Ser Ile Leu
            260                 265                 270
Leu Ser Leu Val Val Phe Leu Leu Leu Val Ser Lys Ile Leu Pro Pro
        275                 280                 285
Thr Ser Leu Val Leu Pro Leu Ile Ala Lys Tyr Leu Leu Phe Thr Phe
    290                 295                 300
Ile Met Asn Thr Val Ser Ile Leu Val Thr Val Ile Ile Ile Asn Trp
305                 310                 315                 320
Asn Phe Arg Gly Pro Arg Thr His Arg Met Pro Gln Leu Ile Arg Lys
                325                 330                 335
Ile Phe Leu Lys Tyr Leu Pro Thr Ile Leu Met Met Arg Arg Pro Lys
            340                 345                 350
Lys Thr Arg Leu Arg Trp Met Met Glu Ile Pro Asn Val Thr Leu Pro
        355                 360                 365
Thr Ser Thr Tyr Ser Gly Ser Pro Thr Glu Leu Pro Lys His Leu Pro
    370                 375                 380
Thr Ser Leu Thr Lys Ser Lys Met Glu Val Met Glu Leu Ser Asp Leu
385                 390                 395                 400
His His Pro Asn Cys Lys Ile Asn Arg Lys Val His His Thr Thr Ser
                405                 410                 415
Ser Ser Ser Ala Ala Gly Gly Glu Gly Leu Ala Asp Arg Arg Gly Ser
            420                 425                 430
Glu Ser Ser Asp Ser Val Leu Leu Ser Pro Glu Ala Ser Lys Ala Thr
        435                 440                 445
Glu Ala Val Glu Phe Ile Ala Glu His Leu Arg Asn Glu Asp Leu Tyr
    450                 455                 460
Ile Gln Thr Arg Glu Asp Trp Lys Tyr Val Ala Met Val Ile Asp Arg
465                 470                 475                 480
Leu Gln Leu Tyr Ile Phe Phe Leu Val Thr Thr Ala Gly Thr Ile Gly
                485                 490                 495
Ile Leu Met Asp Ala Pro His Ile Phe Glu Tyr Val Asp Gln Asp His
            500                 505                 510
Ile Ile Glu Ile Tyr Arg Gly Lys
        515                 520
```

<210> 26
<211> 427
<212> PRT
<213> Apis mellifera

<220>
<223> nicotinic acetylcholine receptor beta2 subunit amino acid sequence

<400> 26

```
Met Leu Asn Met Lys Asn Ile Phe Pro Val Leu Phe Val Ile Ile Asn
1               5                   10                  15
Val Leu Leu His Gly Gln Val Ile Cys Phe Val Cys Lys Asp Ile Thr
            20                  25                  30
Ser Thr Ser Ala Leu Tyr Arg Leu Lys Leu Tyr Leu Phe Cys Asp Tyr
        35                  40                  45
Asp Arg Asp Ile Ile Pro Glu Gln Lys Asn Ala Thr Lys Ile Asp Phe
    50                  55                  60
Gly Leu Ser Ile Gln His Tyr Asn Val Asp Glu Tyr Ser His Thr Val
65                  70                  75                  80
Asp Phe His Val Met Leu Lys Leu Met Trp Glu Gln Ser His Leu Thr
                85                  90                  95
```

```
Trp Lys Ser Ser Glu Phe Asp Ser Ile Asn Ser Ile Arg Val Lys Ser
            100                 105                 110
Tyr Glu Ile Trp Val Pro Asp Ile Val Met His Ser Val Thr Ser Val
        115                 120                 125
Gly Ile Asp Leu Glu Met Pro Ser Val Glu Cys Ile Val Phe Asn Ser
    130                 135                 140
Gly Thr Ile Leu Cys Val Pro Phe Thr Thr Tyr Thr Pro Val Cys Glu
145                 150                 155                 160
Tyr Asp His Thr Trp Trp Pro Tyr Asp Ile Leu Asn Cys Thr Ile His
                165                 170                 175
Ile Ala Ser Trp Ser His Gly Ser Asn Glu Ile Lys Leu Asn Ser Leu
            180                 185                 190
Asp Thr Glu Gln Ile Leu Asp Asp Met Tyr Asn Asn Asn Thr Glu Trp
        195                 200                 205
Glu Ile Val His Met Ser His Ser Glu Ser Thr Ile Asp Ser Lys Phe
    210                 215                 220
Gly Leu Gly Phe Thr Thr Asp Leu Leu Ser Tyr Asn Ile Leu Leu Arg
225                 230                 235                 240
Arg His Tyr Ser Met Asn Ser Thr Thr Tyr Val Thr Leu Thr Ile Val
                245                 250                 255
Leu Met Thr Met Thr Leu Met Thr Leu Trp Leu Glu Pro Ser Ser Thr
            260                 265                 270
Glu Arg Met Ile Ile Ala Asn Leu Asn Phe Ile Leu His Leu Phe Cys
        275                 280                 285
Leu Leu Asp Val Gln Trp Arg Ile Pro Phe Asn Gly Ile Gln Met Pro
    290                 295                 300
Asn Leu Met Val Phe Tyr Glu Lys Ser Leu Ala Leu Ala Ala Phe Ser
305                 310                 315                 320
Leu Met Leu Thr Ser Ile Leu Arg Tyr Leu Gln Glu Leu His Val Asp
                325                 330                 335
Ala Pro Thr Trp Ile Ser Ser Val Thr Glu Ser Val Leu Lys Ser Lys
            340                 345                 350
Ile Gly Gln Val Phe Leu Ile Thr Ile Leu Asp Ser Lys Val Ser Ala
        355                 360                 365
Arg Ile Glu Met Asn Glu Asp Asp Asn Thr Ser Leu Val Ser Leu Asp
    370                 375                 380
Lys Lys Gln Tyr Thr Trp Arg His Thr Ser Val Leu Ile Gly Trp Ser
385                 390                 395                 400
Ala Phe Leu Cys Ile Ser Phe Val Tyr Ile Ile Met Leu Ile Ile Phe
                405                 410                 415
Ile Pro Arg Asn Ile Tyr Glu Asn Phe Ile Ser
            420                 425
```

<210> 27
<211> 454
<212> PRT
<213> Apis mellifera

<220>
<223> chaperone protein resistance to inhibitors of cholinesterase 3 (ric-3) amino acid sequence

<400> 27

```
Met Ala Glu Ile Thr Asp Phe Gly Pro Arg Lys Thr Ile Phe Ile Leu
1               5                   10                  15
Ala Ile Val Ala Gly Cys Phe Ala Val Leu Trp Pro Lys Ile Phe Tyr
            20                  25                  30
Pro Met Leu Thr Ala Ser Val Asn Pro His His Ile Thr Asp Asn Ser
        35                  40                  45
Ala Cys Cys Gly Val Ile Phe Glu Ser Asp Val Thr Ala Ala Asp Ile
    50                  55                  60
Met Tyr Glu Ile Cys Gln Asn Ile Leu Lys His His Gln Ile Asp Pro
65                  70                  75                  80
```

Arg Ile Arg Asp Ala Leu Arg Thr Ile Lys Leu Thr Pro Gln Ser Ala
85 90 95
Ser Leu Cys Arg Glu Glu Ile Leu Ala Arg Cys Gly Ile Asp Leu Ser
100 105 110
Thr Phe Leu Ala Lys Arg Glu His Leu Glu Lys Ser Tyr Lys Gln Val
115 120 125
Leu Glu Glu Ile Arg Ser Phe Asn Ser Ser Leu Cys Leu Lys Ile Asn
130 135 140
Phe Gly Ile Pro Leu Ser Gln Leu Gly Thr Pro His Leu Ile Arg Tyr
145 150 155 160
His Ile Leu Met Pro His Asn Thr Ile Lys Gln Glu Arg Arg Thr Pro
165 170 175
Pro His Ala Gly Gly Leu His Pro Ala Leu Arg Glu Arg Gly Arg Ala
180 185 190
Ile Pro Ser Ser His Ile Val Pro Lys Val Ser Asp Arg Pro Asp His
195 200 205
Val Val Pro Lys Met Arg Pro Pro Leu Gly Gly Ala Gly His Val Val
210 215 220
Pro Ala Pro Lys Gly Ser Gly Thr Met Gly Ile Ile Met Pro Leu Tyr
225 230 235 240
Thr Leu Gly Ile Val Leu Phe Phe Leu Tyr Thr Ile Val Lys Val Leu
245 250 255
Arg Lys Asn Ser Asp Ser Glu Ile Ile Ser Glu Tyr Pro Gly Ala Ala
260 265 270
Ala Glu Lys Glu Phe Arg Lys Met Val Phe Ser Pro Glu Ala Phe Ala
275 280 285
Thr Ala Met Thr Gly Gly Thr Met Asn Tyr Gln Lys Glu Arg Ser Pro
290 295 300
Ser Pro Gln Arg Pro Thr Pro Thr Leu Glu Glu Leu Lys Asp Leu Asp
305 310 315 320
Gly Pro Pro Lys Ser Lys Gly Leu Ala Gly Ile Asp Arg Ser Ser Lys
325 330 335
Asp Asn Thr Ile Ala Gln Val Gln Tyr Ser Thr Glu Asp Lys Val Glu
340 345 350
Asn Ile Glu His Ser Pro Thr Ile Lys Val Met Gly Met Glu Met Thr
355 360 365
Ala Ser Cys Glu Asn Lys Gly Ser Arg Pro Thr Thr Pro Ile Ile Pro
370 375 380
Ile Ser Pro Ser His Ile Glu Arg Glu Lys Thr Pro Pro Lys Pro Ile
385 390 395 400
Tyr Leu Glu Gly Ala Leu Pro Pro Gln Cys Glu Leu Leu Val Thr Asp
405 410 415
Ser Glu Thr Gln Ala Gln Lys Ala Glu Glu Asp Val Glu Ala Pro Val
420 425 430
Val Leu Ser Gly Lys Met Thr Leu Ser Leu Ile Ser Leu Asp Gln Asn
435 440 445
Ala Ala Val Arg Lys Tyr
450

<210> 28
<211> 113
<212> PRT
<213> Apis mellifera

<220>
<223> chaperone protein emc-6 amino acid sequence

<400> 28
Met Leu Gly Lys Ile Lys Thr Lys Gln Glu Lys Gly Glu Ile Ile Ala
1 5 10 15
Tyr Ser Glu Ser Ala Val Val Asn Asn Ala Ala Val Val Glu Tyr Cys
20 25 30
Arg Ile Ser Met Ala Ala Leu Ser Gly Gly Thr Ala Gly Leu Leu Gly

```
        35                  40                  45
Leu Thr Gly Leu Tyr Gly Phe Gly Phe Tyr Ile Phe Ala Val Phe Gly
    50                  55                  60
Leu Trp Ala Met Leu Leu Leu Lys Ala Gly Gly Gln Trp Lys Lys Tyr
65                  70                  75                  80
Phe Ile Ser Arg Arg Asn Leu Leu Thr Ser Gly Phe Phe Gly Gly Leu
                85                  90                  95
Phe Thr Tyr Val Leu Phe Trp Thr Phe Leu Tyr Gly Met Val His Val
            100                 105                 110
Tyr


<210> 29
<211> 267
<212> PRT
<213> Apis mellifera

<220>
<223> chaperone protein unc-50 amino acid sequence

<400> 29
Met Lys Tyr Ser Thr Ser Pro Pro Val Ser Arg Cys Asn Ser Pro Gly
1               5                   10                  15
Pro Ser Glu Thr Ser Ser Asn Leu Pro Met Pro Val Thr Tyr Arg Gln
            20                  25                  30
Asp Cys Met Gly Ala Ala Thr Lys Cys Tyr Lys Tyr Leu Arg Lys Leu
        35                  40                  45
Leu Lys Phe Glu Gln Met Asp Phe Glu Phe Ala Leu Trp Gln Met Ile
    50                  55                  60
Phe Leu Phe Ile Ser Pro Gln Lys Val Tyr Arg Asn Phe Gln Asn Arg
65                  70                  75                  80
Lys Gln Thr Lys Ser Gln Phe Ala Arg Asp Asp Pro Ala Phe Leu Val
                85                  90                  95
Leu Leu Met Cys Cys Leu Cys Ile Ser Ser Val Gly Phe Thr Ile Val
            100                 105                 110
Leu Gly Leu Gly Phe Leu Gln Phe Ile Lys Leu Leu Phe Tyr Met Ile
        115                 120                 125
Phe Ile Asp Tyr Leu Ala Ala Gly Leu Ile Val Ala Thr Ile Phe Trp
    130                 135                 140
Phe Ile Thr Asn His Tyr Leu Arg Ile Asp Lys Thr Gln Asp Val Glu
145                 150                 155                 160
Trp Gly Tyr Ala Phe Asp Ile His Leu Asn Ala Phe Phe Pro Pro Leu
                165                 170                 175
Ile Ile Leu His Ile Val Gln Leu Phe Leu Tyr Lys Ser Phe Ile Asn
            180                 185                 190
Tyr Asp Thr Phe Ser Ser Arg Phe Val Gly Asn Thr Ile Trp Leu Ile
        195                 200                 205
Ala Val Ser Tyr Tyr Ile Tyr Ile Thr Phe Leu Gly Tyr Thr Ser Met
    210                 215                 220
Glu Ile Val His Lys Thr His Met Ile Leu Ser Thr Leu Pro Ile Ile
225                 230                 235                 240
Leu Leu Ile Tyr Ile Met Thr Leu Cys Ala Gly Ile Asn Ile Ser His
                245                 250                 255
Leu Val Met Glu Phe Tyr His Tyr Arg Val Val
            260                 265

<210> 30
<211> 431
<212> PRT
<213> Apis mellifera

<220>
<223> chaperone protein unc-74 amino acid sequence
```

<400> 30
Met Val Ile Ile Thr Lys Leu Met Phe Ile Ala Val Ile Tyr Val Phe
1 5 10 15
Ser Gly Thr Phe Thr Ser Val Ile Ala Ser Arg Val Leu Glu Leu Ser
20 25 30
Asp Arg Phe Leu Asp Ile His Lys Asp Gly Gln Trp Leu Val Met Met
35 40 45
Tyr Ala Pro Trp Cys Ala His Cys Lys Arg Leu Glu Pro Ile Trp Ala
50 55 60
His Val Ala Gln Tyr Leu His Ala Thr Ser Ile Arg Val Gly Arg Val
65 70 75 80
Asp Cys Thr Arg Phe Thr Asn Val Ala His Ala Phe Lys Val Lys Gly
85 90 95
Phe Pro Thr Ile Ile Phe Leu Lys Gly Glu Gln Glu Phe Ile Tyr Asn
100 105 110
Gly Asp Arg Thr Arg Asp Glu Ile Val Lys Phe Ala Leu Arg Val Ser
115 120 125
Gly Pro Pro Val Gln Gly Ile Thr Lys Thr Gln Ser Phe Asp Thr Ile
130 135 140
Lys Lys Glu His Asp Ile Tyr Phe Leu Tyr Val Gly Glu Arg Ser Gly
145 150 155 160
Pro Leu Trp Glu Phe Tyr His Lys Thr Ala Asn Val Phe Gln Pro His
165 170 175
Ala Phe Phe Tyr Gln Ser His Pro Asn Ile Val Ser Lys His Ala Pro
180 185 190
Val Glu Asn Thr Pro Ala Leu Phe Val Tyr Lys Glu Asn Ile His Tyr
195 200 205
Asn Phe Ser Asp His Asn Ile Asp Asp Ile Glu Lys Leu Asn Glu Thr
210 215 220
Met Tyr Lys Trp Ile Asn Ala Glu Arg Phe Pro Thr Phe Pro Lys Val
225 230 235 240
Thr Arg Gly Asn Ile Asn Gln Leu Phe Leu Thr Asn Lys Asn Leu Val
245 250 255
Leu Ala Val Val Glu Glu Asn Gln Leu Glu Lys Ile Pro Leu His Met
260 265 270
Ala Arg Phe Lys Asp Met Val Glu Ser Ile Ile Lys Asn Lys Arg Glu
275 280 285
Lys Tyr His Asp Tyr Phe Gln Phe Gly Trp Ile Ala Ser Pro Asp Leu
290 295 300
Val Asn Ser Ile Ala Met Met Val Leu Pro Leu Pro Ser Leu Ile Val
305 310 315 320
Ile Asn Thr Thr Thr Asn His His His Ile Pro Glu Asp Glu Thr Glu
325 330 335
Lys Leu Thr Pro His Val Ile Glu Leu Phe Leu Glu Gln Ile Arg Asn
340 345 350
Glu Ser Ala Pro Arg Tyr Gly Gly Asn Ser Trp Leu Ile Arg Ile Tyr
355 360 365
Arg Ser Trp Phe Glu Leu Lys Thr Thr Leu Ser Ala Met Trp Met Gly
370 375 380
Asn Pro Ile Leu Thr Met Val Leu Phe Gly Leu Pro Ala Gly Phe Leu
385 390 395 400
Ser Leu Ile Cys Tyr Gly Ile Cys Cys Pro Asp Ile Leu Asp Ala Asp
405 410 415
Asp Asp Glu Glu Asp His Pro Gly Ala Asn His Met Lys Lys Asp
420 425 430

<210> 31
<211> 23
<212> DNA
<213> Artificial Sequence

<220>

<221> source
<222> 1..23
<223> /mol_type="unassigned DNA"
/note="amnachra1-001S"
/organism="Artificial Sequence"

<400> 31
catggcgacg gccatttcct gtc 23

<210> 32
<211> 22
<212> DNA
<213> Artificial Sequence

<220>
<221> source
<222> 1..22
<223> /mol_type="unassigned DNA"
/note="amnachra1-002AS "
/organism="Artificial Sequence"

<400> 32
ctagtcctcc tcgggaccca tg 22

<210> 33
<211> 29
<212> DNA
<213> Artificial Sequence

<220>
<221> source
<222> 1..29
<223> /mol_type="unassigned DNA"
/note="amnachra2-001S "
/organism="Artificial Sequence"

<400> 33
catgatactc cagacgatca tcttgatcc 29

<210> 34
<211> 28
<212> DNA
<213> Artificial Sequence

<220>
<221> source
<222> 1..28
<223> /mol_type="unassigned DNA"
/note="amnachra2-002AS "
/organism="Artificial Sequence"

<400> 34
ctacacgaga gtttccaaaa agtcatcg 28

<210> 35
<211> 25
<212> DNA
<213> Artificial Sequence

<220>
<221> source
<222> 1..25
<223> /mol_type="unassigned DNA"
/note="amnachra3-001S "
/organism="Artificial Sequence"

<400> 35
catgatgaag agcctggtgg ggatc 25

<210> 36
<211> 24
<212> DNA
<213> Artificial Sequence

<220>
<221> source
<222> 1..24
<223> /mol_type="unassigned DNA"
/note="amnachra3-002AS "
/organism="Artificial Sequence"

<400> 36
ttagaggctc gtaacgatgt gtgg 24

<210> 37
<211> 24
<212> DNA
<213> Artificial Sequence

<220>
<221> source
<222> 1..24
<223> /mol_type="unassigned DNA"
/note="amnachra4-001S "
/organism="Artificial Sequence"

<400> 37
catgcccccc ataatagggg aaac 24

<210> 38
<211> 24
<212> DNA
<213> Artificial Sequence

<220>
<221> source
<222> 1..24
<223> /mol_type="unassigned DNA"
/note="amnachra4-002AS "
/organism="Artificial Sequence"

<400> 38
ctattgtggc ggacagttta ccac 24

<210> 39
<211> 22
<212> DNA
<213> Artificial Sequence

<220>
<221> source
<222> 1..22
<223> /mol_type="unassigned DNA"
/note="amnachra5-003S "
/organism="Artificial Sequence"

<400> 39
catgtcgcct ttggtcctgt tc 22

<210> 40
<211> 26
<212> DNA
<213> Artificial Sequence

<220>
<221> source
<222> 1..26
<223> /mol_type="unassigned DNA"
/note="amnachra5-002AS "
/organism="Artificial Sequence"

<400> 40
ctcttaaccc tctttggcaa tgttcg 26

<210> 41
<211> 27
<212> DNA
<213> Artificial Sequence

<220>
<221> source
<222> 1..27
<223> /mol_type="unassigned DNA"
/note="amnachra6-001S "
/organism="Artificial Sequence"

<400> 41
catgcgcgca agtagtgtat tacaagc 27

<210> 42
<211> 25
<212> DNA
<213> Artificial Sequence

<220>
<221> source
<222> 1..25
<223> /mol_type="unassigned DNA"
/note="amnachra6-002AS "
/organism="Artificial Sequence"

<400> 42
cttattggac gattatgtgt ggcgc 25

<210> 43
<211> 25
<212> DNA

<213> Artificial Sequence

<220>
<221> source
<222> 1..25
<223> /mol_type="unassigned DNA"
/note="amnachra7-001S "
/organism="Artificial Sequence"

<400> 43
catgagacgt tggactctca tggcg 25

<210> 44
<211> 25
<212> DNA
<213> Artificial Sequence

<220>
<221> source
<222> 1..25
<223> /mol_type="unassigned DNA"
/note="amnachra7-002AS "
/organism="Artificial Sequence"

<400> 44
gaatcacgtg acgatgatgt gtggc 25

<210> 45
<211> 30
<212> DNA
<213> Artificial Sequence

<220>
<221> source
<222> 1..30
<223> /mol_type="unassigned DNA"
/note="amnachra8-001S "
/organism="Artificial Sequence"

<400> 45
catgtttaaa atgcaaatat tgacgcttgg 30

<210> 46
<211> 30
<212> DNA
<213> Artificial Sequence

<220>
<221> source
<222> 1..30
<223> /mol_type="unassigned DNA"
/note="amnachra8-002AS "
/organism="Artificial Sequence"

<400> 46
ttatccttct ggagaaatgt ctatatttgg 30

<210> 47
<211> 30

<212> DNA
<213> Artificial Sequence

<220>
<221> source
<222> 1..30
<223> /mol_type="unassigned DNA"
/note="amnachra9-001S "
/organism="Artificial Sequence"

<400> 47
catgaaaatg agaataataa cagctcttgg 30

<210> 48
<211> 25
<212> DNA
<213> Artificial Sequence

<220>
<221> source
<222> 1..25
<223> /mol_type="unassigned DNA"
/note="amnachra9-002AS "
/organism="Artificial Sequence"

<400> 48
tcacgtggat ggtacaagag tgatc 25

<210> 49
<211> 26
<212> DNA
<213> Artificial Sequence

<220>
<221> source
<222> 1..26
<223> /mol_type="unassigned DNA"
/note="amnachrb1-001S "
/organism="Artificial Sequence"

<400> 49
catgcataat atttgctcga ggctcg 26

<210> 50
<211> 30
<212> DNA
<213> Artificial Sequence

<220>
<221> source
<222> 1..30
<223> /mol_type="unassigned DNA"
/note="amnachrb1-002AS "
/organism="Artificial Sequence"

<400> 50
ttattttcca cggtagatct ctattatatg 30

<210> 51

<211> 29
<212> DNA
<213> Artificial Sequence

<220>
<221> source
<222> 1..29
<223> /mol_type="unassigned DNA"
/note="amnachrb2-001S "
/organism="Artificial Sequence"

<400> 51
catgttaaac atgaagaata tattccccg 29

<210> 52
<211> 37
<212> DNA
<213> Artificial Sequence

<220>
<221> source
<222> 1..37
<223> /mol_type="unassigned DNA"
/note="amnachrb2-007AS "
/organism="Artificial Sequence"

<400> 52
gaaaaagatt aacacaagtt acattccata agttaac 37

<210> 53
<211> 23
<212> DNA
<213> Artificial Sequence

<220>
<221> source
<222> 1..23
<223> /mol_type="unassigned DNA"
/note="primer ceric3-001S "
/organism="Artificial Sequence"

<400> 53
catgccaaaa actgaacggc gtc 23

<210> 54
<211> 26
<212> DNA
<213> Artificial Sequence

<220>
<221> source
<222> 1..26
<223> /mol_type="unassigned DNA"
/note="ceric3-002AS "
/organism="Artificial Sequence"

<400> 54
tcaagtcttt ttaggtctcc gccttc 26

<210> 55
<211> 23
<212> DNA
<213> Artificial Sequence

<220>
<221> source
<222> 1..23
<223> /mol_type="unassigned DNA"
/note="amric3-008S "
/organism="Artificial Sequence"

<400> 55
catggctgaa ataacagatt tcg 23

<210> 56
<211> 25
<212> DNA
<213> Artificial Sequence

<220>
<221> source
<222> 1..25
<223> /mol_type="unassigned DNA"
/note="amric3-005AS"
/organism="Artificial Sequence"

<400> 56
catgtggagg tgtacgtcgt tcttg 25

<210> 57
<211> 29
<212> DNA
<213> Artificial Sequence

<220>
<221> source
<222> 1..29
<223> /mol_type="unassigned DNA"
/note="emc6-001S "
/organism="Artificial Sequence"

<400> 57
catgttggga aagattaaaa caaaacaag 29

<210> 58
<211> 32
<212> DNA
<213> Artificial Sequence

<220>
<221> source
<222> 1..32
<223> /mol_type="unassigned DNA"
/note="emc6-002AS "
/organism="Artificial Sequence"

<400> 58
cttttaatca gtatacatgt accattccat at 32

<210> 59
<211> 30
<212> DNA
<213> Artificial Sequence

<220>
<221> source
<222> 1..30
<223> /mol_type="unassigned DNA"
/note="amunc50-001S "
/organism="Artificial Sequence"

<400> 59
catgaaatat tctacatcac caccagtaag 30

<210> 60
<211> 31
<212> DNA
<213> Artificial Sequence

<220>
<221> source
<222> 1..31
<223> /mol_type="unassigned DNA"
/note="amunc50-002AS "
/organism="Artificial Sequence"

<400> 60
tattcaaaca actcgataat gataaaattc c 31

<210> 61
<211> 32
<212> DNA
<213> Artificial Sequence

<220>
<221> source
<222> 1..32
<223> /mol_type="unassigned DNA"
/note="amunc74-001S "
/organism="Artificial Sequence"

<400> 61
catggtaata ataacgaaat tgatgtttat tg 32

<210> 62
<211> 31
<212> DNA
<213> Artificial Sequence

<220>
<221> source
<222> 1..31
<223> /mol_type="unassigned DNA"
/note="amunc74-002AS "
/organism="Artificial Sequence"

<400> 62
caattattct aatctttctt catatgattt g 31

<210> 63
<211> 28
<212> DNA
<213> Artificial Sequence

<220>
<221> source
<222> 1..28
<223> /mol_type="unassigned DNA"
/note="amric3-007AS "
/organism="Artificial Sequence"

<400> 63
caaggctcca cagattcaga tttgattc 28

<210> 64
<211> 1137
<212> DNA
<213> Caenorhabditis elegans

<220>
<221> source
<222> 1..1137
<223> /mol_type="unassigned DNA"
/note="nucleic acid Cele ric3"
/organism="Caenorhabditis elegans"

<400> 64
atgccaaaaa ctgaacggcg tcgtgataga gatcgagaca gagatcgaga aaggagaaac 60

agacgaaaaa gggatgatag ttacgatgat tacgatgaag aaggtggaat atcaggatgg 120

aagcttggtt tagtatttgg agtaatcgtt gtttgctttg caatgcttta tccaacactt 180

ttccatccaa tgctaatggg attcttgggt cgttcaccac catcaagtcc atcaataaac 240

caacaacgtc caccaattca tccagcaatg ggtgggggaa gtggacaacg tcatccaggt 300

ggtggtgcag atgtacatcc agcaatgaga atggctcaag cacaagctga aagtcaatct 360

ggtggatcaa agggaatgtt cacatggatg ttacctgtat atacaatcgg agttgtttta 420

tttcttttgt atacgctgtt taaatcaaaa ggaaagaaat caaagcgaaa gaagagaaat 480

tattttgatt ctgaagacga tgatgatgaa tctgaaagtg agactaaata tggcggaaaa 540

tttggtaaaa agaagcttga aggacttcag aaaaggcttc gagagactga aagtgcaatg 600

tctaagattt tagaacaact tgaatcagta caagctggcg ctaatcctgt cgacttagat 660

gctgccgata agaggtcgga acaacttgaa gaagatccat cagttaaaga agcagttgga 720

ttaactgaaa ccaatgaaca atacataaaa gatcttgagg ttgcactgaa agagtttcag 780

tccttgtcaa aagaatatga taaagcgaaa atgaagaaac tgaaaagaaa agattcttcg 840

agtgacgaag atgaagaaga cgaagaagag aatagctctg aattgtcaga aatcgaagag 900

gaagaagagg aagttaaacc agtgaaaaag tcaaaatcat cctcccaatc agttggaaaa 960

aggaagaatc gaccaaaaag cacatcagaa gaagaagatg aaggagaaga agaatcacga 1020

aaagttgcag aagatgctga agaagaagga attgacattg attctgagat ccgagagcac 1080

gcggaaaagg agaaaaaaga taaaaatgtg cgaaggcgga gacctaaaaa gacttga 1137

<210> 65
<211> 378
<212> PRT
<213> Caenorhabditis elegans

<220>
<223> amino acid Cele ric3

<400> 65
Met Pro Lys Thr Glu Arg Arg Arg Asp Arg Asp Arg Asp Arg Asp Arg
1 5 10 15
Glu Arg Arg Asn Arg Arg Lys Arg Asp Asp Ser Tyr Asp Asp Tyr Asp
20 25 30
Glu Glu Gly Gly Ile Ser Gly Trp Lys Leu Gly Leu Val Phe Gly Val
35 40 45
Ile Val Val Cys Phe Ala Met Leu Tyr Pro Thr Leu Phe His Pro Met
50 55 60
Leu Met Gly Phe Leu Gly Arg Ser Pro Pro Ser Ser Pro Ser Ile Asn
65 70 75 80
Gln Gln Arg Pro Pro Ile His Pro Ala Met Gly Gly Gly Ser Gly Gln
85 90 95
Arg His Pro Gly Gly Gly Ala Asp Val His Pro Ala Met Arg Met Ala
100 105 110
Gln Ala Gln Ala Glu Ser Gln Ser Gly Gly Ser Lys Gly Met Phe Thr
115 120 125
Trp Met Leu Pro Val Tyr Thr Ile Gly Val Val Leu Phe Leu Leu Tyr
130 135 140
Thr Leu Phe Lys Ser Lys Gly Lys Lys Ser Lys Arg Lys Lys Arg Asn
145 150 155 160
Tyr Phe Asp Ser Glu Asp Asp Asp Asp Glu Ser Glu Ser Glu Thr Lys
165 170 175
Tyr Gly Gly Lys Phe Gly Lys Lys Lys Leu Glu Gly Leu Gln Lys Arg
180 185 190
Leu Arg Glu Thr Glu Ser Ala Met Ser Lys Ile Leu Glu Gln Leu Glu
195 200 205
Ser Val Gln Ala Gly Ala Asn Pro Val Asp Leu Asp Ala Ala Asp Lys
210 215 220
Arg Ser Glu Gln Leu Glu Glu Asp Pro Ser Val Lys Glu Ala Val Gly
225 230 235 240
Leu Thr Glu Thr Asn Glu Gln Tyr Ile Lys Asp Leu Glu Val Ala Leu
245 250 255
Lys Glu Phe Gln Ser Leu Ser Lys Glu Tyr Asp Lys Ala Lys Met Lys
260 265 270
Lys Leu Lys Arg Lys Asp Ser Ser Ser Asp Glu Asp Glu Glu Asp Glu
275 280 285
Glu Glu Asn Ser Ser Glu Leu Ser Glu Ile Glu Glu Glu Glu Glu Glu
290 295 300
Val Lys Pro Val Lys Lys Ser Lys Ser Ser Ser Gln Ser Val Gly Lys
305 310 315 320
Arg Lys Asn Arg Pro Lys Ser Thr Ser Glu Glu Glu Asp Glu Gly Glu
325 330 335
Glu Glu Ser Arg Lys Val Ala Glu Asp Ala Glu Glu Glu Gly Ile Asp
340 345 350
Ile Asp Ser Glu Ile Arg Glu His Ala Glu Lys Glu Lys Lys Asp Lys
355 360 365
Asn Val Arg Arg Arg Arg Pro Lys Lys Thr

370 375